(19) Europäisches Patentamt
European Patent Office
Office européen des brevets

(11) EP 1 274 687 B1

(12) **EUROPEAN PATENT SPECIFICATION**

(45) Date of publication and mention of the grant of the patent:
**05.01.2005 Bulletin 2005/01**

(21) Application number: **01933685.8**

(22) Date of filing: **12.03.2001**

(51) Int Cl.[7]: **C07D 217/20**, C07D 217/04, C07D 401/12, A61K 31/435, A61P 3/04, A61P 25/20

(86) International application number:
**PCT/EP2001/002733**

(87) International publication number:
**WO 2001/068609 (20.09.2001 Gazette 2001/38)**

# (54) 1,2,3,4- TETRAHYDROISOQUINOLINE DERIVATIVES

1,2,3,4-TETRAHYDROISOCHINOLIN-DERIVATE

DERIVES DE 1,2,3,4-TETRAHYDROISOQUINOLINE

(84) Designated Contracting States:
**AT BE CH CY DE DK ES FI FR GB GR IE IT LI LU MC NL PT SE TR**

(30) Priority: **14.03.2000 WOPCT/EP00/02245**

(43) Date of publication of application:
**15.01.2003 Bulletin 2003/03**

(73) Proprietor: **Actelion Pharmaceuticals Ltd.**
**4123 Allschwil (CH)**

(72) Inventors:
- **AISSAOUI, Hamed**
  **68270 Wittenheim (FR)**
- **CAPPI, Michael**
  **81369 Muenchen (DE)**
- **CLOZEL, Martine**
  **68300 Saint-Louis (FR)**
- **FISCHLI, Walter, Actelion Pharmaceuticals Ltd.**
  **4123 Allschwil (CH)**
- **KOBERSTEIN, Ralf**
  **79539 Lörrach (DE)**

(74) Representative: **Hofmann, Dieter, Dr.**
**Therwillerstrasse 87**
**4153 Reinach (CH)**

(56) References cited:

| | |
|---|---|
| **EP-A- 0 494 623** | **WO-A-00/29399** |
| **WO-A-00/35882** | **WO-A-00/78742** |
| **WO-A-00/78744** | **WO-A-01/02368** |
| **WO-A-98/23593** | **WO-A-99/58533** |
| **DD-A- 258 817** | **DD-A- 261 158** |
| **DD-B- 204 917** | **US-A- 3 480 714** |

- **CHEMICAL ABSTRACTS, vol. 109, no. 19, 7 November 1988 (1988-11-07) Columbus, Ohio, US; abstract no. 170679, KHALMURATOV, KH. A. ET AL: "Synthesis of substituted aminoacetyl derivatives of 2,5-2,7- diaminofluorene" XP002172557 & UZB. KHIM. ZH. (1988), (1), 34-6,**
- **LUKEVICS, E. ET AL: "Silyl modification of biologically active compounds. 4. Derivatives of amino acids in the tetrahydroquinoline, tetrahydroisoquinoline, and tetrahydrosilaisoquinoline series" CHEM. HETEROCYCL. COMPD. (N. Y.) (1997), 33(2), 234-238, XP001008092**
- **PATENT ABSTRACTS OF JAPAN vol. 1998, no. 09, 31 July 1998 (1998-07-31) & JP 10 095766 A (SANWA KAGAKU KENKYUSHO CO LTD), 14 April 1998 (1998-04-14)**
- **PATENT ABSTRACTS OF JAPAN vol. 010, no. 214 (C-362), 25 July 1986 (1986-07-25) & JP 61 053268 A (HOKURIKU SEIYAKU CO LTD), 17 March 1986 (1986-03-17)**
- **PATENT ABSTRACTS OF JAPAN vol. 1996, no. 02, 29 February 1996 (1996-02-29) & JP 07 267961 A (TAISHO PHARMACEUT CO LTD), 17 October 1995 (1995-10-17)**
- **CHEMICAL ABSTRACTS, vol. 110, no. 23, 5 June 1989 (1989-06-05) Columbus, Ohio, US; abstract no. 205079, SAIITKULOV, A. M. ET AL: "Interferon-inducing activity of fluorenone derivatives" XP002172558 & UZB. BIOL. ZH. (1988), (6), 5-9,**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 274 687 B1

- **CHEMICAL ABSTRACTS, vol. 110, no. 19, 8 May 1989 (1989-05-08) Columbus, Ohio, US; abstract no. 173523, AUELBEKOV, S. A. ET AL: "Reaction of 2,7-bis(chloroacetylamino)fluoren-9-one with amines" XP002172559 & UZB. KHIM. ZH. (1988), (3), 23-6,**
- **CHEMICAL ABSTRACTS, vol. 110, no. 1, 2 January 1989 (1989-01-02) Columbus, Ohio, US; abstract no. 459, KHALMURATOV, KH. A. ET AL: "DNA complexes with derivatives of fluorene and fluorenone having interferon-inducing activity" XP002172560 & KHIM. PRIR. SOEDIN. (1988), (3), 404-9,**
- **HAZEBROUCQ, GEORGES: "2,3,4,5-Tetrahydro-1H-3-benzazepin-1-ones and hexahydroimidazoisoquinolines" ANN. CHIM. (PARIS) (1966), 1(5/6), 221-54, XP001007459**
- **KEMPTER, G. ET AL: "Synthesis of heteroanalogs of piperidinoacetanilides" WISS. Z. - MARTIN-LUTHER-UNIV. HALLE-WITTENBERG, MATH.-NATURWISS. REIHE (1983), 32(5), 3-25, XP000943458**
- **CHEMICAL ABSTRACTS, vol. 68, no. 1, 1 January 1968 (1968-01-01) Columbus, Ohio, US; abstract no. 2798, HORI, MIKIO ET AL: "Benzaza cycloalkane derivatives. IV" XP002172561 & GIFU YAKKA DAIGAKU KIYO (1966), NO. 16, 68-70,**

## Description

The present invention relates to novel 1,2,3,4-tetrahydroisoquinoline derivatives of the general formula I and their use as pharmaceuticais. The invention also concerns related aspects including processes for the preparation of the compounds, pharmaceutical compositions containing one or more compounds of formula I, and especially their use as orexin receptor antagonists.

The orexins (hypocretins) comprise two neuropeptides produced in the hypothalamus: the orexin A (OX-A) (a 33 aminoacid peptide) and the orexin B (OX-B) (a 28 aminoacid peptide) (Sakurai T. *et al*., **Cell**, 1998, 92, 573-585). Orexins are found to stimulate food consumption in rats suggesting a physiological role for these peptides as mediators in the central feedback mechanism that regulates feeding behavior (Sakurai *T. et al*., **Cell**, 1998, 92, 573-585). On the other hand, it was also proposed that orexins regulate states of sleep and wakefulness opening potentially novel therapeutic approaches for narcoleptic patients (Chemelli R.M. *et al*., **Cell,** 1999, 98, 437-451). Two orexin receptors have been cloned and characterized in mammals which belong to the G-protein coupled receptor superfamily (Sakurai T. *et al*., **Cell,** 1998, 92, 573-585), the orexin-1 receptor ($OX_1$) which is selective for OX-A and the orexin-2 receptor ($OX_2$) which is capable to bind OX-A as well as OX-B.

Orexin receptors are found in the mammalian host and may be responsible for many biological functions such as pathologies including, but not limited to, depression; anxiety; addictions; obsessive compulsive disorder; affective neurosis; depressive neurosis; anxiety neurosis; dysthymic disorder; behaviour disorder; mood disorder; sexual dysfunction; psychosexual dysfunction; sex disorder; schizophrenia; manic depression; delerium; dementia; severe mental retardation and dyskinesias such as Huntington's disease and Tourette syndrome; feeding disorders such as anorexia, bulimia, cachexia and obesity; diabetes; appetite/taste disorders; vomiting/nausea; asthma; cancer; Parkinson's disease; Cushing's syndrome/disease; basophil adenoma; prolactinoma; hyperprolactinemia; hypopituitarism; hypophysis tumor/adenoma; hypothalamic diseases; inflammatory bowel disease; gastric diskinesia; gastric ulcus; Froehlich's syndrome; adrenohypophysis disease; hypophysis disease; pituitary growth hormone; adrenohypophysis hypofunction; adrenohypophysis hyperfunction; hypothalamic hypogonadism; Kallman's syndrome (anosmia, hyposmia); functional or psychogenic amenorrhea; hypopituitarism; hypothalamic hypothyroidism; hypothalamic-adrenal dysfunction; idiopathic hyperprolactinemia; hypothalamic disorders of growth hormone deficiency; idiopathic growth deficiency; dwarfism; gigantism; acromegaly; disturbed biological and circadian rhythms; sleep disturbances associated with deseases such as neurological disorders, neuropathic pain and restless leg syndrome; heat and lung diseases, acute and congestive heart failure; hypotension; hypertension; urinary retention; osteoporosis; angina pectoris; myocardinal infarction; ischaemic or haemorrhagic stroke; subarachnoid haemorrhage; ulcers; allergies; benign prostatic hypertrophy; chronic renal failure; renal disease; impaired glucose tolerance; migraine; hyperalgesia; pain; enhanced or exaggerated sensitivity to pain such as hyperalgesia, causalgia, and allodynia; acute pain; burn pain; atypical facial pain; neuropathic pain; back pain; complex regional pain syndrome I and II; arthritic pain; sports injury pain; pain related to infection e.g. HIV, post-chemotherapy pain; post-stroke pain; post-operative pain; neuralgia; conditions associated with visceral pain such as irritable bowel syndrome, migraine and angina; urinary bladder incontinence e.g. urge incontinence; tolerance to narcotics or withdrawal from narcotics; sleep disorders; sleep apnea; narcolepsy; insomnia; parasomnia; jet-lag syndrome; and neurodegerative disorders including 'nosological entities such as disinhibition-dementia-parkinsonism-amyotrophy complex; pallido-ponto-nigral degeneration epilepsy; seizure disorders and other diseases related to orexin.

The present invention provides 1,2,3,4-tetrahydroisoquinoline derivatives which are non-peptide antagonists of human orexin receptors, in particular $OX_1$ receptors. In particular, these compounds are of potential use in the treatment of obesity and/or sleep disorders.

So far not much is known about low molecular weight compounds which have a potential to antagonise either specifically $OX_1$ or $OX_2$ or both receptors at the same time. Recently WO 9909024 has been published wherein phenylurea and phenylthiourea derivatives as $OX_1$ antagonists are disclosed. Also quite recently WO 9958533 has been published disclosing the same type of compounds which are again described as being preferably $OX_1$ receptor antagonists. The novel compounds of the present invention belong to an entirely different class of low molecular weight compounds as compared to all prior art orexin receptor antagonists so far published.

The present invention relates to novel 1,2,3,4-tetrahydroisoquinoline derivatives of the general formula (I).

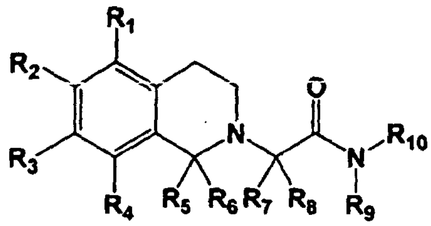

Formula (I)

wherein:

$R^1$, $R^2$, $R^3$, $R^4$ independently represent cyano, nitro, halogen, hydrogen, hydroxy, lower alkyl, lower alkenyl, lower alkoxy, lower alkenyloxy, trifluoromethyl, trifluoromethoxy, cycloalkyloxy, aryloxy, aralkyloxy, heterocyclyloxy, heterocyclylalkyloxy, $R^{11}CO$-, $NR^{12}R^{13}CO$-, $R^{12}R^{13}N$-, $R^{11}OOC$-, $R^{11}SO_2NH$- or $R^{14}$-CO-NH- or $R^2$ and $R^3$ together as well as $R^1$ and $R^2$ together and $R^3$ and $R^4$ together may form with the phenyl ring a five, six or seven-membered ring containing one or two oxygen atoms;

$R^5$, $R^6$, $R^7$,$R^8$, $R^9$, $R^{10}$ independently represent hydrogen, aryl, aralkyl, lower alkyl, lower alkenyl, trifluoromethyl, cycloalkyl, heterocyclyl or heterocyclyl-lower alkyl;

$R^{11}$ represents lower alkyl, aryl, aralkyl, heterocyclyl or heterocyclyl-lower alkyl;

$R^{12}$and $R^{13}$ independently represent hydrogen, alkyl, cycloalkyl, aryl, aralkyl, heterocyclyl or heterocyclyl-lower alkyl;

$R^{14}$ represents alkyl, aryl, cycloalkyl, heterocyclyl, $R^{12}R^{13}N$- or $R^{11}O$-;

wherein the term "lower alkyl", alone or in combination, signifies a straight-chain or branched-chain alkyl group with 1 to 8 carbon atoms, the term "lower alkenyl", alone or in combination, signifies a straight-chain or a branched-chain alkenyl group with 2 to 5 carbon atoms, the term "cycloalkyl", alone or in combination, signifies a cycloalkyl ring with 3 to 8 carbon atoms; the term "aryl", alone or in combination, signifies a phenyl or a naphtyl group which optionally carries one or more substituents; and the term "heterocyclyl", alone or in combination, signifies a 5- to 10-membered monocyclic or bicyclic ring, which may be saturated, partially unsaturated or aromatic containing 1, 2 or 3 heteroatoms selected from oxygen, nitrogen and sulphur which may be the same or different and which may have up to 5 optional substituents;

The compounds of formula I can contain one or more asymmetric centres and can be present in the form of optically pure enantiomers, mixtures of enantiomers such as, for example, racemates, optically pure diastereoisomers, mixtures of diastereoisomers, diastereoisomeric racemates, mixture of diastereoisomeric racemates, or meso forms and pharmaceutically acceptable salts thereof.

In the present description the term "lower alkyl", alone or in combination, signifies a straight-chain or branched-chain alkyl group with 1 to 8 carbon atoms, preferably a straight or branched-chain alkyl group with 1-5 carbon atoms. Examples of straight-chain and branched $C_1$-$C_8$ alkyl groups are methyl, ethyl, propyl, isopropyl, butyl, pentyl, hexyl, heptyl, octyl, isobutyl, tert-butyl, the isomeric pentyls, the isomeric hexyls, the isomeric heptyls and the isomeric octyls, preferably methyl, ethyl, propyl, isopropyl, butyl, 2-butyl, tert-butyl and pentyl.

The term "lower alkenyl", alone or in combination, signifies a straight-chain or branched-chain alkenyl group with 2 to 5 carbon atoms, preferably allyl and vinyl.

The term "lower alkoxy", alone or in combination, signifies a group of the formula alkyl-O- in which the term "alkyl" has the previously given significance, such as methoxy, ethoxy, n-propoxy, isopropoxy, n-butoxy, isobutoxy, sec-butoxy and tert-butoxy, preferably methoxy and ethoxy.

Lower alkenyloxy groups are preferably vinyloxy and allyloxy.

The term "cycloalkyl", alone or in combination, signifies a cycloalkyl ring with 3 to 8 carbon atoms and preferably a cycloalkyl ring with 3 to 6 carbon atoms. Examples of $C_3$-$C_8$ cycloalkyl are cyclopropyl, cyclobutyl, cyclopentyl, cyclohexyl, cycloheptyl and cyclooctyl, preferably cyclopropyl, cyclohexyl and particularly cyclohexyl or lower alkyl substituted cycloalkyl which may preferably be substituted with lower alkyl such as methyl-cyclopropyl, dimethyl-cyclopropyl, methyl-cyclobutyl, methyl-cyclopentyl, methyl-cyclohexyl, dimethyl-cyclohexyl.

The term "aryl", alone or in combination, signifies a phenyl or naphthyl group which optionally carries one or more substituents, preferably one or two substituents, each independently selected from cyano, halogen, hydroxy, lower alkyl, lower alkenyl, lower alkoxy, lower alkenyloxy, nitro, trifluoromethyl, trifluoromethoxy, amino, carboxy and

the like, such as phenyl, p-tolyl, 4-methoxyphenyl, 4-tert-butoxyphenyl, 4-fluorophenyl, 2-chlorophenyl, 4-hydroxyphenyl, 1-naphthyl and 2-naphthyl. Preferred are carboxyphenyl, lower alkoxy-phenyl, hydroxyphenyl and particularly phenyl.

The term "aralkyl", alone or in combination, signifies an alkyl or cycloalkyl group as previously defined in which one hydrogen atom has been replaced by an aryl group as previously defined. Preferred are benzyl and benzyl substituted in the phenyl ring with hydroxy, lower alkyl, lower alkoxy or halogen preferably chlorine. Particularly preferred is benzyl.

For the term "heterocyclyl" and "heterocyclyl-lower alkyl", the heterocyclyl group is preferably a 5- to 10-membered monocyclic or bicyclic ring, which may be saturated, partially unsaturated or aromatic containing for example 1, 2 or 3 heteroatoms selected from oxygen, nitrogen and sulphur which may be the same or different. Example of such heterocyclyl groups are pyrrolidinyl, piperidinyl, piperazinyl, morpholinyl, pyridyl, pyrimidinyl, pyrazinyl, pyridazinyl, quinolyl, isoquinolyl, thienyl, thiazolyl, isothiazolyl, furyl, imidazoyl, pyrazolyl, pyrrolyl, indazolyl, indolyl, isoindolyl, isoxazolyl, oxazolyl, quinoxalinyl, phthalazinyl, cinnolinyl, dihydropyrrolyl, pyrrolidinyl, isobenzofuranyl, tetrahydrofuranyl, dihydropyranyl. The heterocyclyl group may have up to 5, preferably 1, 2 or 3 optional substituents. Examples of suitable substituents include halogen, lower alkyl, amino, nitro, cyano, hydroxy, lower alkoxy, carboxy and lower alkyloxy-carbonyls.

The term "halogen" signifies fluorine, chlorine, bromine or iodine and preferably chlorine and bromine and particularly chlorine.

The term "carboxy", alone or in combination, signifies a -COOH group.

A group of preferred compounds according to the present invention are compounds of formula (I) wherein $R^2$, $R^3$, $R^6$, $R^7$, $R^8$ and $R^9$ are hydrogen. Examples of preferred compounds are:

2-[1-(3,4-Dimethoxy-benzyl)-5,8-dimethoxy-3,4-dihydro-1*H*-isoquinolin-2-yl]-*N*-(pyridin-2-yl-methyl)-acetamide:

2-[1-(3,4-dimethoxy-benzyl)-8-ethoxy-5-methoxy-3,4-dihydro-1*H*-isoquinolin-2-yl]-*N*-(pyridin-2-yl-methyl)-acetamide:

2-[1-(3,4-dimethoxy-benzyl)-8-propoxy-5-methoxy-3,4-dihydro-1*H*-isoquinolin-2-yl]-*N*-(pyridin-2-yl-methyl)-acetamide:

2-[1-(3,4-dimethoxy-benzyl)-8-allyloxy-5-methoxy-3,4-dihydro-1*H*-isoquinolin-2-yl]-*N*-(pyridin-2-yl-methyl)-acetamide:

2-[1-(3,4-dimethoxy-benzyl)-8-isopropoxy-5-methoxy-3,4-dihydro-1*H*-isoquinolin-2-yl]-*N*-(pyridin-2-yl-methyl)-acetamide:

2-[1-(3,4-dimethoxy-benzyl)-5-propoxy-8-methoxy-3,4-dihydro-1*H*-isoquinolin-2-yl]-*N*-(pyridin-2-yl-methyl)-acetamide:

Another group of preferred compounds according to the present invention are compounds of formula (II)

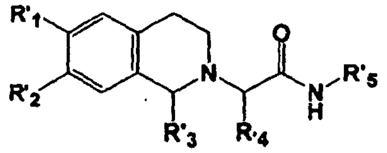

General formula II

wherein:

$R'^1$ and $R'^2$ independently represent hydrogen, hydroxy, alkoxy, heteroaryloxy, carbamoyloxy or halogen or may form with the phenyl ring a five, six or seven membered-ring containing one or two oxygen atoms,
$R'^3$, $R'^4$, $R'^5$ independently represent aryl, aralkyl, lower alkyl, lower alkenyl, trifluoromethyl, cycloalkyl, heterocyclyl or heterocyclyl-lower alkyl.

The compounds of formula (II) can contain one or more asymmetric centres and can be present in the form of optically pure enantiomers, mixtures of enantiomers such as, for example, racemates, optically pure diastereoisomers, mixtures of diastereoisomers, diastereoisomeric racemates, mixture of diastereoisomeric racemates, or meso forms and pharmaceutically acceptable salts thereof.

Examples of preferred compounds of formula (II) are:

2-[1-(3,4-Dimethoxy-benzyl)-6,7-dimethoxy-3,4-dihydro-1*H*-isoquinolin-2-yl]-*N*-benzyl-acetamide

2-[1-(3,4-Dimethoxy-benzyl)-6,7-dimethoxy-3,4-dihydro-1*H*-isoquinolin-2-yl]-*N*-naphthalen-1-ylmethyl-acetamide

2-[1-(3,4-Dimethoxy-benzyl)-6,7-dimethoxy-3,4-dihydro-1*H*-isoquinolin-2-yl]-*N*-(2-methoxy-benzyl)-acetamide

2-[1-(3,4-Dimethoxy-benzyl)-6,7-dimethoxy-3,4-dihydro-1*H*-isoquinolin-2-yl]-*N*-(4-fluoro-benzyl)-acetamide

2-[1-(3,4-Dimethoxy-benzyl)-6,7-dimethoxy-3,4-dihydro-1*H*-isoquinolin-2-yl]-*N*-(6-methoxy-naphthalen-2-ylmethyl)-acetamide

2-[1-(3,4-Dimethoxy-benzyl)-6,7-dimethoxy-3,4-dihydro-1*H*-isoquinolin-2-yl]-*N*-(4-methoxy-naphthalen-2-ylmethyl)-acetamide

2-[1-(3,4-Dimethoxy-benzyl)-6,7-dimethoxy-3,4-dihydro-1*H*-isoquinolin-2-yl]-*N*-(3,6)-difluoro-benzyl)-acetamide

2-[1-(3,4-Dimethoxy-benzyl)-6,7-dimethoxy-3,4-dihydro-1*H*-isoquinolin-2-yl]-*N*-(1-phenyl-ethyl)-acetamide

2-[1-(3,4-Dimethoxy-benzyl)-6,7-dimethoxy-3,4-dihydro-1*H*-isoquinolin-2-yl]-*N*-(pyridin-3-ylmethyl)-acetamide

2-[1-(3,4-Dimethoxy-benzyl)-6,7-dimethoxy-3,4-dihydro-1*H*-isoquinolin-2-yl]-*N*-(2-methyl-benzyl)-acetamide

2-[1-(3,4-Dimethoxy-benzyl)-6,7-dimethoxy-3,4-dihydro-1*H*-isoquinolin-2-yl]-*N*-(3-methyl-benzyl)-acetamide

2-{1-[4-(pyrimidin-2-yloxy)-3-methoxy-benzyl]-6,7-dimethoxy-3,4-dihydro-1*H*-isoquinolin-2-yl}-*N*-benzyl-acetamide

2-[1-(3,4-dimethoxy-benzyl)-7-(1-methyl-prop-2-oxy)-6-methoxy-3,4-dihydro-1*H*-isoquinolin-2-yl]-*N*-benzyl-acetamide

2-[1-(3,4-Dimethoxy-benzyl)-6-methoxy-7-isopropoxy-3,4-dihydro-1*H*-isoquinolin-2-yl]-*N*-benzyl-acetamide

2-[1-(3,4-dimethoxy-benzyl)-7-ethoxy-6-methoxy-3,4-dihydro-1*H*-isoquinolin-2-yl]-*N*-benzyl-acetamide

2-[1-(3,4-dimethoxy-benzyl)-7-propoxy-6-methoxy-3,4-dihydro-1*H*-isoquinolin-2-yl]-*N*-benzyl-acetamide

2-[1-(3,4-dimethoxy-benzyl)-7-allyloxy-6-methoxy-3,4-dihydro-1*H*-isoquinolin-2-yl]-*N*-benzyl-acetamide

*N*-benzyl-2-[1-(3,4-Dimethyl-benzyl)-6,7-dimethoxy-3,4-dihydro-1*H*-isoquinolin-2-yl]-acetamide

*N*-benzyl-2-[1-(3,4-Diethyl-benzyl)-6,7-dimethoxy-3,4-dihydro-1*H*-isoquinolin-2-yl]-acetamide

2-[1-(3,4-Diethyl-benzyl)-6,7-dimethoxy-3,4-dihydro-1*H*-isoquinolin-2-yl]-*N*-(pyridin-2-yl-methyl)-acetamide

2-[1-(3,4-Diethyl-benzyl)-6,7-dimethoxy-3,4-dihydro-1*H*-isoquinolin-2-yl]-*N*-(pyridin-3-yl-methyl)-acetamide

2-[1-(3,4-Diethyl-benzyl)-6,7-dimethoxy-3,4-dihydro-1*H*-isoquinolin-2-yl]-*N*-(pyridin-4-yl-methyl)-acetamide

2-[1-(3,4-Dichloro-benzyl)-6,7-dimethoxy-3,4-dihydro-1*H*-isoquinolin-2-yl]-*N*-(pyridin-3-yl-methyl)-acetamide

Examples of particularly preferred compounds of formula (II) are:

2-[1-(3,4-Dimethoxy-benzyl)-6,7-dimethoxy-3,4-dihydro-1*H*-isoquinolin-2-yl]-*N*-benzyl-acetamide

2-[1-(3,4-Dimethoxy-benzyl)-6,7-dimethoxy-3,4-dihydro-1*H*-isoquinolin-2-yl]-*N*-naphthalen-1-ylmethyl-acetamide

2-[1-(3,4-dimethoxy-benzyl)-7-(1-methyl-prop-2-oxy)-6-methoxy-3,4-dihydro-1*H*-isoquinolin-2-yl]-*N*-benzyl-acetamide

2-[1-(3,4-Dimethoxy-benzyl)-6-methoxy-7-isopropoxy-3,4-dihydro-1*H*-isoquinolin-2-yl]-N-benzyl-acetamide

2-[1-(3,4-dimethoxy-benzyl)-7-ethoxy-6-methoxy-3,4-dihydro-1*H*-isoquinolin-2-yl]-*N*-benzyl-acetamide

2-[1-(3,4-dimethoxy-benzyl)-7-propoxy-6-methoxy-3,4-dihydro-1*H*-isoquinolin-2-yl]-*N*-benzyl-acetamide

2-[1-(3,4-dimethoxy-benzyl)-7-allyloxy-6-methoxy-3,4-dihydro-1*H*-isoquinolin-2-yl]-*N*-benzyl-acetamide

*N*-benzyl-2-[1-(3,4-Dimethyl-benzyl)-6,7-dimethoxy-3,4-dihydro-1*H*-isoquinolin-2-yl]-acetamide

*N*-benzyl-2-[1-(3,4-Diethyl-benzyl)-6,7-dimethoxy-3,4-dihydro-1*H*-isoquinolin-2-yl]-acetamide

2-[1-(3,4-Diethyl-benzyl)-6,7-dimethoxy-3,4-dihydro-1*H*-isoquinolin-2-yl]-*N*-(pyridin-2-yl-methyl)-acetamide

2-[1-(3,4-Diethyl-benzyl)-6,7-dimethoxy-3,4-dihydro-1*H*-isoquinolin-2-yl]-*N*-(pyridin-3-yl-methyl)-acetamide

2-[1-(3,4-Diethyl-benzyl)-6,7-dimethoxy-3,4-dihydro-1*H*-isoquinolin-2-yl]-*N*-(pyridin-4-yl-methyl)-acetamide

2-[1-(3,4-Dichloro-benzyl)-6,7-dimethoxy-3,4-dihydro-1*H*-isoquinolin-2-yl]-*N*-(pyridin-3-yl-methyl)-acetamide

Examples of physiologically usable or pharmaceutically acceptable salts of the compounds of formula (I) are salts with physiologically compatible mineral acids such as hydrochloric acid, sulphuric or phosphoric acid; or with organic acids such as methanesulphonic acid, acetic acid, trifluoroacetic acid, citric acid, fumaric acid, maleic acid, tartaric acid, succinic acid or salicylic acid. The compounds of formula (I) with free carboxy groups can also form salts with physiologically compatible bases.

Examples of such salts are alkali metal, alkali earth metal, ammonium and alkylammoniumsalts such as Na, K, Ca or tetraalkylammonium salt. The compounds of formula (I) can also be present in the form of a zwitterion.

The compounds of formula (I) can contain several asymmetric centres and can be present in the form of optically pure enantiomers, mixtures of enantiomers such as, for example, racemates, optically pure diastereoisomers, mixtures of diastereoisomers, diasteroisomeric racemates or mixtures of diastereoisomeric racemates and the meso-forms.

Preferred compounds as described above have $IC_{50}$ values below 1000 nM; especially preferred compounds have $IC_{50}$ values below 100 nM which have been determinated with the FLIPR (Fluorometric Imaging Plates Reader) method described in the beginning of the experimental section.

The compounds of the general formula (I) and their pharmaceutically usable salts can be used for the treatment of diseases or disorders where an antagonist of a human orexin receptor is required such as obesity, diabetes, prolactinoma, narcolepsy, insomnia, sleep apnea, parasomnia, depression, anxiety, addictions, schizophrenia and dementia.

The compounds of formula (I) and their pharmaceutically usable salts are particularly useful for the treatment of obesity and sleep disorders.

The compounds of formula (I) and their pharmaceutically usable salts can be used as medicament (e.g. in the form of pharmaceutical preparations). The pharmaceutical preparations can be administered internally, such as orally (e.g. in the form of tablets, coated tablets, dragées, hard and soft gelatine capsules, solutions, emulsions or suspensions), nasally (e.g. in the form of nasal sprays) or rectally (e.g. in the form of suppositories). However, the administration can also be effected parentally, such as intramuscularly or intravenously (e.g. in the form of injection solutions).

The compounds of formula (I) and their pharmaceutically usable salts can be processed with pharmaceutically inert, inorganic or organic adjuvants for the production of tablets, coated tablets, dragées, and hard gelatine capsules. Lactose, corn starch or derivatives thereof, talc, stearic acid or its salts etc. can be used, for example, as such adjuvants for tablets, dragées, and hard gelatine capsules.

Suitable adjuvants for soft gelatine capsules, are, for example, vegetable oils, waxes, fats, semi-solid substances and liquid polyols, etc.

Suitable adjuvants for the production of solutions and syrups are, for example, water, polyols, saccharose, invert sugar, glucose, etc.

Suitable adjuvants for injection solutions are, for example, water, alcohols, polyols, glycerol, vegetable oils, etc.

Suitable adjuvants for suppositories are, for example, natural or hardened oils, waxes, fats, semi-solid or liquid polyols, etc.

Morever, the pharmaceutical preparations can contain preservatives, solubilizers, viscosity-increasing substances, stabilizers, wetting agents, emulsifiers, sweeteners, colorants, flavorants, salts for varying the osmotic pressure, buffers, masking agents or antioxidants. They can also contain still other therapeutically valuable substances. The invention also relates to processes for the preparation of compounds of Formula I.

The compounds of general formula (I) of the present invention are prepared according to the general sequence of reactions outlined in the schemes below, wherein $R^1$, $R^2$, $R^3$, $R^4$, $R^5$, $R^6$, $R^7$, $R^8$, $R^{10}$ are as defined in formula (I) above. As the case may be any compound obtained with one or more optically active carbon atom may be resolved into pure enantiomers or diastereomers, mixtures of enantiomers or diastereomers, diastereomeric racemates and the meso-forms in a manner known per se.

The compounds obtained may also be converted into a pharmaceutically acceptable salt thereof in a manner known per se.

The compounds of formula (I) may be prepared as single compounds or as libraries of compounds comprising at least 2, e.g. 5 to 1000 compounds of formula (I).

Compound libraries may be prepared by a combinatorial approach or by multiple parallel synthesis using solution phase chemistry.

For the combinatorial approach, the compounds of general formula (I) wherein $R^6$, $R^7$, $R^9$ are hydrogen, are prepared using an Ugi-three-components-condensation reaction (Ugi-3-CC) which involves the one-pot reaction between a 1,2,3,4-tetrahydroisoquinoline derivative, an aldehyde and an isocyanide (*Scheme 1*).

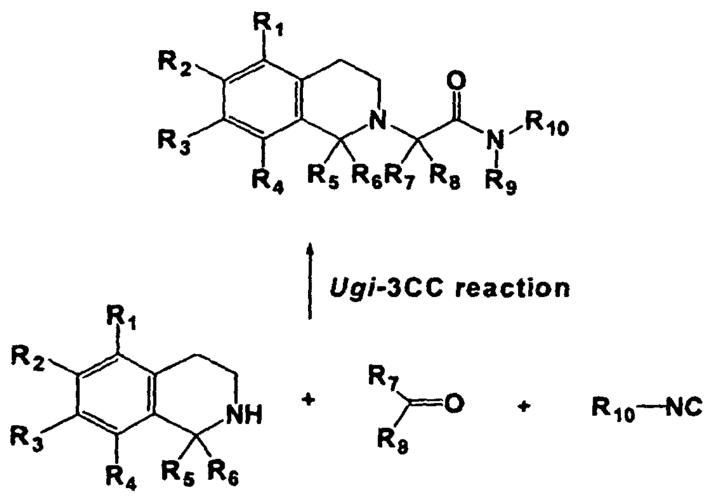


*Scheme 1*

Isocyanides not commercially available might be prepared from the corresponding amines by N-formylation followed by treatment with $POCl_3$ (see e.g. J. March, fourth edition, Wiley-Interscience publication, p. 1042).

The compounds of the general formula (I) wherein $R^6$ and $R^7$ are hydrogen, may also be prepared by different procedures. The synthetic route depends on the last chemical transformation which has to be carried out.

In all cases in which the coupling of the tetrahydroisoquinoline with the amide side-chain is the final step the standard procedure shown in (*Scheme2*) was followed. The tetrahydroisoauinolines as well as the amines ($R^9R^{10}NH$) could be either commercially available or synthesized.

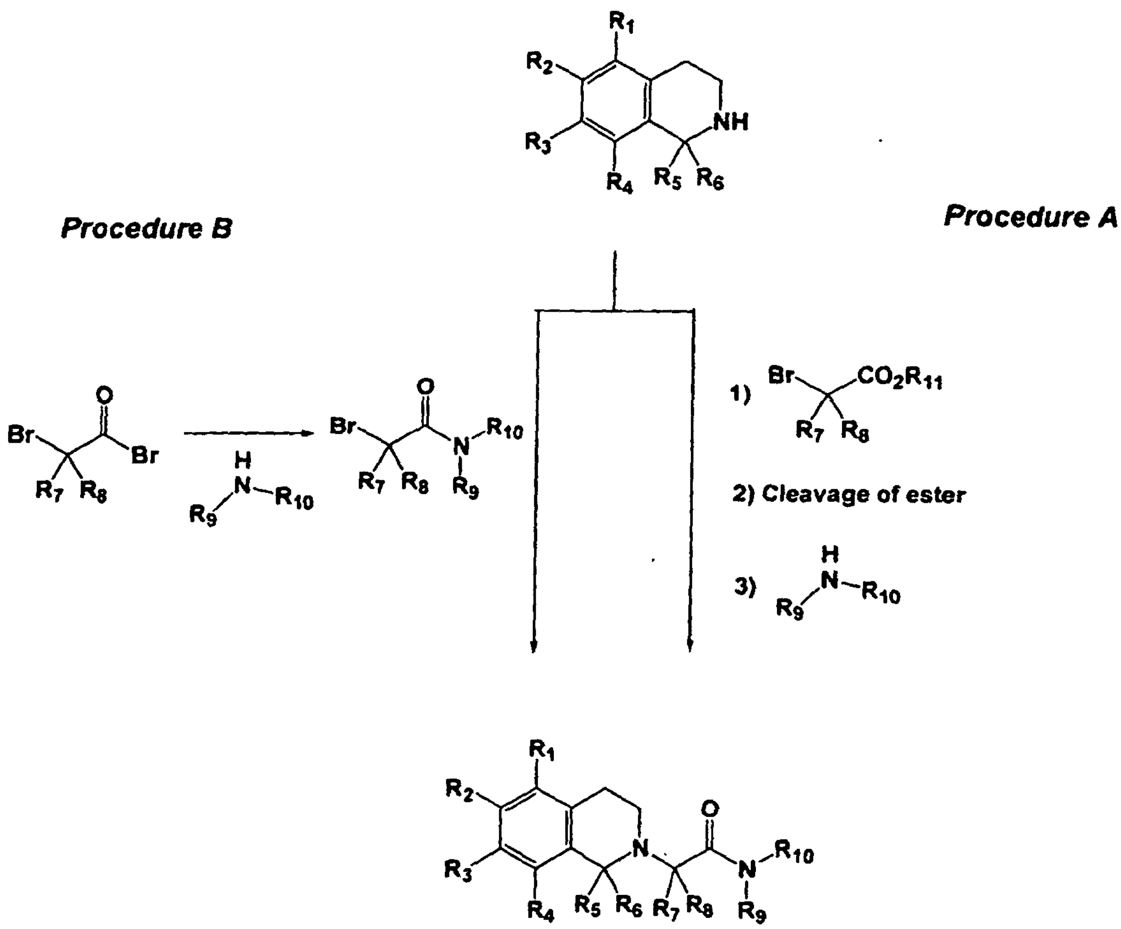


Scheme 2

Tetrahydroisoquinolines not commercially available might be prepared from the corresponding phenylethylamines by coupling with the desired carboxylic acid followed by treatment with $POCl_3$ and finally $NaBH_4$ (see experimental part).

Compounds of general formula (I) wherein one substituent of the 1-benzyl-tetrahydro-isoquinoline scaffold is a carbamoyloxy-, heteroaryloxy- or alkoxy-residue (not methoxy) are synthesized according to (*Scheme 3*). The benzyl-protected phenols are prepared by the procedure shown in (*Scheme 2*).

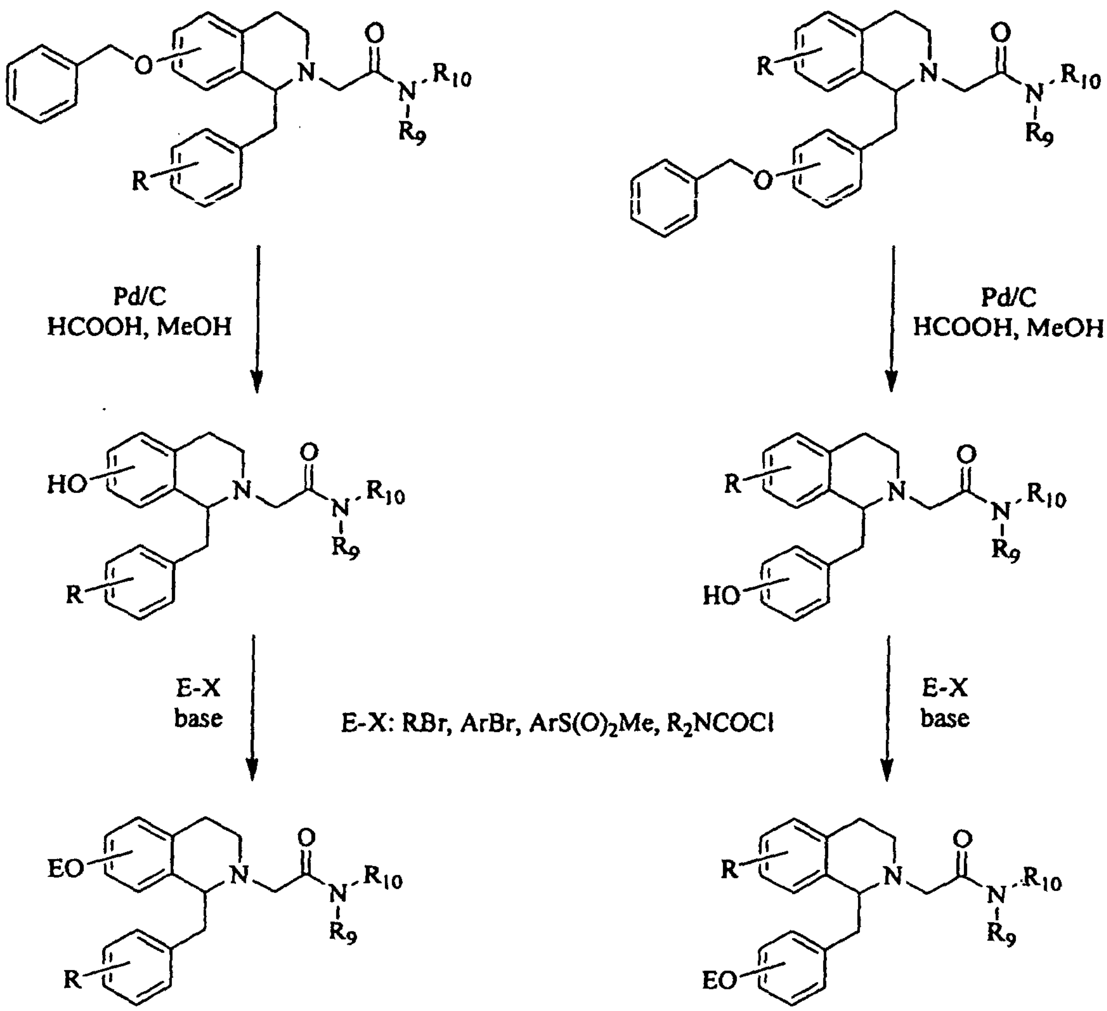


*Scheme 3*

In the case $R^5$ (general formula I) is a heterocyclyl-methyl substituent the final step is the substitution of a mesylate function with the corresponding nitrogen containing nucleophile according to (*Scheme 4*). The required starting material was synthesized by the same procedure as described earlier (*Scheme 2*).

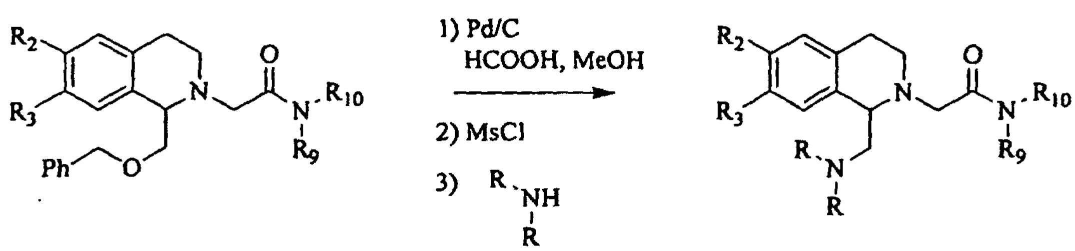


*Scheme 4*

Stereochemically pure compounds of general formula I are obtained by kinetic resolution of the tetrahydroisoquinoline (Corrodi H., Hardegger E., **Helv. Chim. Acta,** 1956, 39, 889-897) and coupling of the pure enantiomer with the amide linker according to *Scheme 2.*

## Experimental Section

### I. Biology

### Determination of $OX_1$ receptor antagonist activity

The $OX_1$ receptor antagonist activity of the compounds of formula (I) was determined in accordance with the following experimental method.

### Experimental method:

### Intracellular calcium measurements

Chinese hamster ovary (CHO) cells expressing the human orexin-1 receptor and the human orexin-2 receptor, respectively, were grown in culture medium (Ham F-12 with L-Glutamine) containing 300 μg/ml G418, 100 U/ml penicillin, 100 μg/ml streptomycin and 10 % inactivated foetal calf serum (FCS).

The cells were seeded at 80'000 cells / well into 96-well black clear bottom sterile plates (Costar) which had been precoated with 1% gelatine in Hanks' Balanced Salt Solution (HBSS). All reagents were from Gibco BRL.

The seeded plates were incubated overnight at 37°C in 5% $CO_2$.

Human orexin-A as an agonist was prepared as 1 mM stock solution in methanol:water (1:1), diluted in HBSS containing 0.1 % bovine serum albumin (BSA) and 2 mM HEPES for use in the assay at a final concentration of 10 nM.

Antagonists were prepared as 10 mM stock solution in DMSO, then diluted in 96-well plates, first in DMSO, then in HBSS containing 0.1 % bovine serum albumin (BSA) and 2 mM HEPES.

On the day of the assay, 100 μl of loading medium (HBSS containing 1% FCS, 2 mM HEPES, 5 mM probenecid (Sigma) and 3 μM of the fluorescent calcium indicator fluo-3 AM (1 mM stock solution in DMSO with 10% pluronic acid) (Molecular Probes) was added to each well.

The 96-well plates were incubated for 60 min at 37° C in 5% $CO_2$. The loading solution was then aspirated and cells were washed 3 times with 200 μl HBSS containing 2.5 mM probenecid, 0.1% BSA, 2 mM HEPES. 100 μl of that same buffer was left in each well. Within the Fluorescent Imaging Plate Reader (FLIPR, Molecular Devices), antagonists were added to the plate in a volume of 50 μl, incubated for 20 min and finally 100 μl of agonist was added. Fluorescence was measured for each well at 1 second intervals, and the height of each fluorescence peak was compared to the height of the fluorescence peak induced by 10 nM orexin-A with buffer in place of antagonist. For each antagonist, $IC_{50}$ value (the concentration of compound needed to inhibit 50 % of the agonistic response) was determined.

### II. Chemistry

The following examples illustrate the preparation of pharmacologically active compounds of the invention but do not at all limit the scope thereof. All temperatures are stated in °C.

All hydrochloride salts were prepared by dissolving the free-base in dichloromethane and treating with an excess of ethereal HCl (2M).

### General procedures:

A. General procedure A:

### 1-[(3,4-Dimethoxy-benzyl)-6,7-dimethoxy-3,4-dihydro-1H-isoquinolin-2-yl]-acetic acid benzyl ester

To a white suspension of 1-(4,5-dimethoxybenzyl)-6,7-dimethoxy-1,2,3,4-tetrahydroisoquinoline-hydrochloride (1g, 2.632 mmol) in a mixture of toluene/ DMF (9/1) (10 ml), were added triethylamine (1.1 ml, 7.896 mmol) and chlorobenzylacetate (440 μl, 2.895 mmol). The reaction mixture was stirred at reflux under argon for 20 h. After cooling, the mixture was diluted in $CH_2Cl_2$ and washed with water.

The aqueous phase was extracted twice with $CH_2Cl_2$, the combined organic phases were dried over anhydrous $MgSO_4$, filtered and concentrated to give a crude brown- orange oil. Flash chromatography (AcOEt/ hexane 1/1) gave 1.15 g (89%) of the title product as a brown-orange oil.

TLC (AcOEt/ hexane: 1/1): $R_f$ = 0.55.

LC-MS (MeCN/ $H_2O$: 1/1): $R_t$ = 4.16 min. *m/z* = 492 (M + 1).

**1-(3,4-Dimethoxybenzyl)-6,7-dimethoxy-(3,4-dihydro-1H-isoquinolin-2-yl)-acetic acid.**

To a solution of 1-[(3,4-dimethoxy-benzyl)-6,7-dimethoxy-3,4-dihydro-1H-isoquinolin-2-yl]-acetic acid benzyl ester (1.15g, 2.34 mmol) in dry AcOEt (20 ml) was added in one portion Pd-C 10% (250mg). The resulting black suspension was hydrogenated at normal pressure and room temperature for 20 h. The mixture was then filtered over celite and concentrated in vacuo to give brown crystals.

LC-MS (MeCN/ $H_2O$: 1/1): $R_t$ = 3.34 min. *m*/*z* = 402 (M + 1).

**Example 1**

**2-[1-(3,4-Dimethoxy-benzyl)-6,7-dimethoxy-3,4-dihydro-1*H*-isoquinolin-2-yl]-*N*-benzyl-acetamide**

To a solution of 1-(4,5-dimethoxybenzyl)-6,7-dimethoxy-(3,4-dihydro-1H-isoquinolin-2-yl)-acetic acid (100 mg, 0.249 mmol) in 4 ml of dry DMF, were added 129.6 mg (0.249 mmol) of PyBOP, 29.9 µl (0.226 mmol) of benzylamine and dropwise 110 µl (0.521 mmol) of diisopropylethylamine (Hünig's base). The mixture reaction was stirred at RT under argon for 20 h.The mixture was then dissolved in $CH_2Cl_2$ and washed with water. The aqueous phase was extracted twice with $CH_2Cl_2$, the combined organic extracts were dried over $MgSO_4$, filtered and concentrated to give a crude brown residue. Flash chromatography (AcOEt/ hexane 8/2) gave 126 mg (94%) of the title compound as a brown viscous oil.

TLC (AcOEt/ hexane: 8/2): $R_f$ = 0.65.

LC-MS (MeCN/ $H_2O$: 1/1): $R_t$ = 4.83 min. *m*/*z* = 491(M + 1).

**Example 2**

**2-[1-(3,4-Dimethoxy-benzyl)-6,7-dimethoxy-3,4-dihydro-1*H*-isoquinolin-2-yl]-*N*-naphthalen-1-ylmethyl-acetamide**

In analogy to Example 1 but for the final step, reaction of 1-(4,5-dimethoxybenzyl)-6,7-dimethoxy-(3,4-dihydro-1H-isoquinolin-2-yl)-acetic acid with 1-naphthlalenemethylamine to give the title compound as the free-base (brown viscous oil) and the hydrochloride salt (brown crystals)

- TLC (AcOEt): $R_f$ = 0.55.
- LC-MS (MeCN/$H_2O$: 1/1): $R_t$ = 5.97 min. *m*/*z* = 541 (M + 1).

**Example 3**

**2-[1-(3,4-Dimethoxy-benzyl)-6,7-dimethoxy-3,4-dihydro-1*H*-isoquinolin-2-yl]-*N*-(6-methoxy-naphthalen-2-ylmethyl)-acetamide**

In analogy to Example 1 but for the final step, reaction of 1-(4,5-dimethoxybenzyl)-6,7-dimethoxy-(3,4-dihydro-1H-isoquinolin-2-yl)-acetic acid with 6-methoxynaphthalene-2-methylamine to give the title compound as the free-base (brown oil).

- TLC (AcOEt): $R_f$ = 0.40 -LC-MS (MeCN/$H_2O$: 1/1): $R_t$ = 4.68 min. *m*/*z* = 57 1 (M + 1).

**2-(3-Bromo-4-methoxy-phenyl)-*N*-[2-(3,4-dimethoxy)-ethyl]-acetamide**

LC-MS (MeCN/ $H_2O$: 1/1): $R_t$ 4.28 min, 409 (M+1, ES+).

***N*-[2-(3,4-Dimethoxy-phenyl)-ethyl]-2-(3,4-dimethyl-phenyl)-acetamide**

LC-MS (MeCN/ $H_2O$: 1/1): $R_t$ 4.36 min, 328 (M+1, ES+).

**2-(3,4-Diethyl-phenyl)-*N*-[2-(3,4-dimethoxy)-ethyl]-acetamide**

LC-MS (MeCN/ $H_2O$: 1/1): $R_t$ 4.18 min, 356 (M+1, ES+).

**2-(3,4-Dichloro-phenyl)-*N*-[2-(3,4-dimethoxy)-ethyl]-acetamide**

LC-MS (MeCN/ $H_2O$: 1/1): $R_t$ 4.12 min, 369 (M+1, ES+).

**1-(4-Bromo-3-methoxy-benzyl)-6,7-dimethoxy-1,2,3,4-tetrahydro-isoquinoline**

LC-MS (MeCN/ $H_2O$: 1/1): $R_t$ 2.96 min, 393 (M+1, ES+).

**1-(3,4-Dimethyl-benzyl)-6,7-dimethoxy-1,2,3,4-tetrahydro-isoquinoline**

LC-MS (MeCN/ $H_2O$: 1/1): $R_t$ 3.19 min, 312 (M+1, ES+).

**1-(3,4-Diethyl-benzyl)-6,7-dimethoxy-1,2,3,4-tetrahydro-isoquinoline**

LC-MS (MeCN/ $H_2O$: 1/1): $R_t$ 2.25 min, 340 (M+1, ES+).

**1-(3,4-Dichloro-benzyl)-6,7-dimethoxy-1,2,3,4-tetrahydro-isoquinoline**

LC-MS (MeCN/ $H_2O$: 1/1): $R_t$ 3.20 min, 353 (M+1, ES+).

**1-[(3,4-Dimethoxy-benzyl)-6,7-dimethoxy-1,2,3,4-tetrahydro-naphthalen-2-yl]-phenyl- acetic acid methyl ester**

To a white suspension of 1-(4,5-dimethoxybenzyl)-6,7-dimethoxy-1,2,3,4-tetrahydroisoquinoline-hydrochloride (5g, 0.013 mol) in dry toluene (50 ml), were added triethylamine (5.5 ml, 0.039 mol) and bromo-phenyl-acetic acid methyl ester (2.07 ml, 0.013 mol). The reaction mixture was stirred at reflux under argon for 20 h. After cooling, the mixture was diluted in $CH_2Cl_2$ and washed with water. The aqueous phase was extracted twice with $CH_2Cl_2$, the combined organic phases were dried over anhydrous $MgSO_4$, filtered and concentrated to give a crude brown- orange oil. Flash chromatography (AcOEt/ hexane 1/1) gave 5.85 g (90%) of the title product as a brown-orange oil.
TLC (AcOEt/ hexane: 1/1): $R_f$ = 0.55.
LC-MS (MeCN/ $H_2O$: 1/1): $R_t$ 4.00 min and $R_t$ 4.36 min, 492 (M+1, ES+).

**1-[(3,4-Dimethoxy-benzyl)-6,7-dimethoxy-1,2,3,4-tetrahydro-naphthalen-2-yl]-phenyl- acetic acid**

To a solution of 1-[(3,4-Dimethoxy-henzyl)-6,7-dimethoxy-1,2,3,4-tetrahydro-naphthalen-2-yl]-phenyl- acetic acid methyl ester (5.85 g, 0.011 mmol) in a mixture dioxane/ MeOH (4/3) (160 ml) was added dropwise 2M $NaOH_{(aq)}$ (81 ml). The resulting mixture was stirred at RT for 20 h under nitrogen. The mixture was then concentrated in vacuo, combined with water and AcOEt. The aqueous phase was acidified until pH 1 with 2N HCl, extracted three times with with $CH_2Cl_2$, the combined organic phases were dried over anhydrous $MgSO_4$, filtered and concentrated to give the titled product (5.55 g, 97%) as yellow-green crystals.
LC-MS (MeCN/ $H_2O$: 1/1): $R_t$ 3.62 min and $R_t$ 3.65 min, 478 (M+1, ES+).

**Example 4**

***N*-benzyl-2-[1-(3,4-Dimethyl-benzyl)-6,7-dimethoxy-3,4-dihydro-1*H*-isoquinolin-2-yl]-acetamide:**

prepared by reaction of 1-(3,4-dimethyl-benzyl)-6,7-dimethoxy-1,2,3,4-tetrahydroisoquinoline and 2-bromoacetyl bromide with benzylamine.
LC-MS (MeCN/ $H_2O$: 1/1): $R_t$ = 4.35 min, 459 (M+1, ES+).

**Example 5**

**2-[1-(3,4-Dimethyl-benzyl)-6,7-dimethoxy-3,4-dihydro-1*H*-isoquinolin-2-yl]-*N*-pyridin-2-yl-acetamide:**

prepared by reaction of 1-(3,4-dimethyl-benzyl)-6,7-dimethoxy-1,2,3,4-tetrahydroisoquinoline and 2-bromoacetyl bromide with 2-picolylamine.
LC-MS (MeCN/ $H_2O$: 1/1): $R_t$ = 2.99 min, 460 (M+1, ES+).

**Example 6**

**2-[1-(3,4-Dimethyl-benzyl)-6,7-dimethoxy-3,4-dihydro-1*H*-isoquinolin-2-yl]-*N*-pyridin-3-yl-acetamide:**

prepared by reaction of 1-(3,4-dimethyl-benzyl)-6,7-dimethoxy-1,2,3,4-tetrahydroisoquinoline and 2-bromoacetyl bromide with 3-picolylamine.
LC-MS (MeCN/ $H_2O$: 1/1): $R_t$ = 2.61 min, 460 (M+1, ES+).

**Example 7**

***N*-benzyl-2-[1-(3,4-Diethyl-benzyl)-6,7-dimethoxy-3,4-dihydro-1*H*-isoquinolin-2-yl]-acetamide:**

prepared by reaction of 1-(3,4-diethyl-benzyl)-6,7-dimethoxy-1,2,3,4-tetrahydroisoquinoline and 2-bromoacetyl bromide with benzylamine.
LC-MS (MeCN/ $H_2O$: 1/1): $R_t$ = 4.35 min, 459 (M+1, ES+).

**Example 8**

**2-[1-(3,4-Diethyl-benzyl)-6,7-dimethoxy-3,4-dihydro-1*H*-isoquinolin-2-yl]-*N*-(pyridin-2-yl-methyl)-acetamide:**

prepared by reaction of 1-(3,4-diethyl-benzyl)-6,7-dimethoxy-1,2,3,4-tetrahydroisoquinoline and 2-bromoacetyl bromide with 2-picolylamine.
LC-MS (MeCN/ $H_2O$: 1/1): $R_t$ = 2.87 min, 488 (M+1, ES+).

**Example 9**

**2-[1-(3,4-Diethyl-benzyl)-6,7-dimethoxy-3,4-dihydro-1*H*-isoquinolin-2-yl]-*N*-(pyridin-3-yl-methyl)-acetamide:**

prepared by reaction of 1-(3,4-diethyl-benzyl)-6,7-dimethoxy-1,2,3,4-tetrahydroisoquinoline and 2-bromoacetyl bromide with 3-picolylamine.
LC-MS (MeCN/ $H_2O$: 1/1): $R_t$ = 2.85 min, 488 (M+1, ES+).

**Example 10**

**2-[1-(3,4-Diethyl-benzyl)-6,7-dimethoxy-3,4-dihydro-1*H*-isoquinolin-2-yl]-*N*-(pyridin-4-yl-methyl)-acetamide:**

prepared by reaction of 1-(3,4-diethyl-benzyl)-6,7-dimethoxy-1,2,3,4-tetrahydroisoquinoline and 2-bromoacetyl bromide with 4-picolylamine.
LC-MS (MeCN/ $H_2O$: 1/1): $R_t$ = 2.71 min, 488 (M+1, ES+).

**Example 11**

**2-[1-(3,4-Dichloro-benzyl)-6,7-dimethoxy-3,4-dihydro-1*H*-isoquinolin-2-yl]-*N*-(pyridin-2-yl-methyl)-acetamide:**

prepared by reaction of 1-(3,4-dichloro-benzyl)-6,7-dimethoxy-1,2,3,4-tetrahydroisoquinoline and 2-bromoacetyl bromide with 2-picolylamine.
LC-MS (MeCN/ $H_2O$: 1/1): $R_t$ = 3.72 min, 501 (M+1, ES+).

**Example 12**

**2-[1-(3,4-Dichloro-benzyl)-6,7-dimethoxy-3,4-dihydro-1*H*-isoquinolin-2-yl]-*N*-(pyridin-3-yl-methyl)-acetamide:**

prepared by reaction of 1-(3,4-dichloro-benzyl)-6,7-dimethoxy-1,2,3,4-tetrahydroisoquinoline and 2-bromoacetyl bromide with 3-picolylamine.
LC-MS (MeCN/ $H_2O$: 1/1): $R_t$ = 3.29 min, 501 (M+1, ES+).

**B Coupling of 1,2,3,4-Tetrahydroisoquinolines with 2-Bromoacetamides**

**B.1 Starting materials: Synthesis of 1,2,3,4-Tetrahydroisoquinoline derivatives:**

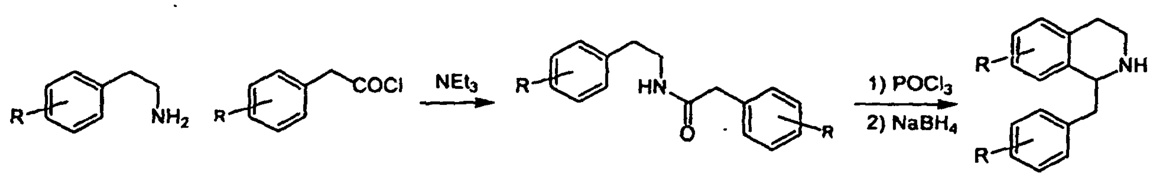


**B.1.1 Synthesis of the phenylethylamides:**

**Procedure I:**

A solution of the respective phenylethylamine (80 mmol) and of triethylamine (90 mmol) in THF (120 mL) was cooled to 0°C and treated portionwise with the respective acetyl chloride (80 mmol). After stirring for 10 min at 0°C and for 14 h at room temperature a sat, aqueous $NaHCO_3$ solution was added, the phases were separated and the aqueous phase was extracted three times with ethyl acetate (150 mL). The solvent was removed in vacuo and the residue was either recrystalized from toluene or purified by flash chromatography to give the following amides:

**N-[2-(3-Methoxy-phenyl)-ethyl]-3,4-dimethoxyphenyl-acetamide:**

prepared by reaction of 3-methoxyphenylethylamine with 3,4-dimethoxyphenyl acetyl chloride.
LC-MS: rt = 4.1 min, 330 (M+1, ES+).

**N-[2-(3,4-Dimethoxy-phenyl)-ethyl]-phenyl-acetamide:**

prepared by reaction of 3,4-dimethoxyphenylethylamine with phenyl acetyl chloride.

**N-[2-(3,4-Dimethoxy-phenyl)-ethyl]-3-methoxyphenyl-acetamide:**

prepared by reaction of 3,4-dimethoxyphenylethylamine with 3-methoxyphenyl acetyl chloride.
LC-MS: rt = 4.0 min, 330 (M+1, ES+).

**N-[2-(3,4-Dimethoxy-phenyl)-ethyl)-4-methoxyphenyl-acetamide:**

prepared by reaction of 3,4-dimethoxyphenylethylamine with 4-methoxyphenyl acetyl chloride.
LC-MS: rt = 4.0 min, 330 (M+1, ES+).

**N-[2-(3,4-Dimethoxy-phenyl)-ethyl]-2,5-dimethoxyphenyl-acetamide:**

prepared by reaction of 3,4-dimethoxyphenylethylamine with 2,5-dimethoxyphenyl acetyl chloride.
LC-MS: rt= 4.1 min, 360 (M+1, ES+).

**N-[2-(2,5-Dimethoxy-phenyl)-ethyl]-3,4-dimethoxyphenyl-acetamide:**

prepared by reaction of 2,5-dimethoxyphenylethylamine with 3,4-dimethoxyphenyl acetyl chloride.
LC-MS: rt = 4.2 min, 360 (M+1, ES+).

**N-[2-(3,4-Dimethoxy-phenyl)-ethyl]-3-phenyl-propionamide:**

prepared by reaction of 3,4-dimethoxyphenylethylamine with 3-phenyl propionyl chloride.
LC-MS: rt = 4.2 min, 314 (M+1, ES+).

**N-[2-(3,4-Dimethoxy-phenyl)-ethyl]-2-phenyl-butyramide:**

prepared by reaction of 3,4-dimethoxyphenylethylamine with 2-Phenylbutyryl chloride.

$R_f$= 0.21 (ethyl acetate/heptane 1/1)

**N-[2-(2,5-Dimethoxy-phenyl)-ethyl]-diphenyl-acetamide:**

prepared by reaction of 2,5-dimethoxyphenylethylamine with diphenylacetyl chloride.

LC-MS: rt = 5.3 min, 376 (M+1, ES+).

**N-[2-(2,5-Dimethoxy-phenyl)-ethyl]-2,5-dimethoxyphenyl-acetamide:**

prepared by reaction of 2,5-dimethoxyphenylethylamine with 2,5-dimethoxyphenyl acetyl chloride.

LC-MS: rt = 4.6 min, 360 (M+1, ES+).

**N-[2-(3,4-Dimethoxy-phenyl)-ethyl]-4-chlorophenyl-acetamide:**

prepared by reaction of 3,4-dimethoxyphenylethylamine with 4-chlorophenyl acetyl chloride.

LC-MS: rt = 4.4 min, 334 (M+1, ES+).

**N-[2-(2,5-Dimethoxy-phenyl)-ethyl]-phenyl-acetamide:**

prepared by reaction of 2,5-dimethoxyphenylethylamine with phenylacetyl chloride.

LC-MS: rt = 4.5 min, 300 (M+1, ES+).

**N-[2-(3-methoxy-4-isopropoxy -phenyl)-ethyl]-3,4-dimethoxyphenyl-acetamide:**

prepared by reaction of 3-methoxy-4-isopropoxyphenylethylamine with 3,4-dimethoxyphenyl acetyl chloride.

LC-MS: rt = 4.2 min, 388 (M+1, ES+).

**N-[2-(3,4,5-Trimethoxy -phenyl)-ethyl]-3,4-dimethoxyphenyl-acetamide:**

prepared by reaction of 3,4,5-trimethoxyphenylethylamine with 3,4-dimethoxyphenyl acetyl chloride.

LC-MS: rt = 3.8 min, 390 (M+1, ES+).

**N-[2-(2,3,4-Trimethoxy -phenyl)-ethyl]-3,4-dimethoxyphenyl-acetamide:**

prepared by reaction of 2,3,4-trimethoxyphenylethylamine with 3,4-dimethoxyphenyl acetyl chloride.

LC-MS: rt = 4.1 min, 390 (M+1, ES+).

**N-(2-(3,5-Dimethoxy -phenyl)-ethyl]-3,4-dimethoxyphenyl-acetamide:**

prepared by reaction of 3,5-trimethoxyphenylethylamine with 3,4-dimethoxyphenyl acetyl chloride.

LC-MS: rt = 4.2 min, 360 (M+1, ES+).

**N-[2-(3-Benzyloxy-4-methoxy-phenyl)-ethyl]-3,4-dimethoxyphenyl-acetamide:**

prepared by reaction of 3-benzyloxy-4-methoxyphenylethylamine with 3,4-dimethoxyphenyl acetyl chloride.

LC-MS: rt = 4.7 min, 436 (M+1, ES+), 434 (M-1, ES-).

**N-[2-(4-Benzyloxy-3-methoxy-phenyl)-ethyl]-3,4-dimethoxyphenyl-acetamide:**

prepared by reaction of 4-benzyloxy-3-methoxyphenylethylamine with 3,4-dimethoxyphenyl acetyl chloride.

LC-MS: rt = 4.8 min, 436 (M+1, ES+).

**N-[2-(2-Benzyloxy-5-methoxy-phenyl)-ethyl]-3,4-dimethoxyphenyl-acetamide:**

prepared by reaction of 2-benzyloxy-5-methoxyphenylethylamine with 3,4-dimethoxyphenyl acetyl chloride.

LC-MS: rt = 4.8 min, 436 (M+1, ES+).

**N-[2-(5-Benzyloxy-2-methoxy-phenyl)-ethyl]-3,4-dimethoxyphenyl-acetamide:**

prepared by reaction of 5-benzyloxy-2-methoxyphenylethylamine with 3,4-dimethoxyphenyl acetyl chloride.
LC-MS: rt = 4.9 min, 436 (M+1, ES+).

**N-[2-(3,4-Dimethoxy-phenyl)-ethyl]-benzyloxy-acetamide:**

prepared by reaction of 3,4-dimethoxyphenylethylamine with benzyloxy acetyl chloride.
LC-MS: rt = 4.2 min, 330 (M+1, ES+).

**Procedure II:**

A solution of the respective phenylethylamine (25.0 mmol) and the respective phenylacetic acid (25.0 mmol) in 100 mL toluene was refluxed for 24 h in the presence of a Dean-Stark. The solvent was removed in vacuo and the residue was either recrystalized from toluene or purified by flash chromatography to give the following amides:

**N-[2-(3,4-Dimethoxy-phenyl)-ethyl]-3,4-methylenedioxyphenyl-acetamide:**

prepared by reaction of 3,4-dimethoxyphenylethylamine and 3,4-methylenedioxyphenylacetic acid.
LC-MS: rt = 4.1 min, 344 (M+1, ES+).

**N-[2-(3,4-Dimethoxy-phenyl)-ethyl]-4-dimethylaminophenyl-acetamide:**

prepared by reaction of 3,4-dimethoxyphenylethylamine and 4-dimethyl-aminophenylacetic acid.
LC-MS: rt = 3.1 min, 343 (M+1, ES+).

**N-[2-(3,4-Dimethoxy-phenyl)-ethyl]-4-fluorophenyl-acetamide:**

prepared by reaction of 3,4-dimethoxyphenylethylamine and 4-fluorophenyl-acetic acid.
LC-MS: rt = 4.1 min, 318 (M+1, ES+).

**N-[2-(3,4-Dimethoxy-phenyl)-ethyl]-3,4-difluorophenyl-acetamide:**

prepared by reaction of 3,4-dimethoxyphenylethylamine and 3,4-difluorophenylacetic acid.
LC-MS: rt = 4.2 min, 336 (M+1, ES+).

**N-[2-(3,4-Dimethoxy-phenyl)-ethyl]-3,4,5-trimethoxyphenyl-acetamide:**

prepared by reaction of 3,4-dimethoxyphenylethylamine and 3,4,5-trimethoxyphenylacetic acid.
LC-MS: rt = 3.8 min, 390 (M+1, ES+).

**N-[2-(3,4-Dimethoxy-phenyl)-ethyl]-2,3,4-trimethoxyphenyl-acetamide:**

prepared by reaction of 3,4-dimethoxyphenylethylamine and 2,3,4-trimethoxyphenylacetic acid.
LC-MS: rt = 4.1 min, 390 (M+1, ES+).

**N-[2-(3,4-Dimethoxy-phenyl)-ethyl]-naphthalen-2-yl-acetamide:**

prepared by reaction of 3,4-dimethoxyphenylethylamine and 2-naphthylacetic acid.
LC-MS: rt = 4.9 min, 350 (M+1, ES+).

**N-[2-(2,5-Dimethoxy-phenyl)-ethyl]-3,4-methylenedioxyphenyl-acetamide:**

prepared by reaction of 2,5-dimethoxyphenylethylamine and 3,4-methylene-dioxyphenylacetic acid.
LC-MS: rt = 4.3 min, 344 (M+1, ES+).

**N-[2-(3,4-Dimethoxy-phenyl)-ethyl]-4-hydroxy-3-methoxy-phenyl-acetamide:**

prepared by reaction of 3,4-dimethoxyphenylethylamine and 4-hydroxy-3-methoxy-phenylacetic acid.

LC-MS: rt = 3.6 min, 346 (M+1, ES+), 344 (M-1, ES-).

**N-[2-(3,4-Dimethoxy-phenyl)-ethyl]-3-benzyloxy-4-methoxy-phenyl-acetamide:**

prepared by reaction of 3,4-dimethoxyphenylethylamine and 3-benzyloxy-4-methoxy-phenylacetic acid.

LC-MS: rt = 4.6 min, 436 (M+1, ES+), 434 (M-1, ES-).

**B.1.2. Synthesis of 1,2,3,4-Tetrahydroisoquinolines via Bischler-Napieralski-reaction (general procedure):**

To a suspension of the respective acetamide (60 mmol) in acetonitrile (100 mL) was added phosphorus oxychloride (16.2 mL, 177 mmol). The mixture was heated to reflux for 6 h and the solvent was removed in vacuo. The resulting oil was taken up in MeOH (70 mL), evaporated to dryness, dissolved in MeOH (130 mL) and cooled to 0°C. $NaBH_4$ was added in small (!) portions and the reaction mixture was stirred for 14 h. The solvent was removed in vacuo, dichloromethane (150 mL) and water (100 mL) were added, the phases were separated and the aqueous phase was extracted three times with dichloromethane (100 mL). The combined organic phases were concentrated in vacuo to give the following tetrahydroisoquinolines, which were purified either by flash chromatography or by crystallization as hydrochloride salt:

**1-(3,4-Dimethoxy-benzyl)-6-methoxy-1,2,3,4-tetrahydroisoquinoline:**

prepared by cyclisation of N-[2-(3-Methoxy-phenyl)-ethyl]-3,4-dimethoxyphenyl-acetamide.

LC-MS: rt= 3.1 min, 314 (M+1, ES+).

**1-Benzyl-6,7-dimethoxy-1,2,3,4-tetrahydroisoquinoline:**

prepared by cyclisation of N-[2-(3,4-Dimethoxy-phenyl)-ethyl]-phenyl acetamide. $R_f$ (dichloromethane/methanol 5/1) = 0.51.

LC-MS: rt = 3.1 min, 284 (M+1, ES+).

**1-(3-Methoxy-benzyl)-6,7-dimethoxy-1,2,3,4-tetrahydroisoquinoline:**

prepared by cyclisation of N-[2-(3,4-Dimethoxy-phenyl)-ethyl]-3-methoxyphenyl acetamide.

LC-MS: rt = 3.0 min, 314 (M+1, ES+).

**1-(4-Methoxy-benzyl)-6,7-dimethoxy-1,2,3,4-tetrahydroisoquinoline:**

prepared by cyclisation of N-[2-(3,4-Dimethoxy-phenyl)-ethyl]-4-methoxyphenyl acetamide.

LC-MS: rt = 3.0 min, 314 (M+1, ES+).

**1-(2,5-Dimethoxy-benzyl)-6,7-dimethoxy-1,2,3,4-tetrahydroisoquinoline:**

prepared by cyclisation of N-[2-(3,4-Dimethoxy-phenyl)-ethyl]-2,5-dimethoxy-phenyl acetamide.

LC-MS: rt = 3.2 min, 344 (M+1, ES+).

**1-(3,4-Dimethoxy-benzyl)-5,8-dimethoxy-1,2,3,4-tetrahydroisoquinoline:**

prepared by cyclisation of N-[2-(2,5-Dimethoxy-phenyl)-ethyl]-3,4-dimethoxy-phenyl acetamide.

LC-MS: rt = 3.3 min, 344 (M+1, ES+).

**1-(2-Phenyl-ethyl)-6,7-dimethoxy-1,2,3,4-tetrahydroisoquinoline:**

prepared by cyclisation of N-[2-(3,4-Dimethoxy-phenyl)-ethyl]-3-phenyl-propionamide.

LC-MS: rt = 3.2 min, 298 (M+1, ES+).

**1-(1-Phenyl-propyl)-6,7-dimethoxy-1,2,3,4-tetrahydroisoquinoline:**

prepared by cyclisation of N-[2-(3,4-Dimethoxy-phenyl)-ethyl]-2-phenyl-butyramide.

LC-MS: rt = 3.3 min, 312 (M+1, ES+).

**1-(Diphenylmethyl)-5,8-dimethoxy-1,2,3,4-tetrahydroisoquinoline:**

prepared by cyclisation of N-[2-(2,5-Dimethoxy-phenyl)-ethyl]-diphenyl acetamide.

LC-MS: rt = 3.7 min, 360 (M+1, ES+).

**1-(2,5-Dimethoxy-benzyl)-5,8-dimethoxy-1,2,3,4-tetrahydroisoquinoline:**

prepared by cyclisation of N-[2-(2,5-Dimethoxy-phenyl)-ethyl]-2,5-dimethoxy-phenyl acetamide.

LC-MS: rt = 3.6 min, 344 (M+1, ES+).

**1-(4-Chloro-benzyl)-6,7-dimethoxy-1,2,3,4-tetrahydroisoquinoline:**

prepared by cyclisation of N-[2-(3,4-Dimethoxy-phenyl)-ethyl]-4-chloro-phenyl acetamide.

LC-MS: rt = 3.2 min, 318 (M+1, ES+).

**1-Benzyl-5,8-dimethoxy-1,2,3,4-tetrahydroisoquinoline:**

prepared by cyclisation of N-[2-(2,5-Dimethoxy-phenyl)-ethyl]-phenyl acetamide.

LC-MS: rt = 3.4 min, 284 (M+1, ES+).

**1-(3,4-Dimethoxy-benzyl)-6-methoxy-7-isopropoxy-1,2,3,4-tetrahydro-isoquinoline:**

prepared by cyclisation of N-[2-(3-Methoxy-4-isopropoxy-phenyl)-ethyl]-3,4-dimethoxy-phenyl acetamide.

LC-MS: rt = 3.32 min, 372 (M+1, ES+).

**1-(3,4-Methylenedioxy-benzyl)-6,7-dimethoxy-1,2,3,4-tetrahydro-isoquinoline:**

prepared by cyclisation of N-[2-(3,4-Dimethoxy-phenyl)-ethyl]-3,4-methylenedioxy-phenyl acetamide.

LC-MS: rt = 3.0 min, 328 (M+1, ES+).

**1-(4-Dimethylamino-benzyl)-6,7-dimethoxy-1,2,3,4-tetrahydro-isoquinoline:**

prepared by cyclisation of N-[2-(3,4-Dimethoxy-phenyl)-ethyl]-4-dimethyl-amino-phenyl acetamide.

LC-MS: rt = 2.6 min, 327 (M+1, ES+).

**1-(4-Fluoro-benzyl)-6,7-dimethoxy-1,2,3,4-tetrahydroisoquinoline:**

prepared by cyclisation of N-[2-(3,4-Dimethoxy-phenyl)-ethyl]-4-fluoro-phenyl acetamide.

LC-MS: rt = 3.1 min, 302 (M+1, ES+).

**1-(3,4-Difluoro-benzyl)-6,7-dimethoxy-1,2,3,4-tetrahydroisoquinoline:**

prepared by cyclisation of N-[2-(3,4-Dimethoxy-phenyl)-ethyl]-3,4-difluoro-phenyl acetamide.

LC-MS: rt = 3.1 min, 320 (M+1, ES+).

**1-(3,4,5-Trimethoxy-benzyl)-6,7-dimethoxy-1,2,3,4-tetrahydro-isoquinoline:**

prepared by cyclisation of N-[2-(3,4-Dimethoxy-phenyl)-ethyl]-3,4,5-trimethoxy-phenyl acetamide.

LC-MS: rt = 3.0 min, 374 (M+1, ES+).

**1-(3,4-Dimethoxy-benzyl)-6,7,8-trimethoxy-1,2,3,4-tetrahydro-isoquinoline:**

prepared by cyclisation of N-[2-(3,4,5-Trimethoxy-phenyl)-ethyl]-3,4-dimethoxy-phenyl acetamide.

LC-MS: rt = 3.2 min, 374 (M+1, ES+).

**1-(3,4-Dimethoxy-benzyl)-5,6,7-trimethoxy-1,2,3,4-tetrahydro-isoquinoline:**

prepared by cyclisation of N-[2-(2,3,4-Trimethoxy-phenyl)-ethyl]-3,4-dimethoxy-phenyl acetamide.

LC-MS: rt = 3.2 min, 374 (M+1, ES+).

**1-(3,4-Dimethoxy-benzyl)-6,8-dimethoxy-1,2,3,4-tetrahydro-isoquinoline:**

prepared by cyclisation of N-[2-(3,5-Dimethoxy-phenyl)-ethyl]-3,4-dimethoxy-phenyl acetamide.

LC-MS: rt = 3.5 min, 344 (M+1, ES+).

**1-(2,3,4-Trimethoxy-benzyl)-6,7-dimethoxy-1,2,3,4-tetrahydro-isoquinoline:**

prepared by cyclisation of N-[2-(3,4-Dimethoxy-phenyl)-ethyl]-2,3,4-trimethoxy-phenyl acetamide.

LC-MS: rt = 3.2 min, 374 (M+1, ES+).

**1-(Naphthalen-2-yl-methyl)-6,7-dimethoxy-1,2,3,4-tetrahydro-isoquinoline:**

prepared by cyclisation of N-[2-(3,4-Dimethoxy-phenyl)-ethyl]-naphthalen-2-yl acetamide.

LC-MS: rt = 3.6 min, 334 (M+1, ES+).

**1-(3,4-Methylenedioxy-benzyl)-5,8-dimethoxy-1,2,3,4-tetrahydro-isoquinoline:**

prepared by cyclisation of N-[2-(2,5-Dimethoxy-phenyl)-ethyl]-3,4-methylenedioxy-phenyl acetamide.

LC-MS: rt = 3.2 min, 328 (M+1, ES+).

**1-(3,4-Dimethoxy-benzyl)-6-benzyloxy-7-methoxy-1,2,3,4-tetrahydro-isoquinoline:**

prepared by cyclisation of N-[2-(3-Benzyloxy-4-methoxy-phenyl)-ethyl]-3,4-dimethoxy-phenyl acetamide.

LC-MS: rt = 3.7 min, 420 (M+1, ES+).

**1-(3,4-Dimethoxy-benzyl)-7-benzyloxy-6-methoxy-1,2,3,4-tetrahydro-isoquinoline:**

prepared by cyclisation of N-[2-(4-Benzyloxy-3-methoxy-phenyl)-ethyl]-3,4-dimethoxy-phenyl acetamide.

LC-MS: rt = 3.6 min, 420 (M+1, ES+).

**1-(3,4-Dimethoxy-benzyl)-5-benzyloxy-8-methoxy-1,2,3,4-tetrahydro-isoquinoline:**

prepared by cyclisation of N-[2-(2-Benzyloxy-5-methoxy-phenyl)-ethyl]-3,4-dimethoxy-phenyl acetamide.

LC-MS: rt = 4.1 min, 420 (M+1, ES+).

**1-(3,4-Dimethoxy-benzyl)-8-benzyloxy-5-methoxy-1,2,3,4-tetrahydro-isoquinoline:**

prepared by cyclisation of N-[2-(5-Benzyloxy-2-methoxy-phenyl)-ethyl]-3,4-dimethoxy-phenyl acetamide.

LC-MS: rt = 3.9 min, 420 (M+1, ES+).

**1-(4-Hydroxy-3-methoxy-benzyl)-6,7-dimethoxy-1,2,3,4-tetrahydro-isoquinoline:**

prepared by cyclisation of N-[2-(3,4-dimethoxy-phenyl)-ethyl]-4-hydroxy-3-methoxy-phenyl acetamide.

LC-MS: rt = 2.8 min, 330 (M+1, ES+).

**1-(3-Benzyloxy-4-methoxy-benzyl)-6,7-dimethoxy-1,2,3,4-tetrahydro-isoquinoline:**

prepared by cyclisation of N-[2-(3,4-dimethoxy-phenyl)-ethyl]-3-benzyloxy-4-methoxy-phenyl acetamide.

LC-MS: rt = 3.6 min, 420 (M+1, ES+).

**1-Benzyloxymethyl-6,7-dimethoxy-1,2,3,4-tetrahydro-isoquinoline:**

prepared by cyclisation of N-[2-(3,4-dimethoxy-phenyl)-ethyl]-benzyloxy-acetamide.

**B.2. Alkylation of 1,2,3,4-Tetrahydroisoquinolines with 2-Bromo-acetamides (general procedure)**

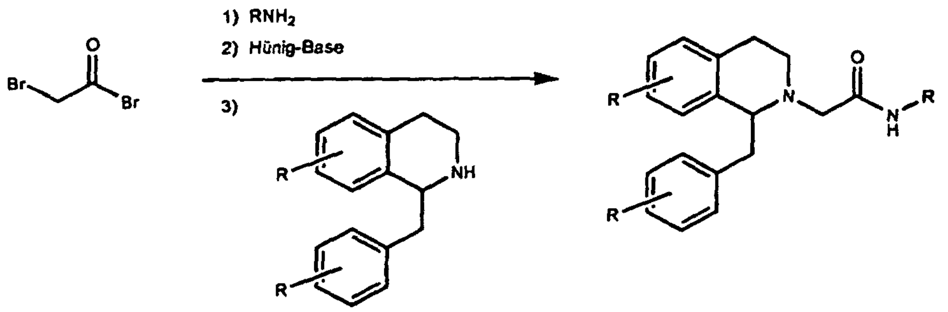


At-15°C a solution of the respective amine in THF (250 µL, 0.40 M) was added to a solution of 2-bromoacetyl bromide in THF (500 µL, 0.20 M). The reaction mixture was treated with a solution of diisopropylethylamine in THF (250 µL, 2.0 M), allowed to warm up to room temperature and stirred for 30 min. A solution of the respective tetrahydroisoquinoline in DMSO (500 µL, 0.20 M) was added and the mixture was heated to 75°C for 18 h. After cooling to room temperature water (2.0 mL) and ethyl acetate (2.0 mL) were added, the phases were separated and the aqueous phase was extracted two times with ethyl acetate. The combined organic phases were concentrated in vacuo to give the following tetrahydroisoquinoline derivatives:

Example 13

**2-(1-Benzyl-3,4-dihydro-1*H*-isoquinolin-2-yl)-*N*-(2-methyl-benzyl)-acetamide:**

prepared by reaction of 1-Benzyl-1,2,3,4-tetrahydroisoquinoline and 2-bromoacetyl bromide with 2-methylbenzylamine

LC-MS: rt = 4.6 min, 385 (M+1, ES+).

**Example 14**

**2-(1-Benzyl-3,4-dihydro-1*H*-isoquinolin-2-yl)-*N*-(2-chloro-benzyl)-acetamide:**

prepared by reaction of 1-Benzyl-1,2,3,4-tetrahydroisoquinoline and 2-bromoacetyl bromide with 2-chlorobenzylamine

LC-MS: rt = 4.7 min, 405 (M+1, ES+).

**Example 15**

**2-(1-Benzyl-3,4-dihydro-1*H*-isoquinolin-2-yl)-*N*-(1-naphthalen-1-yl-ethyl)-acetamide:**

prepared by reaction of 1-Benzyl-1,2,3,4-tetrahydroisoquinoline and 2-bromoacetyl bromide with 1-naphthaleneethylamine

LC-MS: rt = 4.7 and 4.8 min, 435 (M+1, ES+).

**Example 16**

**2-(1-Benzyl-3,4-dihydro-1*H*-isoquinolin-2-yl)-*N*-benzyl-*N*-methyl-acetamide:**

prepared by reaction of 1-Benzyl-1,2,3,4-tetrahydroisoquinoline and 2-bromoacetyl bromide with N-benzylmethylamine

LC-MS: rt = 3.9 min, 385 (M+1, ES+).

**Example 17**

**2-[1-(3,4-Dimethoxy-benzyl)-6-methoxy-3,4-dihydro-1*H*-isoquinolin-2-yl]-*N*-(2-methoxy-benzyl)-acetamide:**

prepared by reaction of 1-(3,4-dimethoxy-benzyl)-6-methoxy-1,2,3,4-tetrahydroisoquinoline and 2-bromoacetyl bromide with 2-methoxybenzylamine

LC-MS: rt = 4.0 min, 491 (M+1, ES+).

**Example 18**

**2-[1-(3,4-Dimethoxy-benzyl)-6-methoxy-3,4-dihydro-1*H*-isoquinolin-2-yl]-*N*-benzyl-acetamide:**

prepared by reaction of 1-(3,4-dimethoxy-benzyl)-6-methoxy-1,2,3,4-tetrahydroisoquinoline and 2-bromoacetyl bromide with benzylamine

LC-MS: rt = 3.9 min, 461 (M+1, ES+).

**Example 19**

**2-[1-(3,4-Dimethoxy-benzyl)-6-methoxy-3,4-dihydro-1*H*-isoquinolin-2-yl]-*N*-(4-methoxy-benzyl)-acetamide:**

prepared by reaction of 1-(3,4-dimethoxy-benzyl)-6-methoxy-1,2,3,4-tetrahydroisoquinoline and 2-bromoacetyl bromide with 4-methoxybenzylamine

LC-MS: rt = 3.9 min, 491 (M+1, ES+).

**Example 20**

**2-[1-(3,4-Dimethoxy-benzyl)-6-methoxy-3,4-dihydro-1*H*-isoquinolin-2-yl]-*N*-(naphthalen-1-yl-methyl)-acetamide:**

prepared by reaction of 1-(3,4-dimethoxy-benzyl)-6-methoxy-1,2,3,4-tetrahydroisoquinoline and 2-bromoacetyl bromide with 1-napthalenemethylamine

LC-MS: rt = 4.3 min, 511 (M+1, ES+).

**Example 21**

**2-[1-(3,4-Dimethoxy-benzyl)-6-methoxy-3,4-dihydro-1*H*-isoquinolin-2-yl]-*N*-(3-methyl-benzyl)-acetamide:**

prepared by reaction of 1-(3,4-dimethoxy-benzyl)-6-methoxy-1,2,3,4-tetrahydroisoquinoline and 2-bromoacetyl bromide with 3-methylbenzylamine

LC-MS: rt = 4.1 min, 475 (M+1, ES+).

**Example 22**

**2-[1-(3,4-Dimethoxy-benzyl)-6-methoxy-3,4-dihydro-1*H*-isoquinolin-2-yl]-*N*-(pyridin-3-yl-methyl)-acetamide:**

prepared by reaction of 1-(3,4-dimethoxy-benzyl)-6-methoxy-1,2,3,4-tetrahydroisoquinoline and 2-bromoacetyl bromide with 3-piconylamine

LC-MS: rt = 3.1 min, 462 (M+1, ES+).

**Example 23**

**2-[1-(3,4-Dimethoxy-benzyl)-6-methoxy-3,4-dihydro-1*H*-isoquinolin-2-yl]-*N*-(pyridin-4-yl-methyl)-acetamide:**

prepared by reaction of 1-(3,4-dimethoxy-benzyl)-6-methoxy-1,2,3,4-tetrahydroisoquinoline and 2-bromoacetyl bromide with 4-piconylamine

LC-MS: rt = 3.1 min, 462 (M+1, ES+).

**Example 24**

**2-[1-(3,4-Dimethoxy-benzyl)-6-methoxy-3,4-dihydro-1*H*-isoquinolin-2-yl]-*N*-(2-fluoro-benzyl)-acetamide:**

prepared by reaction of 1-(3,4-dimethoxy-benzyl)-6-methoxy-1,2,3,4-tetrahydroisoquinoline and 2-bromoacetyl bromide with 2-fluorobenzylamine

LC-MS: rt = 4.0 min, 479 (M+1, ES+).

**Example 25**

**2-(1-Benzyl-6,7-dimethoxy-3,4-dihydro-1*H*-isoquinolin-2-yl)-*N*-benzyl-acetamide:**

prepared by reaction of 1-benzyl-6,7-dimethoxy-1,2,3,4-tetrahydroisoquinoline and 2-bromoacetyl bromide with benzylamine

LC-MS: rt = 3.9 min, 431 (M+1, ES+).

**Example 26**

**2-(1-Benzyl-6,7-dimethoxy-3,4-dihydro-1*H*-isoquinolin-2-yl)-*N*-(pyridin-3-yl-methyl)-acetamide:**

prepared by reaction of 1-benzyl-6,7-dimethoxy-1,2,3,4-tetrahydroisoquinoline and 2-bromoacetyl bromide with 3-piconylamine

LC-MS: rt = 3.0 min, 432 (M+1, ES+).

**Example 37**

**2-(1-Benzyl-6,7-dimethoxy-3,4-dihydro-1*H*-isoquinolin-2-yl)-*N*-(2-methyl-benzyl)-acetamide:**

prepared by reaction of 1-benzyl-6,7-dimethoxy-1,2,3,4-tetrahydroisoquinoline and 2-bromoacetyl bromide with 2-methylbenzylamine

LC-MS: rt = 4.1 min, 445 (M+1, ES+).

**Example 38**

**2-(1-Benzyl-6,7-dimethoxy-3,4-dihydro-1*H*-isoquinolin-2-yl)-*N*-(2,5-difluoro-benzyl)-acetamide:**

prepared by reaction of 1-benzyl-6,7-dimethoxy-1,2,3,4-tetrahydroisoquinoline and 2-bromoacetyl bromide with 2,5-difluorobenzylamine

LC-MS: rt = 4.1 min, 467 (M+1, ES+).

**Example 39**

**2-(1-Benzyl-6,7-dimethoxy-3,4-dihydro-1*H*-isoquinolin-2-yl)-*N*-(4-fluoro-benzyl)-acetamide:**

prepared by reaction of 1-benzyl-6,7-dimethoxy-1,2,3,4-tetrahydroisoquinoline and 2-bromoacetyl bromide with 4-fluorobenzylamine

LC-MS: rt = 4.0 min, 449 (M+1, ES+).

**Example 40**

**2-(1-Benzyl-6,7-dimethoxy-3,4-dihydro-1*H*-isoquinolin-2-yl)-*N*-(2-chloro-benzyl)-acetamide:**

prepared by reaction of 1-benzyl-6,7-dimethoxy-1,2,3,4-tetrahydroisoquinoline and 2-bromoacetyl bromide with 2-chlorobenzylamine

LC-MS: rt = 4.2 min, 465 (M+1, ES+).

**Example 41**

**2-(1-Benzyl-6,7-dimethoxy-3,4-dihydro-1*H*-isoquinolin-2-yl)-*N*-(1-naphthalen-1-yl-ethyl)-acetamide:**

prepared by reaction of 1-benzyl-6,7-dimethoxy-1,2,3,4-tetrahydro-isoquinoline and 2-bromoacetyl bromide with 1-naphthaleneethylamine

LC-MS: rt = 4.3 and 4.4 min, 495 (M+1, ES+).

**Example 42**

**2-(1-Benzyl-6,7-dimethoxy-3,4-dihydro-1*H*-isoquinolin-2-yl)-*N*-benzyl-*N*-methyl-acetamide:**

prepared by reaction of 1-benzyl-6,7-dimethoxy-1,2,3,4-tetrahydroisoquinoline and 2-bromoacetyl bromide with N-benzylmethylamine

LC-MS: rt = 3.8 min, 445 (M+1, ES+).

**Example 43**

**2-[1-(3-Methoxy-benzyl)-6,7-dimethoxy-3,4-dihydro-1*H*-isoquinolin-2-yl]-*N*-(2-methoxy-benzyl)-acetamide:**

prepared by reaction of 1-(3-Methoxy-benzyl)-6,7-dimethoxy-1,2,3,4-tetrahydro-isoquinoline and 2-bromoacetyl bromide with 2-methoxybenzylamine

LC-MS: rt = 4.0 min, 491 (M+1, ES+).

**Example 44**

**2-[1-(3-Methoxy-benzyl)-6,7-dimethoxy-3,4-dihydro-1*H*-isoquinolin-2-yl]-*N*-benzyl-acetamide:**

prepared by reaction of 1-(3-Methoxy-benzyl)-6,7-dimethoxy-1,2,3,4-tetrahydro-isoquinoline and 2-bromoacetyl bromide with benzylamine

LC-MS: rt = 3.9 min, 461 (M+1, ES+).

**Example 45**

**2-[1-(3-Methoxy-benzyl)-6,7-dimethoxy-3,4-dihydro-1*H*-isoquinolin-2-yl]-*N*-(naphthalen-1-yl-methyl)-acetamide:**

prepared by reaction of 1-(3-Methoxy-benzyl)-6,7-dimethoxy-1,2,3,4-tetrahydro-isoquinoline and 2-bromoacetyl bromide with naphthalen-1-yl-methylamine

LC-MS: rt = 4.3 min, 511 (M+1, ES+).

**Example 46**

**2-[1-(3-Methoxy-benzyl)-6,7-dimethoxy-3,4-dihydro-1*H*-isoquinolin-2-yl]-*N*-(3-methyl-benzyl)-acetamide:**

prepared by reaction of 1-(3-Methoxy-benzyl)-6,7-dimethoxy-1,2,3,4-tetrahydro-isoquinoline and 2-bromoacetyl bromide with 3-methyl-benzylamine

LC-MS: rt = 4.1 min, 475 (M+1, ES+).

**Example 47**

**2-[1-(3-Methoxy-benzyl)-6,7-dimethoxy-3,4-dihydro-1*H*-isoquinolin-2-yl]-*N*-(pyridin-3-yl-methyl)-acetamide:**

prepared by reaction of 1-(3-Methoxy-benzyl)-6,7-dimethoxy-1,2,3,4-tetrahydro-isoquinoline and 2-bromoacetyl bromide with 3-aminomethyl-pyridine

LC-MS: rt = 3.1 min, 462 (M+1, ES+).

**Example 48**

**2-[1-(3-Methoxy-benzyl)-6,7-dimethoxy-3,4-dihydro-1*H*-isoquinolin-2-yl]-*N*-(2-fluoro-benzyl)-acetamide:**

prepared by reaction of 1-(3-Methoxy-benzyl)-6,7-dimethoxy-1,2,3,4-tetrahydro-isoquinoline and 2-bromoacetyl bromide with 2-fluoro-benzylamine

LC-MS: rt = 4.0 min, 479 (M+1, ES+).

**Example 49**

**2-[1-(4-Methoxy-benzyl)-6,7-dimethoxy-3,4-dihydro-1*H*-isoquinolin-2-yl]-*N*-benzyl-acetamide:**

prepared by reaction of 1-(4-Methoxy-benzyl)-6,7-dimethoxy-1,2,3,4-tetrahydro-isoquinoline and 2-bromoacetyl bromide with benzylamine

LC-MS: rt = 3.9 min, 461 (M+1, ES+).

**Example 50**

**2-[1-(4-Methoxy-benzyl)-6,7-dimethoxy-3,4-dihydro-1*H*-isoquinolin-2-yl]-*N*-(naphthalen-1-yl-methyl)-acetamide:**

prepared by reaction of 1-(4-Methoxy-benzyl)-6,7-dimethoxy-1,2,3,4-tetrahydro-isoquinoline and 2-bromoacetyl bromide with naphthalen-1-yl-methylamine

LC-MS: rt = 4.2 min, 511 (M+1, ES+).

**Example 51**

**2-[1-(4-Methoxy-benzyl)-6,7-dimethoxy-3,4-dihydro-1*H*-isoquinolin-2-yl]-*N*-(2-fluoro-benzyl)-acetamide:**

prepared by reaction of 1-(4-Methoxy-benzyl)-6,7-dimethoxy-1,2,3,4-tetrahydro-isoquinoline and 2-bromoacetyl bromide with 2-fluoro-benzylamine

LC-MS: rt = 3.9 min, 479 (M+1, ES+).

**Example 52**

**2-[1-(3,4-Dimethoxy-benzyl)-6,7-dimethoxy-3,4-dihydro-1*H*-isoquinolin-2-yl]-*N*-(2-methoxy-benzyl)-acetamide:**

prepared by reaction of 1-(3,4-Dimethoxy-benzyl)-6,7-dimethoxy-1,2,3,4-tetrahydroisoquinoline and 2-bromoacetyl bromide with 2-methoxybenzylamine

LC-MS: rt = 3.7 min, 521 (M+1, ES+).

**Example 53**

**2-[1-(3,4-Dimethoxy-benzyl)-6,7-dimethoxy-3,4-dihydro-1*H*-isoquinolin-2-yl]-*N*-(4-methoxy-benzyl)-acetamide:**

prepared by reaction of 1-(3,4-Dimethoxy-benzyl)-6,7-dimethoxy-1,2,3,4-tetrahydroisoquinoline and 2-bromoacetyl bromide with 4-methoxybenzylamine LC-MS: rt = 3.7 min, 521 (M+1, ES+).

**Example 54**

**2-[1-(3,4-Dimethoxy-benzyl)-6,7-dimethoxy-3,4-dihydro-1*H*-isoquinolin-2-yl]-*N*-(3-methyl-benzyl)-acetamide:**

prepared by reaction of 1-(3,4-Dimethoxy-benzyl)-6,7-dimethoxy-1,2,3,4-tetrahydroisoquinoline and 2-bromoacetyl bromide with 3-methylbenzylamine
LC-MS: rt = 3.8 min, 505 (M+1, ES+).

**Example 55**

**2-[1-(3,4-Dimethoxy-benzyl)-6,7-dimethoxy-3,4-dihydro-1*H*-isoquinolin-2-yl]-*N*-(4-methyl-benzyl)-acetamide:**

prepared by reaction of 1-(3,4-Dimethoxy-benzyl)-6,7-dimethoxy-1,2,3,4-tetrahydroisoquinoline and 2-bromoacetyl bromide with 4-methylbenzylamine
LC-MS: rt = 3.8 min, 505 (M+1, ES+).

**Example 56**

**2-[1-(3,4-Dimethoxy-benzyl)-6,7-dimethoxy-3,4-dihydro-1*H*-isoquinolin-2-yl]-*N*-(pyridin-3-yl-methyl)-acetamide:**

prepared by reaction of 1-(3,4-Dimethoxy-benzyl)-6,7-dimethoxy-1,2,3,4-tetrahydroisoquinoline and 2-bromoacetyl bromide with 3-piconylamine
LC-MS: rt = 2.9 min, 492 (M+1, ES+).

**Example 57**

**2-[1-(3,4-Dimethoxy-benzyl)-6,7-dimethoxy-3,4-dihydro-1*H*-isoquinolin-2-yl]-*N*-(pyridin-4-yl-methyl)-acetamide:**

prepared by reaction of 1-(3,4-Dimethoxy-benzyl)-6,7-dimethoxy-1,2,3,4-tetrahydroisoquinoline and 2-bromoacetyl bromide with 4-piconylamine
LC-MS: rt = 2.9 min, 492 (M+1, ES+).

**Example 58**

**2-[1-(3,4-Dimethoxy-benzyl)-6,7-dimethoxy-3,4-dihydro-1*H*-isoquinolin-2-yl]-*N*-phenyl-acetamide:**

prepared by reaction of 1-(3,4-Dimethoxy-benzyl)-6,7-dimethoxy-1,2,3,4-tetrahydroisoquinoline and 2-bromoacetyl bromide with aniline
LC-MS: rt = 3.7 min, 477 (M+1, ES+).

**Example 59**

**2-[1-(3,4-Dimethoxy-benzyl)-6,7-dimethoxy-3,4-dihydro-1*H*-isoquinolin-2-yl]-*N*-(2-fluoro-benzyl)-acetamide:**

prepared by reaction of 1-(3,4-Dimethoxy-benzyl)-6,7-dimethoxy-1,2,3,4-tetrahydroisoquinoline and 2-bromoacetyl bromide with 2-fluorobenzylamine
LC-MS: rt = 3.7 min, 509 (M+1, ES+).

**Example 60**

**2-[1-(3,4-Dimethoxy-benzyl)-6,7-dimethoxy-3,4-dihydro-1*H*-isoquinolin-2-yl]-*N*-[2-(4-methoxy-phenyl)-ethyl]-acetamide:**

prepared by reaction of 1-(3,4-Dimethoxy-benzyl)-6,7-dimethoxy-1,2,3,4-tetrahydroisoquinoline and 2-bromoacetyl bromide with 4-methoxyphenylethylamine
LC-MS: rt = 3.8 min, 535 (M+1, ES+).

**Example 61**

**2-[1-(3,4-Dimethoxy-benzyl)-6,7-dimethoxy-3,4-dihydro-1*H*-isoquinolin-2-yl]-*N*-(2-methyl-benzyl)-acetamide:**

prepared by reaction of 1-(3,4-Dimethoxy-benzyl)-6,7-dimethoxy-1,2,3,4-tetrahydroisoquinoline and 2-bromoacetyl bromide with 2-methylbenzylamine

LC-MS: rt = 3.9 min, 505 (M+1, ES+).

**Example 62**

**2-[1-(3,4-Dimethoxy-benzyl)-6,7-dimethoxy-3,4-dihydro-1*H*-isoquinolin-2-yl]-*N*-(2-trifluoromethyl-benzyl)-acetamide:**

prepared by reaction of 1-(3,4-Dimethoxy-benzyl)-6,7-dimethoxy-1,2,3,4-tetrahydroisoquinoline and 2-bromoacetyl bromide with 2-trifluorobenzylamine

LC-MS: rt = 4.0 min, 559 (M+1, ES+).

**Example 63**

**2-[1-(3,4-Dimethoxy-benzyl)-6,7-dimethoxy-3,4-dihydro-1*H*-isoquinolin-2-yl]-*N*-(2,5-difluoro-benzyl)-acetamide:**

prepared by reaction of 1-(3,4-Dimethoxy-benzyl)-6,7-dimethoxy-1,2,3,4-tetrahydroisoquinoline and 2-bromoacetyl bromide with 2,5-difluorobenzylamine LC-MS: rt = 3.8 min, 527 (M+1, ES+).

**Example 64**

**2-[1-(3,4-Dimethoxy-benzyl)-6,7-dimethoxy-3,4-dihydro-1*H*-isoquinolin-2-yl]-*N*-(4-fluoro-benzyl)-acetamide:**

prepared by reaction of 1-(3,4-Dimethoxy-benzyl)-6,7-dimethoxy-1,2,3,4-tetrahydroisoquinoline and 2-bromoacetyl bromide with 4-fluorobenzylamine

LC-MS: rt = 3.8 min, 509 (M+1, ES+).

**Example 65**

**2-[1-(3,4-Dimethoxy-benzyl)-6,7-dimethoxy-3,4-dihydro-1*H*-isoquinolin-2-yl]-*N*-(2-chloro-benzyl)-acetamide:**

prepared by reaction of 1-(3,4-Dimethoxy-benzyl)-6,7-dimethoxy-1,2,3,4-tetrahydroisoquinoline and 2-bromoacetyl bromide with 2-chlorobenzylamine

LC-MS: rt = 3.9 min, 525 (M+1, ES+).

**Example 66**

**2-[1-(3,4-Dimethoxy-benzyl)-6,7-dimethoxy-3,4-dihydro-1*H*-isoquinolin-2-yl]-*N*-(2,4-dimethoxy-benzyl)-acetamide:**

prepared by reaction of 1-(3,4-Dimethoxy-benzyl)-6,7-dimethoxy-1,2,3,4-tetrahydroisoquinoline and 2-bromoacetyl bromide with 2,4-dimethoxybenzylamine

LC-MS: rt = 3.8 min, 551 (M+1, ES+).

**Example 67**

**2-[1-(3,4-Dimethoxy-benzyl)-6,7-dimethoxy-3,4-dihydro-1*H*-isoquinolin-2-yl]-*N*-(1-phenyl-ethyl)-acetamide:**

prepared by reaction of 1-(3,4-Dimethoxy-benzyl)-6,7-dimethoxy-1,2,3,4-tetrahydroisoquinoline and 2-bromoacetyl bromide with 1-phenylethylamine

LC-MS: rt = 3.7 min, 505 (M+1, ES+).

**Example 68**

**2-[1-(3,4-Dimethoxy-benzyl)-6,7-dimethoxy-3,4-dihydro-1*H*-isoquinolin-2-yl]-*N*-(1-naphthalen-1-yl-ethyl)-acetamide:**

prepared by reaction of 1-(3,4-Dimethoxy-benzyl)-6,7-dimethoxy-1,2,3,4-tetrahydroisoquinoline and 2-bromoacetyl bromide with 1-naphthaleneethylamine

LC-MS: rt = 4.0 min, 555 (M+1, ES+).

**Example 69**

**2-[1-(3,4-Dimethoxy-benzyl)-6,7-dimethoxy-3,4-dihydro-1*H*-isoquinolin-2-yl]-*N*-benzyl-*N*-methyl-acetamide:**

prepared by reaction of 1-(3,4-Dimethoxy-benzyl)-6,7-dimethoxy-1,2,3,4-tetrahydroisoquinoline and 2-bromoacetyl bromide with N-benzylmethylamine

LC-MS: rt = 3.6 min, 505 (M+1, ES+).

**Example 70**

**2-[1-(3,4-Dimethoxy-benzyl)-6,7-dimethoxy-3,4-dihydro-1*H*-isoquinolin-2-yl]-*N*-furan-2-yl-methyl-acetamide:**

prepared by reaction of 1-(3,4-Dimethoxy-benzyl)-6,7-dimethoxy-1,2,3,4-tetrahydroisoquinoline and 2-bromoacetyl bromide with 1-aminomethylfurane

LC-MS: rt = 3.5 min, 481 (M+1, ES+).

**Example 71**

**2-[1-(3,4-Dimethoxy-benzyl)-6,7-dimethoxy-3,4-dihydro-1*H*-isoquinolin-2-yl]-*N*-but-2-yl-acetamide:**

prepared by reaction of 1-(3,4-Dimethoxy-benzyl)-6,7-dimethoxy-1,2,3,4-tetrahydroisoquinoline and 2-bromoacetyl bromide with 2-butylamine

LC-MS: rt = 0.57 min, 457 (M+1, ES+).

**Example 72**

**2-[1-(3,4-Dimethoxy-benzyl)-6,7-dimethoxy-3,4-dihydro-1*H*-isoquinolin-2-yl]-*N*-(pyridin-2-yl-methyl)-acetamide:**

prepared by reaction of 1-(3,4-Dimethoxy-benzyl)-6,7-dimethoxy-1,2,3,4-tetrahydroisoquinoline and 2-bromoacetyl bromide with 2-picolylamine

LC-MS: rt = 0.46 min, 492 (M+1, ES+).

**Example 73**

**2-[(1S)-1-(3,4-Dimethoxy-benzyl)-6,7-dimethoxy-3,4-dihydro-1*H*-isoquinolin-2-yl]-*N*-benzyl-acetamide:**

prepared by reaction of (1S)-1-(3,4-Dimethoxy-benzyl)-6,7-dimethoxy-1,2,3,4-tetrahydroisoquinoline and 2-bromoacetyl bromide with benzylamine

LC-MS: rt = 3.7 min, 491 (M+1, ES+).

**Example 74**

**2-[(1S)-1-(3,4-Dimethoxy-benzyl)-6,7-dimethoxy-3,4-dihydro-1*H*-isoquinolin-2-yl]-*N*-(naphthalen-1-yl-methyl)-acetamide:**

prepared by reaction of (1S)-1-(3,4-Dimethoxy-benzyl)-6,7-dimethoxy-1,2,3,4-tetrahydroisoquinoline and 2-bromoacetyl bromide with naphthalen-1-yl-methylamine

LC-MS: rt = 4.0 min, 541 (M+1, ES+).

**Example 75**

**2-[(1S)-1-(3,4-Dimethoxy-benzyl)-6,7-dimethoxy-3,4-dihydro-1*H*-isoquinolin-2-yl]-*N*-(2-methoxy-benzyl)-acetamide:**

prepared by reaction of (1S)-1-(3,4-Dimethoxy-benzyl)-6,7-dimethoxy-1,2,3,4-tetrahydroisoquinoline and 2-bromoacetyl bromide with 2-methoxy-benzylamine

LC-MS: rt = 3.7 min, 521 (M+1, ES+).

**Example 76**

**2-[(1S)-1-(3,4-Dimethoxy-benzyl)-6,7-dimethoxy-3,4-dihydro-1*H*-isoquinolin-2-yl]-*N*-(2-ethoxy-benzyl)-acetamide:**

prepared by reaction of (1S)-1-(3,4-Dimethoxy-benzyl)-6,7-dimethoxy-1,2,3,4-tetrahydroisoquinoline and 2-bromoacetyl bromide with 2-ethoxy-benzylamine

LC-MS: rt = 4.0 min, 535 (M+1, ES+).

**Example 77**

**2-[(1S)-1-(3,4-Dimethoxy-benzyl)-6,7-dimethoxy-3,4-dihydro-1*H*-isoquinolin-2-yl]-*N*-benzyl-*N*-methyl-acetamide:**

prepared by reaction of (1S)-1-(3,4-Dimethoxy-benzyl)-6,7-dimethoxy-1,2,3,4-tetrahydroisoquinoline and 2-bromoacetyl bromide with *N*-benzyl-*N*-methylamine LC-MS: rt = 3.7 min, 505 (M+1, ES+).

**Example 78**

**2-[(1S)-1-(3,4-Dimethoxy-benzyl)-6,7-dimethoxy-3,4-dihydro-1*H*-isoquinolin-2-yl]-*N*-(pyridin-2-yl-methyl)-acetamide:**

prepared by reaction of (1S)-1-(3,4-Dimethoxy-benzyl)-6,7-dimethoxy-1,2,3,4-tetrahydroisoquinoline and 2-bromoacetyl bromide with 2-picolylamine

LC-MS: rt = 3.1 min, 492 (M+1, ES+).

**Example 79**

**2-[(1S)-1-(3,4-Dimethoxy-benzyl)-6,7-dimethoxy-3,4-dihydro-1*H*-isoquinolin-2-yl]-*N*-(2-phenyl-ethyl)-acetamide:**

prepared by reaction of (1S)-1-(3,4-Dimethoxy-benzyl)-6,7-dimethoxy-1,2,3,4-tetrahydroisoquinoline and 2-bromoacetyl bromide with 2-phenyl-ethylamine

LC-MS: rt = 3.8 min, 505 (M+1, ES+).

**Example 80**

**2-[1-(2,5-Dimethoxy-benzyl)-6,7-dimethoxy-3,4-dihydro-1*H*-isoquinolin-2-yl]-*N*-(4-methoxy-benzyl)-acetamide:**

prepared by reaction of 1-(2,5-Dimethoxy-benzyl)-6,7-dimethoxy-1,2,3,4-tetrahydroisoquinoline and 2-bromoacetyl bromide with 4-methoxybenzylamine

LC-MS: rt = 3.9 min, 521 (M+1, ES+).

**Example 81**

**2-[1-(3,4-Dimethoxy-benzyl)-5,8-dimethoxy-3,4-dihydro-1*H*-isoquinolin-2-yl]-*N*-(2-methoxy-benzyl)-acetamide:**

prepared by reaction of 1-(3,4-Dimethoxy-benzyl)-5,8-dimethoxy-1,2,3,4-tetrahydroisoquinoline and 2-bromoacetyl bromide with 2-methoxybenzylamine LC-MS: rt = 4.3 min, 521 (M+1, ES+).

**Example 82**

**2-[1-(3,4-Dimethoxy-benzyl)-5,8-dimethoxy-3,4-dihydro-1*H*-isoquinolin-2-yl]-*N*-benzyl-acetamide:**

prepared by reaction of 1-(3,4-Dimethoxy-benzyl)-5,8-dimethoxy-1,2,3,4-tetrahydroisoquinoline and 2-bromoacetyl bromide with benzylamine
LC-MS: rt = 4.3 min, 491 (M+1, ES+).

**Example 83**

**2-[1-(3,4-Dimethoxy-benzyl)-5,8-dimethoxy-3,4-dihydro-1*H*-isoquinolin-2-yl]-*N*-[2-(3,4-dimethoxy-phenyl)-ethyl]-acetamide:**

prepared by reaction of 1-(3,4-Dimethoxy-benzyl)-5,8-dimethoxy-1,2,3,4-tetrahydroisoquinoline and 2-bromoacetyl bromide with 3,4-dimethoxyphenylethylamine
LC-MS: rt = 4.3 min, 565 (M+1, ES+).

**Example 84**

**2-[1-(3,4-Dimethoxy-benzyl)-5,8-dimethoxy-3,4-dihydro-1*H*-isoquinolin-2-yl]-*N*-(pyridin-3-yl-methyl)-acetamide:**

prepared by reaction of 1-(3,4-Dimethoxy-benzyl)-5,8-dimethoxy-1,2,3,4-tetrahydroisoquinoline and 2-bromoacetyl bromide with 3-picolylamine
LC-MS: rt = 3.4 min, 492 (M+1, ES+).

**Example 85**

**2-[1-(3,4-Dimethoxy-benzyl)-5,8-dimethoxy-3,4-dihydro-1*H*-isoquinolin-2-yl]-*N*-butyl-acetamide:**

prepared by reaction of 1-(3,4-Dimethoxy-benzyl)-5,8-dimethoxy-1,2,3,4-tetrahydroisoquinoline and 2-bromoacetyl bromide with n-butylamine
LC-MS: rt = 4.2 min, 457 (M+1, ES+).

**Example 86**

**2-[1-(3,4-Dimethoxy-benzyl)-5,8-dimethoxy-3,4-dihydro-1*H*-isoquinolin-2-yl]-*N*-(2-fluoro-benzyl)-acetamide:**

prepared by reaction of 1-(3,4-Dimethoxy-benzyl)-5,8-dimethoxy-1,2,3,4-tetrahydroisoquinoline and 2-bromoacetyl bromide with 2-fluorobenzylamine
LC-MS: rt = 4.4 min, 509 (M+1, ES+).

**Example 87**

**2-[1-(3,4-Dimethoxy-benzyl)-5,8-dimethoxy-3,4-dihydro-1*H*-isoquinolin-2-yl]-*N*-(pyridin-2-yl-methyl)-acetamide:**

prepared by reaction of 1-(3,4-Dimethoxy-benzyl)-5,8-dimethoxy-1,2,3,4-tetrahydroisoquinoline and 2-bromoacetyl bromide with 2-picolylamine
LC-MS: rt = 3.7 min, 492 (M+1, ES+).

**Example 88**

**2-[1-(3,4-Dimethoxy-benzyl)-5,8-dimethoxy-3,4-dihydro-1*H*-isoquinolin-2-yl]-*N*-[1,3,4]thiadiazol-2-yl-acetamide:**

prepared by reaction of 1-(3,4-Dimethoxy-benzyl)-5,8-dimethoxy-1,2,3,4-tetrahydroisoquinoline and 2-bromoacetyl bromide with 2-amino-1,3,4-thiadiazole
LC-MS: rt = 3.8 min, 485 (M+1, ES+).

**Example 89**

**2-[1-(3,4-Dimethoxy-benzyl)-5,8-dimethoxy-3,4-dihydro-1*H*-isoquinolin-2-yl]-*N*-(1*H*-benzoimidazol-2yl-methyl)-acetamide:**

prepared by reaction of 1-(3,4-Dimethoxy-benzyl)-5,8-dimethoxy-1,2,3,4-tetrahydroisoquinoline and 2-bromoacetyl bromide with 2-(aminomethyl)-benzimidazole
LC-MS: rt = 3.4 min, 531 (M+1, ES+).

**Example 90**

**2-[1-(2-Phenyl-ethyl)-6,7-dimethoxy-3,4-dihydro-1*H*-isoquinolin-2-yl]-*N*-(pyridin-3-yl-methyl)-acetamide:**

prepared by reaction of 1-(2-Phenyl-ethyl)-6,7-dimethoxy-1,2,3,4-tetrahydro-isoquinoline and 2-bromoacetyl bromide with 3-picolylamine
LC-MS: rt = 2.7 min, 446 (M+1, ES+).

**Example 91**

**2-[1-(2-Phenyl-ethyl)-6,7-dimethoxy-3,4-dihydro-1*H*-isoquinolin-2-yl]-*N*-(2-fluoro-benzyl)-acetamide:**

prepared by reaction of 1-(2-Phenyl-ethyl)-6,7-dimethoxy-1,2,3,4-tetrahydro-isoquinoline and 2-bromoacetyl bromide with 2-fluorobenzylamine
LC-MS: rt = 4.0 min, 463 (M+1, ES+).

**Example 92**

**2-[1-(2-Phenyl-ethyl)-6,7-dimethoxy-3,4-dihydro-1*H*-isoquinolin-2-yl]-*N*-cyclohexyl-acetamide:**

prepared by reaction of 1-(2-Phenyl-ethyl)-6,7-dimethoxy-1,2,3,4-tetrahydro-isoquinoline and 2-bromoacetyl bromide with cyclohexylamine
LC-MS: rt = 4.0 min, 437 (M+1, ES+).

**Example 93**

**2-[1-(1-Phenyl-propyl)-6,7-dimethoxy-3,4-dihydro-1*H*-isoquinolin-2-yl]-*N*-benzyl-acetamide:**

prepared by reaction of 1-(1-Phenyl-propyl)-6,7-dimethoxy-1,2,3,4-tetrahydroisoquinoline and 2-bromoacetyl bromide with benzylamine
LC-MS: rt = 4.4 min, 459 (M+1, ES+).

**Example 94**

**2-[1-(1-Phenyl-propyl)-6,7-dimethoxy-3,4-dihydro-1*H*-isoquinolin-2-yl]-*N*-(pyridin-2-yl-methyl)-acetamide:**

prepared by reaction of 1-(1-Phenyl-propyl)-6,7-dimethoxy-1,2,3,4-tetrahydroisoquinoline and 2-bromoacetyl bromide with 2-picolylamine
LC-MS: rt = 3.7 min, 460 (M+1, ES+).

**Example 95**

**2-[1-(2,5-Dimethoxy-benzyl)-5,8-dimethoxy-3,4-dihydro-1*H*-isoquinolin-2-yl]-*N*-benzyl-acetamide:**

prepared by reaction of 1-(2,5-Dimethoxy-benzyl)-5,8-dimethoxy-1,2,3,4-tetrahydroisoquinoline and 2-bromoacetyl bromide with benzylamine

LC-MS: rt = 4.4 min, 491 (M+1, ES+).

**Example 96**

**2-[1-(2,5-Dimethoxy-benzyl)-5,8-dimethoxy-3,4-dihydro-1*H*-isoquinolin-2-yl]-*N*-(2-methoxy-benzyl)-acetamide:**

prepared by reaction of 1-(2,5-Dimethoxy-benzyl)-5,8-dimethoxy-1,2,3,4-tetrahydroisoquinoline and 2-bromoacetyl bromide with 2-methoxy-benzyl-amine

LC-MS: rt = 4.5 min, 521 (M+1, ES+).

**Example 97**

**2-[1-(2,5-Dimethoxy-benzyl)-5,8-dimethoxy-3,4-dihydro-1*H*-isoquinolin-2-yl]-*N*-(2-ethoxy-benzyl)-acetamide:**

prepared by reaction of 1-(2,5-Dimethoxy-benzyl)-5,8-dimethoxy-1,2,3,4-tetrahydroisoquinoline and 2-bromoacetyl bromide with 2-ethoxy-benzyl-amine

LC-MS: rt = 4.6 min, 535 (M+1, ES+).

**Example 98**

**2-[1-(2,5-Dimethoxy-benzyl)-5,8-dimethoxy-3,4-dihydro-1*H*-isoquinolin-2-yl]-*N*-(pyridin-2-yl-methyl)-acetamide:**

prepared by reaction of 1-(2,5-Dimethoxy-benzyl)-5,8-dimethoxy-1,2,3,4-tetrahydroisoquinoline and 2-bromoacetyl bromide with 2-picolylamine

LC-MS: rt = 3.8 min, 492 (M+1, ES+).

**Example 99**

**2-[1-(4-Chloro-benzyl)-6,7-dimethoxy-3,4-dihydro-1*H*-isoquinolin-2-yl]-*N*-benzyl-acetamide:**

prepared by reaction of 1-(4-Chloro-benzyl)-6,7-dimethoxy-1,2,3,4-tetra-hydroisoquinoline and 2-bromoacetyl bromide with benzylamine

LC-MS: rt = 4.4 min, 465 (M+1, ES+).

**Example 100**

**2-[1-(4-Chloro-benzyl)-6,7-dimethoxy-3,4-dihydro-1*H*-isoquinolin-2-yl]-*N*-(2-ethoxy-benzyl)-acetamide:**

prepared by reaction of 1-(4-Chloro-benzyl)-6,7-dimethoxy-1,2,3,4-tetra-hydroisoquinoline and 2-bromoacetyl bromide with 2-ethoxy-benzylamine

LC-MS: rt = 4.7 min, 509 (M+1, ES+).

**Example 101**

**2-[1-(4-Chloro-benzyl)-6,7-dimethoxy-3,4-dihydro-1*H*-isoquinolin-2-yl]-*N*-(pyridin-2-yl-methyl)-acetamide:**

prepared by reaction of 1-(4-Chloro-benzyl)-6,7-dimethoxy-1,2,3,4-tetra-hydroisoquinoline and 2-bromoacetyl bromide with 2-picolylamine

LC-MS: rt = 3.6 min, 466 (M+1, ES+).

**Example 102**

**2-[1-(3,4-Dimethoxy-benzyl)-6-methoxy-7-isopropoxy-3,4-dihydro-1*H*-isoquinolin-2-yl]-*N*-benzyl-acetamide:**

prepared by reaction of 1-(3,4-Dimethoxy-benzyl)-6-methoxy-7-isopropoxy-1,2,3,4-tetrahydroisoquinoline and 2-bromoacetyl bromide with benzylamine

LC-MS: rt = 3.9 min, 519 (M+1, ES+).

**Example 103**

**2-[1-(3,4-Dimethoxy-benzyl)-6-methoxy-7-isopropoxy-3,4-dihydro-1*H*-isoquinolin-2-yl]-*N*-(naphthalen-1-yl-methyl)-acetamide:**

prepared by reaction of 1-(3,4-Dimethoxy-benzyl)-6-methoxy-7-isopropoxy-1,2,3,4-tetrahydroisoquinoline and 2-bromoacetyl bromide with naphthalen-1-yl-methylamine

LC-MS: rt = 4.3 min, 569 (M+1, ES+).

**Example 104**

**2-[1-(3,4-Dimethoxy-benzyl)-6-methoxy-7-isopropoxy-3,4-dihydro-1*H*-isoquinolin-2-yl]-*N*-(2-methoxy-benzyl)-acetamide:**

prepared by reaction of 1-(3,4-Dimethoxy-benzyl)-6-methoxy-7-isopropoxy-1,2,3,4-tetrahydroisoquinoline and 2-bromoacetyl bromide with 2-methoxy-benzylamine

LC-MS: rt = 4.0 min, 549 (M+1, ES+).

**Example 105**

**2-[1-(3,4-Dimethoxy-benzyl)-6-methoxy-7-isopropoxy-3,4-dihydro-1*H*-isoquinolin-2-yl]-*N*-(2-ethoxy-benzyl)-acetamide:**

prepared by reaction of 1-(3,4-Dimethoxy-benzyl)-6-methoxy-7-isopropoxy-1,2,3,4-tetrahydroisoquinoline and 2-bromoacetyl bromide with 2-ethoxy-benzylamine

LC-MS: rt = 4.2 min, 563 (M+1, ES+).

**Example 106**

**2-[1-(3,4-Dimethoxy-benzyl)-6-methoxy-7-isopropoxy-3,4-dihydro-1*H*-isoquinolin-2-yl]-*N*-benzyl-*N*-methyl-acetamide:**

prepared by reaction of 1-(3,4-Dimethoxy-benzyl)-6-methoxy-7-isopropoxy-1,2,3,4-tetrahydroisoquinoline and 2-bromoacetyl bromide with *N*-benzyl-*N*-methyl-amine

LC-MS: rt = 3.9 min, 533 (M+1, ES+).

**Example 107**

**2-[1-(3,4-Dimethoxy-benzyl)-6-methoxy-7-isopropoxy-3,4-dihydro-1*H*-isoquinolin-2-yl]-*N*-(pyridin-2-yl-methyl)-acetamide:**

prepared by reaction of 1-(3,4-Dimethoxy-benzyl)-6-methoxy-7-isopropoxy-1,2,3,4-tetrahydroisoquinoline and 2-bromoacetyl bromide with 2-picolylamine

LC-MS: rt = 3.4 min, 520 (M+1, ES+).

**Example 108**

**2-[1-(3,4-Dimethoxy-benzyl)-6-methoxy-7-isopropoxy-3,4-dihydro-1*H*-isoquinolin-2-yl]-*N*-(2-phenyl-ethyl)-acetamide:**

prepared by reaction of 1-(3,4-Dimethoxy-benzyl)-6-methoxy-7-isopropoxy-1,2,3,4-tetrahydroisoquinoline and 2-bromoacetyl bromide with 2-phenyl-ethylamine

LC-MS: rt = 4.0 min, 533 (M+1, ES+).

**Example 109**

**2-[1-(3,4-Methylenedioxy-benzyl)-6,7-dimethoxy-3,4-dihydro-1*H*-isoquinolin-2-yl]-*N*-benzyl-acetamide:**

prepared by reaction of 1-(3,4-Methylenedioxy-benzyl)-6,7-dimethoxy-1,2,3,4-tetrahydroisoquinoline and 2-bromoacetyl bromide with benzylamine

LC-MS: rt = 4.0 min, 475 (M+1, ES+).

**Example 110**

**2-[1-(3,4-Methylenedioxy-benzyl)-6,7-dimethoxy-3,4-dihydro-1*H*-isoquinolin-2-yl]-*N*-(2-ethoxy-benzyl)-acetamide:**

prepared by reaction of 1-(3,4-Methylenedioxy-benzyl)-6,7-dimethoxy-1,2,3,4-tetrahydroisoquinoline and 2-bromoacetyl bromide with 2-ethoxy-benzylamine

LC-MS: rt = 4.2 min, 519 (M+1, ES+).

**Example 111**

**2-[1-(4-Dimethylamino-benzyl)-6,7-dimethoxy-3,4-dihydro-1*H*-isoquinolin-2-yl]-*N*-benzyl-acetamide:**

prepared by reaction of 1-(4-Dimethylamino-benzyl)-6,7-dimethoxy-1,2,3,4-tetrahydroisoquinoline and 2-bromoacetyl bromide with benzylamine

LC-MS: rt = 3.5 min, 474 (M+1, ES+).

**Example 112**

**2-[1-(4-Dimethylamino-benzyl)-6,7-dimethoxy-3,4-dihydro-1*H*-isoquinolin-2-yl]-*N*-(naphthalen-1-yl-methyl)-acetamide:**

prepared by reaction of 1-(4-Dimethylamino-benzyl)-6,7-dimethoxy-1,2,3,4-tetrahydroisoquinoline and 2-bromoacetyl bromide with naphthalen-1-yl-methylamine

LC-MS: rt = 4.0 min, 524 (M+1, ES+).

**Example 113**

**2-[1-(4-Dimethylamino-benzyl)-6,7-dimethoxy-3,4-dihydro-1*H*-isoquinolin-2-yl]-*N*-(2-methoxy-benzyl)-acetamide:**

prepared by reaction of 1-(4-Dimethylamino-benzyl)-6,7-dimethoxy-1,2,3,4-tetrahydroisoquinoline and 2-bromoacetyl bromide with 2-methoxy-benzylamine

LC-MS: rt = 3.6 min, 504 (M+1, ES+).

**Example 114**

**2-[1-(4-Dimethylamino-benzyl)-6,7-dimethoxy-3,4-dihydro-1*H*-isoquinolin-2-yl]-*N*-(2-ethoxy-benzyl)-acetamide:**

prepared by reaction of 1-(4-Dimethylamino-benzyl)-6,7-dimethoxy-1,2,3,4-tetrahydroisoquinoline and 2-bromoacetyl bromide with 2-ethoxy-benzylamine

LC-MS: rt = 3.8 min, 518 (M+1, ES+).

**Example 115**

**2-[1-(4-Dimethylamino-benzyl)-6,7-dimethoxy-3,4-dihydro-1*H*-isoquinolin-2-yl]-*N*-(pyridin-2-yl-methyl)-acetamide:**

prepared by reaction of 1-(4-Dimethylamino-benzyl)-6,7-dimethoxy-1,2,3,4-tetrahydroisoquinoline and 2-bromoacetyl bromide with 2-picolylamine

LC-MS: rt = 2.9 min, 475 (M+1, ES+).

**Example 116**

**2-[1-(4-Fluoro-benzyl)-6,7-dimethoxy-3,4-dihydro-1*H*-isoquinolin-2-yl]-*N*-(2-ethoxy-benzyl)-acetamide:**

prepared by reaction of 1-(4-Fluoro-benzyl)-6,7-dimethoxy-1,2,3,4-tetrahydro-isoquinoline and 2-bromoacetyl bromide with 2-ethoxy-benzylamine

LC-MS: rt = 4.3 min, 493 (M+1, ES+).

**Example 117**

**2-[1-(4-Fluoro-benzyl)-6,7-dimethoxy-3,4-dihydro-1*H*-isoquinolin-2-yl]-*N*-benzyl-*N*-methyl-acetamide:**

prepared by reaction of 1-(4-Fluoro-benzyl)-6,7-dimethoxy-1,2,3,4-tetrahydro-isoquinoline and 2-bromoacetyl bromide with *N*-benzyl-*N*-methylamine

LC-MS: rt = 3.8 min, 463 (M+1, ES+).

**Example 118**

**2-[1-(3,4-Difluoro-benzyl)-6,7-dimethoxy-3,4-dihydro-1*H*-isoquinolin-2-yl]-*N*-benzyl-*N*-methyl-acetamide:**

prepared by reaction of 1-(3,4-Difluoro-benzyl)-6,7-dimethoxy-1,2,3,4-tetrahydroisoquinoline and 2-bromoacetyl bromide with *N*-benzyl-*N*-methylamine LC-MS: rt = 3.9 min, 481 (M+1, ES+).

**Example 119**

**2-[1-(3,4-Dimethoxy-benzyl)-6,7,8-trimethoxy-3,4-dihydro-1*H*-isoquinolin-2-yl]-*N*-benzyl-acetamide:**

prepared by reaction of 1-(3,4-Dimethoxy-benzyl)-6,7,8-trimethoxy-1,2,3,4-tetrahydroisoquinoline and 2-bromoacetyl bromide with benzylamine

LC-MS: rt = 4.4 min, 521 (M+1, ES+).

**Example 120**

**2-[1-(3,4-Dimethoxy-benzyl)-6,7,8-trimethoxy-3,4-dihydro-1*H*-isoquinolin-2-yl]-*N*-(naphthalen-1-yl-methyl)-acetamide:**

prepared by reaction of 1-(3,4-Dimethoxy-benzyl)-6,7,8-trimethoxy-1,2,3,4-tetrahydroisoquinoline and 2-bromoacetyl bromide with naphthalen-1-yl-methylamine

LC-MS: rt = 4.8 min, 571 (M+1, ES+).

**Example 121**

**2-[1-(3,4-Dimethoxy-benzyl)-6,7,8-trimethoxy-3,4-dihydro-1*H*-isoquinolin-2-yl]-*N*-(2-methoxy-benzyl)-acetamide:**

prepared by reaction of 1-(3,4-Dimethoxy-benzyl)-6,7,8-trimethoxy-1,2,3,4-tetrahydroisoquinoline and 2-bromoacetyl bromide with 2-methoxy-benzyl-amine
LC-MS: rt = 4.4 min, 551 (M+1, ES+).

**Example 122**

**2-[1-(3,4-Dimethoxy-benzyl)-6,7,8-trimethoxy-3,4-dihydro-1*H*-isoquinolin-2-yl]-*N*-(2-ethoxy-benzyl)-acetamide:**

prepared by reaction of 1-(3,4-Dimethoxy-benzyl)-6,7,8-trimethoxy-1,2,3,4-tetrahydroisoquinoline and 2-bromoacetyl bromide with 2-ethoxy-benzyl-amine LC-MS: rt = 4.6 min, 565 (M+1, ES+).

**Example 123**

**2-[1-(3,4-Dimethoxy-benzyl)-6,7,8-trimethoxy-3,4-dihydro-1*H*-isoquinolin-2-yl]-*N*-benzyl-*N*-methyl-acetamide:**

prepared by reaction of 1-(3,4-Dimethoxy-benzyl)-6,7,8-trimethoxy-1,2,3,4-tetrahydroisoquinoline and 2-bromoacetyl bromide with *N*-benzyl-*N*-methylamine
LC-MS: rt = 4.0 min, 535 (M+1, ES+).

**Example 124**

**2-[1-(3,4-Dimethoxy-benzyl)-6,7,8-trimethoxy-3,4-dihydro-1*H*-isoquinolin-2-yl]-*N*-(pyridin-2-yl-methyl)-acetamide:**

prepared by reaction of 1-(3,4-Dimethoxy-benzyl)-6,7,8-trimethoxy-1,2,3,4-tetrahydroisoquinoline and 2-bromoacetyl bromide with 2-picolylamine
LC-MS: rt = 3.7 min, 522 (M+1, ES+).

**Example 125**

**2-[1-(3,4-Dimethoxy-benzyl)-6,7,8-trimethoxy-3,4-dihydro-1*H*-isoquinolin-2-yl]-*N*-(2-phenyl-ethyl)-acetamide:**

prepared by reaction of 1-(3,4-Dimethoxy-benzyl)-6,7,8-trimethoxy-1,2,3,4-tetrahydroisoquinoline and 2-bromoacetyl bromide with 2-phenyl-ethylamine
LC-MS: rt = 4.5 min, 535 (M+1, ES+).

**Example 126**

**2-[1-(3,4-Dimethoxy-benzyl)-5,6,7-trimethoxy-3,4-dihydro-1*H*-isoquinolin-2-yl]-*N*-benzyl-acetamide:**

prepared by reaction of 1-(3,4-Dimethoxy-benzyl)-5,6,7-trimethoxy-1,2,3,4-tetrahydroisoquinoline and 2-bromoacetyl bromide with benzylamine
LC-MS: rt = 4.1 min, 521 (M+1, ES+).

**Example 127**

**2-[1-(3,4-Dimethoxy-benzyl)-5,6,7-trimethoxy-3,4-dihydro-1*H*-isoquinolin-2-yl]-*N*-(naphthalen-1-yl-methyl)-acetamide:**

prepared by reaction of 1-(3,4-Dimethoxy-benzyl)-5,6,7-trimethoxy-1,2,3,4-tetrahydroisoquinoline and 2-bromoacetyl bromide with naphthalen-1-yl-methylamine
LC-MS: rt = 4.5 min, 571 (M+1, ES+).

**Example 128**

**2-[1-(3,4-Dimethoxy-benzyl)-5,6,7-trimethoxy-3,4-dihydro-1*H*-isoquinolin-2-yl]-*N*-(2-methoxy-benzyl)-acetamide:**

prepared by reaction of 1-(3,4-Dimethoxy-benzyl)-5,6,7-trimethoxy-1,2,3,4-tetrahydroisoquinoline and 2-bromoacetyl bromide with 2-methoxy-benzyl-amine LC-MS: rt = 4.2 min, 551 (M+1, ES+).

**Example 129**

**2-[1-(3,4-Dimethoxy-benzyl)-5,6,7-trimethoxy-3,4-dihydro-1*H*-isoquinolin-2-yl]-*N*-(2-ethoxy-benzyl)-acetamide:**

prepared by reaction of 1-(3,4-Dimethoxy-benzyl)-5,6,7-trimethoxy-1,2,3,4-tetrahydroisoquinoline and 2-bromoacetyl bromide with 2-ethoxy-benzyl-amine LC-MS: rt = 4.3 min, 565 (M+1, ES+).

**Example 130**

**2-[1-(3,4-Dimethoxy-benzyl)-5,6,7-trimethoxy-3,4-dihydro-1*H*-isoquinolin-2-yl]-*N*-benzyl-*N*-methyl-acetamide:**

prepared by reaction of 1-(3,4-Dimethoxy-benzyl)-5,6,7-trimethoxy-1,2,3,4-tetrahydroisoquinoline and 2-bromoacetyl bromide with *N*-benzyl-*N*-methyl-amine LC-MS: rt = 3.9 min, 535 (M+1, ES+).

**Example 131**

**2-[1-(3,4-Dimethoxy-benzyl)-5,6,7-trimethoxy-3,4-dihydro-1*H*-isoquinolin-2-yl]-*N*-(pyridin-2-yl-methyl)-acetamide:**

prepared by reaction of 1-(3,4-Dimethoxy-benzyl)-5,6,7-trimethoxy-1,2,3,4-tetrahydroisoquinoline and 2-bromoacetyl bromide with 2-picolylamine

LC-MS: rt = 3.4 min, 522 (M+1, ES+).

**Example 132**

**2-[1-(3,4-Dimethoxy-benzyl)-5,6,7-trimethoxy-3,4-dihydro-1*H*-isoquinolin-2-yl]-*N*-(2-phenyl-ethyl)-acetamide:**

prepared by reaction of 1-(3,4-Dimethoxy-benzyl)-5,6,7-trimethoxy-1,2,3,4-tetrahydroisoquinoline and 2-bromoacetyl bromide with 2-phenyl-ethylamine

LC-MS: rt = 4.2 min, 535 (M+1, ES+).

**Example 133**

**2-[1-(3,4-Dimethoxy-benzyl)-6,8-dimethoxy-3,4-dihydro-1*H*-isoquinolin-2-yl]-*N*-benzyl-acetamide:**

prepared by reaction of 1-(3,4-Dimethoxy-benzyl)-6,8-dimethoxy-1,2,3,4-tetrahydroisoquinoline and 2-bromoacetyl bromide with benzylamine

LC-MS: rt = 4.2 min, 491 (M+1, ES+).

**Example 134**

**2-[1-(3,4-Dimethoxy-benzyl)-6,8-dimethoxy-3,4-dihydro-1*H*-isoquinolin-2-yl]-*N*-(naphthalen-1-yl-methyl)-acetamide:**

prepared by reaction of 1-(3,4-Dimethoxy-benzyl)-6,8-dimethoxy-1,2,3,4-tetrahydroisoquinoline and 2-bromoacetyl bromide with naphthalen-1-yl-methylamine

LC-MS: rt = 4.5 min, 541 (M+1, ES+).

**Example 135**

**2-[1-(3,4-Dimethoxy-benzyl)-6,8-dimethoxy-3,4-dihydro-1*H*-isoquinolin-2-yl]-*N*-(2-methoxy-benzyl)-acetamide:**

prepared by reaction of 1-(3,4-Dimethoxy-benzyl)-6,8-dimethoxy-1,2,3,4-tetrahydroisoquinoline and 2-bromoacetyl bromide with 2-methoxy-benzyl-amine LC-MS: rt = 4.2 min, 521 (M+1, ES+).

**Example 136**

**2-[1-(3,4-Dimethoxy-benzyl)-6,8-dimethoxy-3,4-dihydro-1*H*-isoquinolin-2-yl]-*N*-(2-ethoxy-benzyl)-acetamide:**

prepared by reaction of 1-(3,4-Dimethoxy-benzyl)-6,8-dimethoxy-1,2,3,4-tetrahydroisoquinoline and 2-bromoacetyl bromide with 2-ethoxy-benzyl-amine LC-MS: rt = 4.4 min, 535 (M+1, ES+).

**Example 137**

**2-[1-(3,4-Dimethoxy-benzyl)-6,8-dimethoxy-3,4-dihydro-1*H*-isoquinolin-2-yl]-*N*-(pyridin-2-yl-methyl)-acetamide:**

prepared by reaction of 1-(3,4-Dimethoxy-benzyl)-6,8-dimethoxy-1,2,3,4-tetrahydroisoquinoline and 2-bromoacetyl bromide with 2-picolylamine

LC-MS: rt = 4.2 min, 492 (M+1, ES+).

**Example 138**

**2-[1-(2,3,4-Trimethoxy-benzyl)-6,7-dimethoxy-3,4-dihydro-1*H*-isoquinolin-2-yl]-*N*-benzyl-acetamide:**

prepared by reaction of 1-(2,3,4-Trimethoxy-benzyl)-6,7-dimethoxy-1,2,3,4-tetrahydroisoquinoline and 2-bromoacetyl bromide with benzylamine

LC-MS: rt = 3.9 min, 521 (M+1, ES+).

**Example 139**

**2-[1-(2,3,4-Trimethoxy-benzyl)-6,7-dimethoxy-3,4-dihydro-1*H*-isoquinolin-2-yl]-*N*-(naphthalen-1-yl-methyl)-acetamide:**

prepared by reaction of 1-(2,3,4-Trimethoxy-benzyl)-6,7-dimethoxy-1,2,3,4-tetrahydroisoquinoline and 2-bromoacetyl bromide with naphthalen-1-yl-methylamine

LC-MS: rt = 4.3 min, 571 (M+1, ES+).

**Example 140**

**2-[1-(2,3,4-Trimethoxy-benzyl)-6,7-dimethoxy-3,4-dihydro-1*H*-isoquinolin-2-yl]-*N*-(2-methoxy-benzyl)-acetamide:**

prepared by reaction of 1-(2,3,4-Trimethoxy-benzyl)-6,7-dimethoxy-1,2,3,4-tetrahydroisoquinoline and 2-bromoacetyl bromide with 2-methoxy-benzyl-amine

LC-MS: rt = 4.0 min, 551 (M+1, ES+).

**Example 141**

**2-[1-(2,3,4-Trimethoxy-benzyl)-6,7-dimethoxy-3,4-dihydro-1*H*-isoquinolin-2-yl]-*N*-(2-ethoxy-benzyl)-acetamide:**

prepared by reaction of 1-(2,3,4-Trimethoxy-benzyl)-6,7-dimethoxy-1,2,3,4-tetrahydroisoquinoline and 2-bromoacetyl bromide with 2-ethoxy-benzyl-amine

LC-MS: rt = 4.1 min, 565 (M+1, ES+).

**Example 142**

**2-[1-(2,3,4-Trimethoxy-benzyl)-6,7-dimethoxy-3,4-dihydro-1*H*-isoquinolin-2-yl]-*N*-(2-phenyl-ethyl)-acetamide:**

prepared by reaction of 1-(2,3,4-Trimethoxy-benzyl)-6,7-dimethoxy-1,2,3,4-tetrahydroisoquinoline and 2-bromoacetyl bromide with 2-phenyl-ethylamine
LC-MS: rt = 4.0 min, 535 (M+1, ES+).

**Example 143**

**2-[1-(Naphthalen-2-yl-methyl)-6,7-dimethoxy-3,4-dihydro-1*H*-isoquinolin-2-yl]-*N*-benzyl-acetamide:**

prepared by reaction of 1-(Naphthalen-2-yl-methyl)-6,7-dimethoxy-1,2,3,4-tetrahydroisoquinoline and 2-bromoacetyl bromide with benzylamine
LC-MS: rt = 4.5 min, 481 (M+1, ES+).

**Example 144**

**2-[1-(Naphthalen-2-yl-methyl)-6,7-dimethoxy-3,4-dihydro-1*H*-isoquinolin-2-yl]-*N*-(naphthalen-1-yl-methyl)-acetamide:**

prepared by reaction of 1-(Naphthalen-2-yl-methyl)-6,7-dimethoxy-1,2,3,4-tetrahydroisoquinoline and 2-bromoacetyl bromide with naphthalen-1-yl-methylamine
LC-MS: rt = 4.8 min, 531 (M+1, ES+).

**Example 145**

**2-[1-(Naphthalen-2-yl-methyl)-6,7-dimethoxy-3,4-dihydro-1*H*-isoquinolin-2-yl]-*N*-(2-methoxy-benzyl)-acetamide:**

prepared by reaction of 1-(Naphthalen-2-yl-methyl)-6,7-dimethoxy-1,2,3,4-tetrahydroisoquinoline and 2-bromoacetyl bromide with 2-methoxy-benzyl-amine
LC-MS: rt = 4.5 min, 511 (M+1, ES+).

**Example 146**

**2-[1-(Naphthalen-2-yl-methyl)-6,7-dimethoxy-3,4-dihydro-1*H*-isoquinolin-2-yl]-*N*-(2-ethoxy-benzyl)-acetamide:**

prepared by reaction of 1-(Naphthalen-2-yl-methyl)-6,7-dimethoxy-1,2,3,4-tetrahydroisoquinoline and 2-bromoacetyl bromide with 2-ethoxy-benzyl-amine
LC-MS: rt = 4.7 min, 525 (M+1, ES+).

**Example 147**

**2-[1-(Naphthalen-2-yl-methyl)-6,7-dimethoxy-3,4-dihydro-1*H*-isoquinolin-2-yl]-*N*-benzyl-*N*-methyl-acetamide:**

prepared by reaction of 1-(Naphthalen-2-yl-methyl)-6,7-dimethoxy-1,2,3,4-tetrahydroisoquinoline and 2-bromoacetyl bromide with *N*-benzyl-*N*-methyl-amine LC-MS: rt = 4.2 min, 495 (M+1, ES+).

**Example 148**

**2-[1-(Naphthalen-2-yl-methyl)-6,7-dimethoxy-3,4-dihydro-1*H*-isoquinolin-2-yl]-*N*-(pyridin-2-yl-methyl)-acetamide:**

prepared by reaction of 1-(Naphthalen-2-yl-methyl)-6,7-dimethoxy-1,2,3,4-tetrahydroisoquinoline and 2-bromoacetyl bromide with 2-picolylamine
LC-MS: rt = 4.4 min, 482 (M+1, ES+).

### Example 149

**2-[1-(3-Bromo-4-methoxy-benzyl)-6,7-dimethoxy-3,4-dihydro-1*H*-isoquinolin-2-yl]-*N*-benzyl-acetamide:**

prepared by reaction of 1-(3-Bromo-4-methoxy-benzyl)-6,7-dimethoxy-1,2,3,4-tetrahydroisoquinoline and 2-bromoacetyl bromide with benzylamine

LC-MS: rt = 4.3 min, 539 (M+1, ES+).

### Example 150

**2-[1-(3-Bromo-4-methoxy-benzyl)-6,7-dimethoxy-3,4-dihydro-1*H*-isoquinolin-2-yl]-*N*-(naphthalen-1-yl-methyl)-acetamide:**

prepared by reaction of 1-(3-Bromo-4-methoxy-benzyl)-6,7-dimethoxy-1,2,3,4-tetrahydroisoquinoline and 2-bromoacetyl bromide with naphthalen-1-yl-methylamine

LC-MS: rt = 4.7 min, 589 (M+1, ES+).

### Example 151

**2-[1-(3-Bromo-4-methoxy-benzyl)-6,7-dimethoxy-3,4-dihydro-1*H*-isoquinolin-2-yl]-*N*-(2-ethoxy-benzyl)-acetamide:**

prepared by reaction of 1-(3-Bromo-4-methoxy-benzyl)-6,7-dimethoxy-1,2,3,4-tetrahydroisoquinoline and 2-bromoacetyl bromide with 2-ethoxy-benzylamine

LC-MS: rt = 4.6 min, 583 (M+1, ES+).

### Example 152

**2-[1-(3-Bromo-4-methoxy-benzyl)-6,7-dimethoxy-3,4-dihydro-1*H*-isoquinolin-2-yl]-*N*-(pyridin-2-yl-methyl)-acetamide:**

prepared by reaction of 1-(3-Bromo-4-methoxy-benzyl)-6,7-dimethoxy-1,2,3,4-tetrahydroisoquinoline and 2-bromoacetyl bromide with 2-picolylamine

LC-MS: rt = 3.6 min, 541 (M+1, ES+).

### Example 153

**2-[1-(3,4-Methylenedioxy-benzyl)-5,8-dimethoxy-3,4-dihydro-1*H*-isoquinolin-2-yl]-*N*-(pyridin-2-yl-methyl)-acetamide:**

prepared by reaction of 1-(3,4-Methylenedioxy-benzyl)-5,8-dimethoxy-1,2,3,4-tetrahydroisoquinoline and 2-bromoacetyl bromide with 2-picolylamine

LC-MS: rt = 3.8 min, 476 (M+1, ES+).

### Example 154

**2-[1-(3,4-Methylenedioxy-benzyl)-5,8-dimethoxy-3,4-dihydro-1*H*-isoquinolin-2-yl]-*N*-(2-methoxy-benzyl)-acetamide:**

prepared by reaction of 1-(3,4-Methylenedioxy-benzyl)-5,8-dimethoxy-1,2,3,4-tetrahydroisoquinoline and 2-bromoacetyl bromide with 2-methoxy-benzylamine

LC-MS: rt = 4.6 min, 505 (M+1, ES+).

**Example 155**

**2-[1-(3,4-Methylenedioxy-benzyl)-5,8-dimethoxy-3,4-dihydro-1*H*-isoquinolin-2-yl]-*N*-[1,3,4]thiadiazol-2-yl-acetamide:**

prepared by reaction of 1-(3,4-Methylenedioxy-benzyl)-5,8-dimethoxy-1,2,3,4-tetrahydroisoquinoline and 2-bromoacetyl bromide with 2-amino-1,3,4-thiadiazole

LC-MS: rt = 4.4 min, 469 (M+1, ES+).

**Example 156**

**2-[1-(3,4-Methylenedioxy-benzyl)-5,8-dimethoxy-3,4-dihydro-1*H*-isoquinolin-2-yl]-*N*-(1*H*-benzoimidazol-2-yl-methyl)-acetamide:**

prepared by reaction of 1-(3,4-Methylenedioxy-benzyl)-5,8-dimethoxy-1,2,3,4-tetrahydroisoquinoline and 2-bromoacetyl bromide with 2-(aminomethyl)-benzimidazole

LC-MS: rt = 3.8 min, 515 (M+1, ES+).

**Example 157**

**2-[1-(3,4-Methylenedioxy-benzyl)-5,8-dimethoxy-3,4-dihydro-1*H*-isoquinolin-2-yl]-*N*-(*1H*-indazol-6-yl)-acetamide:**

prepared by reaction of 1-(3,4-Methylenedioxy-benzyl)-5,8-dimethoxy-1,2,3,4-tetrahydroisoquinoline and 2-bromoacetyl bromide with 6-aminoindazole

LC-MS: rt = 4.4 min, 501 (M+1, ES+).

Analogous to the above mentioned procedure, but in larger scale, the following tetrahydroisoquinoline derivatives were synthesized:

**Example 158**

**2-[1-(3,4-Dimethoxy-benzyl)-6-benzyloxy-7-methoxy-3,4-dihydro-1*H*-isoquinolin-2-yl]-*N*-benzyl-acetamide:**

prepared by reaction of 1-(3,4-Dimethoxy-benzyl)-6-benzyloxy-7-methoxy-1,2,3,4-tetrahydroisoquinoline and 2-bromoacetyl bromide with benzylamine

LC-MS: rt = 4.5 min, 567 (M+1, ES+).

**Example 159**

**2-[1-(3,4-Dimethoxy-benzyl)-7-benzyloxy-6-methoxy-3,4-dihydro-1*H*-isoquinolin-2-yl]-*N*-benzyl-acetamide:**

prepared by reaction of 1-(3,4-Dimethoxy-benzyl)-7-benzyloxy-6-methoxy-1,2,3,4-tetrahydroisoquinoline and 2-bromoacetyl bromide with benzylamine

LC-MS: rt = 4.4 min, 567 (M+1, ES+).

**Example 160**

**2-[1-(3,4-Dimethoxy-benzyl)-5-benzyloxy-8-methoxy-3,4-dihydro-1*H*-isoquinolin-2-yl]-*N*-(pyridin-2-yl-methyl)-acetamide:**

prepared by reaction of 1-(3,4-Dimethoxy-benzyl)-5-benzyloxy-8-methoxy-1,2,3,4-tetrahydroisoquinoline and 2-bromoacetyl bromide with 2-picolylamine

LC-MS: rt = 4.4 min, 568 (M+1, ES+).

**2-[1-(3,4-Dimethoxy-benzyl)-8-benzyloxy-5-methoxy-3,4-dihydro-1*H*-isoquinolin-2-yl]-*N*-(pyridin-2-yl-methyl)-acetamide:**

prepared by reaction of 1-(3,4-Dimethoxy-benzyl)-8-benzyloxy-5-methoxy-1,2,3,4-tetrahydroisoquinoline and

2-bromoacetyl bromide with 2-picolylamine
LC-MS: rt = 4.4 min, 568 (M+1, ES+).

**Example 161**

**2-[1-(4-Hydroxy-3-methoxy-benzyl)-6,7-dimethoxy-3,4-dihydro-1*H*-isoquinolin-2-yl]-*N*-benzyl-acetamide:**

prepared by reaction of 1-(4-Hydroxy-3-methoxy-benzyl)-6,7-dimethoxy-1,2,3,4-tetrahydroisoquinoline and 2-bromoacetyl bromide with benzylamine
LC-MS: rt = 3.4 min, 477 (M+1, ES+).

**2-[1-(3-Benzyloxy-4-methoxy-benzyl)-6,7-dimethoxy-3,4-dihydro-1*H*-isoquinolin-2-yl]-*N*-benzyl-acetamide:**

prepared by reaction of 1-(3-Benzyloxy-4-methoxy-benzyl)-6,7-dimethoxy-1,2,3,4-tetrahydroisoquinoline and 2-bromoacetyl bromide with benzylamine
LC-MS: rt = 4.4 min, 567 (M+1, ES+).

**C Coupling of Phenols with Alkylbromides, Heteroarylchlorides, Heteroaryl-methyl-sulfones and Carbamoylchlorides**

**C.1 Starting materials: Deprotection of Benzylic ethers:**

To a mixture of MeOH (60 mL) and formic acid (11.0 mL) was added Palladium (10% Pd/C, wet, 274 mg). The respective benzylic ether (4.0 mmol) was added portionwise and the mixture was stirred for 40 h. During this period further portions of Pd/C were added until the starting material was consumed. The mixture was filtered, the solvent was removed in vacuo and the residue was purified by flash-chromatography to give the following phenols:

**Example 162**

**2-[1-(3,4-dimethoxy-benzyl)-6-hydroxy-7-methoxy-3,4-dihydro-1*H*-isoquinolin-2-yl]-*N*-benzyl-acetamide:**

prepared by deprotection of 2-[1-(3,4-dimethoxy-benzyl)-6-benzyloxy-7-methoxy-3,4-dihydro-1*H*-isoquinolin-2-yl]-*N*-benzyl-acetamide
LC-MS: rt = 3.5 min, 477 (M+1, ES+).

**Example 163**

**2-[1-(3,4-dimethoxy-benzyl)-7-hydroxy-6-methoxy-3,4-dihydro-1*H*-isoquinolin-2-yl]-*N*-benzyl-acetamide:**

prepared by deprotection of 2-[1-(3,4-dimethoxy-benzyl)-7-benzyloxy-6-methoxy-3,4-dihydro-1*H*-isoquinolin-2-yl]-*N*-benzyl-acetamide
LC-MS: rt = 3.5 min, 477 (M+1, ES+).

**2-[1-(3,4-dimethoxy-benzyl)-5-hydroxy-8-methoxy-3,4-dihydro-1*H*-isoquinolin-2-yl]-*N*-(pyridin-2-yl-methyl)-acetamide:**

prepared by deprotection of 2-[1-(3,4-dimethoxy-benzyl)-5-benzyloxy-8-methoxy-3,4-dihydro-1*H*-isoquinolin-2-yl]-*N*-(pyridin-2-yl-methyl)-acetamide
LC-MS: rt = 3.2 min, 478 (M+1, ES+), 476 (M-1, ES-).

**2-[1-(3,4-dimethoxy-benzyl)-8-hydroxy-5-methoxy-3,4-dihydro-1*H*-isoquinolin-2-yl]-*N*-(pyridin-2-yl-methyl)-acetamide:**

prepared by deprotection of 2-[1-(3,4-dimethoxy-benzyl)-8-benzyloxy-5-methoxy-3,4-dihydro-1*H*-isoquinolin-2-yl]-*N*-(pyridin-2-yl-methyl)-acetamide
LC-MS: rt = 3.3 min, 478 (M+1, ES+), 476 (M-1, ES-).

**Example 164**

**2-[1-(3-Hydroxy-4-methoxy-benzyl)-6,7-dimethoxy-3,4-dihydro-1*H*-isoquinolin-2-yl]-*N*-benzyl-acetamide:**

prepared by deprotection of 2-[1-(3-Benzyloxy-4-methoxy-benzyl)-6,7-dimethoxy-3,4-dihydro-1*H*-isoquinolin-2-yl]-*N*-benzyl-acetamide:
LC-MS: rt = 3.5 min, 477 (M+1, ES+), 475 (M-1, ES-).

**C.2 Alkylation of Phenols with Alkylbromides (general procedure):**

At RT a solution of the respective phenol in DMF (250 µL, 0.40 M) was added to $K_2CO_3$ (70 mg). The reaction mixture was treated with a solution of the respective alkyl bromide in DMF (150 µL, 1.00 M), shaken at 100°C for 90 min and cooled to RT. After addition of another portion of alkyl bromide (150 µL, 1.00 M), shaking (100°C, 90 min) and cooling to RT a solution of triethylamine in THF (250 µL, 2.0 M) was added and the mixture was shaken for 14 h. Water (2.0 mL) and ethyl acetate (2.0 mL) were added, the phases were separated and the aqueous phase was extracted two times with ethyl acetate. The combined organic phases were concentrated in vacuo to give the following tetrahydroisoquinoline derivatives:

**Example 165**

**2-[1-(3,4-dimethoxy-benzyl)-6-ethoxy-7-methoxy-3,4-dihydro-1*H*-isoquinolin-2-yl]-*N*-benzyl-acetamide:**

prepared by reaction of 2-[1-(3,4-dimethoxy-benzyl)-6-hydroxy-7-methoxy-3,4-dihydro-1*H*-isoquinolin-2-yl]-*N*-benzyl-acetamide with ethyl iodide
LC-MS: rt = 3.8 min, 505 (M+1, ES+).

**Example 166**

**2-[1-(3,4-dimethoxy-benzyl)-6-propoxy-7-methoxy-3,4-dihydro-1*H*-isoquinolin-2-yl]-*N*-benzyl-acetamide:**

prepared by reaction of 2-[1-(3,4-dimethoxy-benzyl)-6-hydroxy-7-methoxy-3,4-dihydro-1*H*-isoquinolin-2-yl]-*N*-benzyl-acetamide with propyl bromide
LC-MS: rt = 4.1 min, 519 (M+1, ES+).

**Example 167**

**2-[1-(3,4-dimethoxy-benzyl)-6-allyloxy-7-methoxy-3,4-dihydro-1*H*-isoquinolin-2-yl]-*N*-benzyl-acetamide:**

prepared by reaction of 2-[1-(3,4-dimethoxy-benzyl)-6-hydroxy-7-methoxy-3,4-dihydro-1*H*-isoquinolin-2-yl]-*N*-benzyl-acetamide with allyl bromide
LC-MS: rt = 4.0 min, 517 (M+1, ES+).

**Example 168**

**2-[1-(3,4-dimethoxy-benzyl)-7-ethoxy-6-methoxy-3,4-dihydro-1*H*-isoquinolin-2-yl]-*N*-benzyl-acetamide:**

prepared by reaction of 2-[1-(3,4-dimethoxy-benzyl)-7-hydroxy-6-methoxy-3,4-dihydro-1*H*-isoquinolin-2-yl]-*N*-benzyl-acetamide with ethyl iodide
LC-MS: rt = 3.8 min, 505 (M+1, ES+).

**Example 169**

**2-[1-(3,4-dimethoxy-benzyl)-7-propoxy-6-methoxy-3,4-dihydro-1*H*-isoquinolin-2-yl]-*N*-benzyl-acetamide:**

prepared by reaction of 2-[1-(3,4-dimethoxy-benzyl)-7-hydroxy-6-methoxy-3,4-dihydro-1*H*-isoquinolin-2-yl]-*N*-benzyl-acetamide with propyl bromide
LC-MS: rt = 4.0 min, 519 (M+1, ES+).

**Example 170**

**2-[1-(3,4-dimethoxy-benzyl)-7-butoxy-6-methoxy-3,4-dihydro-1*H*-isoquinolin-2-yl]-*N*-benzyl-acetamide:**

prepared by reaction of 2-[1-(3,4-dimethoxy-benzyl)-7-hydroxy-6-methoxy-3,4-dihydro-1*H*-isoquinolin-2-yl] -*N*-benzyl-acetamide with butyl bromide
LC-MS: rt = 4.2 min, 533 (M+1, ES+).

**Example 171**

**2-[1-(3,4-dimethoxy-benzyl)-7-allyloxy-6-methoxy-3,4-dihydro-1*H*-isoquinolin-2-yl]-*N*-benzyl-acetamide:**

prepared by reaction of 2-[1-(3,4-dimethoxy-benzyl)-7-hydroxy-6-methoxy-3,4-dihydro-1*H*-isoquinolin-2-yl] -*N*-benzyl-acetamide with allyl bromide
LC-MS: rt = 3.9 min, 517 (M+1, ES+).

**Example 172**

**2-[1-(3,4-dimethoxy-benzyl)-5-ethoxy-8-methoxy-3,4-dihydro-1*H*-isoquinolin-2-yl]-*N*-(pyridin-2-yl-methyl)-acetamide:**

prepared by reaction of 2-[1-(3,4-dimethoxy-benzyl)-5-hydroxy-8-methoxy-3,4-dihydro-1*H*-isoquinolin-2-yl] -*N*-(pyridin-2-yl-methyl)-acetamide with ethyl iodide LC-MS: rt = 0.61 min, 506 (M+1, ES+).

**Example 173**

**2-[1-(3,4-dimethoxy-benzyl)-5-propoxy-8-methoxy-3,4-dihydro-1*H*-isoquinolin-2-yl]-*N*-(pyridin-2-yl-methyl)-acetamide:**

prepared by reaction of 2-[1-(3,4-dimethoxy-benzyl)-5-hydroxy-8-methoxy-3,4-dihydro-1*H*-isoquinolin-2-yl] -*N*-(pyridin-2-yl-methyl)-acetamide with propyl bromide
LC-MS: rt = 0.66 min, 520 (M+1, ES+).

**Example 174**

**2-[1-(3,4-dimethoxy-benzyl)-5-allyloxy-8-methoxy-3,4-dihydro-1*H*-isoquinolin-2-yl]-*N*-(pyridin-2-yl-methyl)-acetamide:**

prepared by reaction of 2-[1-(3,4-dimethoxy-benzyl)-5-hydroxy-8-methoxy-3,4-dihydro-1*H*-isoquinolin-2-yl] -*N*-(pyridin-2-yl-methyl)-acetamide with allyl bromide
LC-MS: rt = 0.63 min, 518 (M+1, ES+).

**Example 175**

**2-[1-(3,4-dimethoxy-benzyl)-5-isopropoxy-8-methoxy-3,4-dihydro-1*H*-isoquinolin-2-yl]-*N*-(pyridin-2-yl-methyl)-acetamide:**

prepared by reaction of 2-[1-(3,4-dimethoxy-benzyl)-5-hydroxy-8-methoxy-3,4-dihydro-1*H*-isoquinolin-2-yl] -*N*-(pyridin-2-yl-methyl)-acetamide with isopropyl bromide
LC-MS: rt = 0.64 min, 520 (M+1, ES+).

**Example 176**

**2-[1-(3,4-dimethoxy-benzyl)-5-butoxy-8-methoxy-3,4-dihydro-1*H*-isoquinolin-2-yl]-*N*-(pyridin-2-yl-methyl)-acetamide:**

prepared by reaction of 2-[1-(3,4-dimethoxy-benzyl)-5-hydroxy-8-methoxy-3,4-dihydro-1*H*-isoquinolin-2-yl] -*N*-(pyridin-2-yl-methyl)-acetamide with butyl bromide

LC-MS: rt = 0.70 min, 534 (M+1, ES+).

**Example 177**

**2-[1-(3,4-dimethoxy-benzyl)-5-isobutoxy-8-methoxy-3,4-dihydro-1*H*-isoquinolin-2-yl]-*N*-(pyridin-2-yl-methyl)-acetamide:**

prepared by reaction of 2-[1-(3,4-dimethoxy-benzyl)-5-hydroxy-8-methoxy-3,4-dihydro-1*H*-isoquinolin-2-yl]-*N*-(pyridin-2-yl-methyl)-acetamide with 1-bromo-2-methyl-propane

LC-MS: rt = 0.70 min, 534 (M+1, ES+).

**Example 178**

**2-[1-(3,4-dimethoxy-benzyl)-5-(but-2-oxy)-8-methoxy-3,4-dihydro-1*H*-isoquinolin-2-yl]-*N*-(pyridin-2-yl-methyl)-acetamide:**

prepared by reaction of 2-[1-(3,4-dimethoxy-benzyl)-5-hydroxy-8-methoxy-3,4-dihydro-1*H*-isoquinolin-2-yl]-*N*-(pyridin-2-yl-methyl)-acetamide with 2-bromo-butane

LC-MS: rt = 0.68 min, 534 (M+1, ES+).

**Example 179**

**2-[1-(3,4-dimethoxy-benzyl)-8-ethoxy-5-methoxy-3,4-dihydro-1*H*-isoquinolin-2-yl]-*N*-(pyridin-2-yl-methyl)-acetamide:**

prepared by reaction of 2-[1-(3,4-dimethoxy-benzyl)-8-hydroxy-5-methoxy-3,4-dihydro-1*H*-isoquinolin-2-yl]-*N*-(pyridin-2-yl-methyl)-acetamide with ethyl iodide

LC-MS: rt = 0.62 min, 506 (M+1, ES+).

**Example 180**

**2-[1-(3,4-dimethoxy-benzyl)-8-propoxy-5-methoxy-3,4-dihydro-1*H*-isoquinolin-2-yl]-*N*-(pyridin-2-yl-methyl)-acetamide:**

prepared by reaction of 2-[1-(3,4-dimethoxy-benryl)-8-hydroxy-5-methoxy-3,4-dihydro-1*H*-isoquinolin-2-yl]-*N*-(pyridin-2-yl-methyl)-acetamide with propyl bromide

LC-MS: rt = 0.66 min, 520 (M+1, ES+).

**Example 181**

**2-[1-(3,4-dimethoxy-benzyl)-8-allyloxy-5-methoxy-3,4-dihydro-1*H*-isoquinolin-2-yl]-*N*-(pyridin-2-yl-methyl)-acetamide:**

prepared by reaction of 2-[1-(3,4-dimethoxy-benzyl)-8-hydroxy-5-methoxy-3,4-dihydro-1*H*-isoquinolin-2-yl]-*N*-(pyridin-2-yl-methyl)-acetamide with allyl bromide

LC-MS: rt = 0.63 min, 518 (M+1, ES+).

**Example 182**

**2-[1-(3,4-dimethoxy-benzyl)-8-isopropoxy-5-methoxy-3,4-dihydro-1*H*-isoquinolin-2-yl]-*N*-(pyridin-2-yl-methyl)-acetamide:**

prepared by reaction of 2-[1-(3,4-dimethoxy-benzyl)-8-hydroxy-5-methoxy-3,4-dihydro-1*H*-isoquinolin-2-yl]-*N*-(pyridin-2-yl-methyl)-acetamide with isopropyl bromide

LC-MS: rt = 0.64 min, 520 (M+1, ES+).

**Example 183**

**2-[1-(3,4-dimethoxy-benzyl)-8-butoxy-5-methoxy-3,4-dihydro-1*H*-isoquinolin-2-yl]-*N*-(pyridin-2-yl-methyl)-acetamide:**

prepared by reaction of 2-[1-(3,4-dimethoxy-benzyl)-8-hydroxy-5-methoxy-3,4-dihydro-1*H*-isaquinolin-2-yl]-*N*-(pyridin-2-yl-methyl)-acetamide with butyl bromide
LC-MS: rt = 0.69 min, 534 (M+1, ES+).

**Example 184**

**2-[1-(3,4-dimethoxy-benzyl)-8-isobutoxy-5-methoxy-3,4-dihydro-1*H*-isoquinolin-2-yl]-*N*-(pyridin-2-yl-methyl)-acetamide:**

prepared by reaction of 2-[1-(3,4-dimethoxy-benzyl)-8-hydroxy-5-methoxy-3,4-dihydro-1*H*-isoquinolin-2-yl]-*N*-(pyridin-2-yl-methyl)-acetamide with 1-bromo-2-methyl-propane
LC-MS: rt = 0.69 min, 534 (M+1, ES+).

**Example 185**

**2-[1-(3,4-dimethoxy-benzyl)-8-(but-2-oxy)-5-methoxy-3,4-dihydro-1*H*-isoquinolin-2-yl]-*N*-(pyridin-2-yl-methyl)-acetamide:**

prepared by reaction of 2-[1-(3,4-dimethoxy-benzyl)-8-hydroxy-5-methoxy-3,4-dihydro-1*H*-isoquinolin-2-yl]-*N*-(pyridin-2-yl-methyl)-acetamide with 2-bromo-butane
LC-MS: rt = 0.68 min, 534 (M+1, ES+).

**Example 186**

**2-[1-(3,4-dimethoxy-benzyl)-8-cyclohexyloxy-5-methoxy-3,4-dihydro-1*H*-isoquinolin-2-yl]-*N*-(pyridin-2-yl-methyl)-acetamide:**

prepared by reaction of 2-[1-(3,4-dimethoxy-benzyl)-8-hydroxy-5-methoxy-3,4-dihydro-1*H*-isoquinolin-2-yl]-*N*-(pyridin-2-yl-methyl)-acetamide with cyclohexyl bromide
LC-MS: rt = 0.73 min, 560 (M+1, ES+).

**Example 187**

**2-[1-(4-ethoxy-3-methoxy-benzyl)-6,7-dimethoxy-3,4-dihydro-1*H*-isoquinolin-2-yl]-*N*-benzyl-acetamide:**

prepared by reaction of 2-[1-(4-hydroxy-3-methoxy-benzyl)-6,7-dimethoxy-3,4-dihydro-1*H*-isoquinolin-2-yl]-*N*-benzyl-acetamide with ethyl iodide
LC-MS: rt = 3.9 min, 505 (M+1, ES+).

**Example 188**

**2-[1-(4-propoxy-3-methoxy-benzyl)-6,7-dimethoxy-3,4-dihydro-1*H*-isoquinolin-2-yl]-*N*-benzyl-acetamide:**

prepared by reaction of 2-[1-(4-hydroxy-3-methoxy-benzyl)-6,7-dimethoxy-3,4-dihydro-1*H*-isoquinolin-2-yl]-*N*-benzyl-acetamide with propyl bromide
LC-MS: rt = 4.2 min, 519 (M+1, ES+).

**Example 189**

**2-[1-(4-butoxy-3-methoxy-benzyl)-6,7-dimethoxy-3,4-dihydro-1*H*-isoquinolin-2-yl]-*N*-benzyl-acetamide:**

prepared by reaction of 2-[1-(4-hydroxy-3-methoxy-benzyl)-6,7-dimethoxy-3,4-dihydro-1*H*-isoquinolin-2-yl]-*N*-benzyl-acetamide with butyl bromide

LC-MS: rt = 4.4 min, 533 (M+1, ES+).

**Example 190**

**2-[1-(4-allyloxy-3-methoxy-benzyl)-6,7-dimethoxy-3,4-dihydro-1*H*-isoquinolin-2-yl]-*N*-benzyl-acetamide:**

prepared by reaction of 2-[1-(4-hydroxy-3-methoxy-benzyl)-6,7-dimethoxy-3,4-dihydro-1*H*-isoquinolin-2-yl]-*N*-benzyl-acetamide with allyl bromide

LC-MS: rt = 4.0 min, 517 (M+1, ES+).

**Example 191**

**2-[1-(4-isopropoxy-3-methoxy-benzyl)-6,7-dimethoxy-3,4-dihydro-1*H*-isoquinolin-2-yl]-*N*-benzyl-acetamide:**

prepared by reaction of 2-[1-(4-hydroxy-3-methoxy-benzyl)-6,7-dimethoxy-3,4-dihydro-1*H*-isoquinolin-2-yl]-*N*-benzyl-acetamide with isopropyl bromide

LC-MS: rt = 4.0 min, 519 (M+1, ES+).

**Example 192**

**2-[1-(4-isobutoxy-3-methoxy-benzyl)-6,7-dimethoxy-3,4-dihydro-1*H*-isoquinolin-2-yl]-*N*-benzyl-acetamide:**

prepared by reaction of 2-[1-(4-hydroxy-3-methoxy-benzyl)-6,7-dimethoxy-3,4-dihydro-1*H*-isoquinolin-2-yl]-*N*-benzyl-acetamide with 1-bromo-2-methyl-propane

LC-MS: rt = 4.5 min, 533 (M+1, ES+).

**Example 193**

**2-[1-(4-(cyclopropyl-methoxy)-3-methoxy-benzyl)-6,7-dimethoxy-3,4-dihydro-1*H*-isoquinolin-2-yl]-*N*-benzyl-acetamide:**

prepared by reaction of 2-[1-(4-hydroxy-3-methoxy-benzyl)-6,7-dimethoxy-3,4-dihydro-1*H*-isoquinolin-2-yl]-*N*-benzyl-acetamide with cyclopropyl-methyl bromide

LC-MS: rt = 4.2 min, 531 (M+1, ES+).

**Example 194**

**{4-[2-(Benzylcarbamoyl-methyl)-6,7-dimethoxy-1,2,3,4-tetrahydro-isoquinolin-1-ylmethyl]-2-methoxy-phenoxy}-acetic acid ethyl ester**

prepared by reaction of 2-[1-(4-hydroxy-3-methoxy-benzyl)-6,7-dimethoxy-3,4-dihydro-1*H*-isoquinolin-2-yl]-*N*-benzyl-acetamide with ethyl bromoacetate

LC-MS: rt = 3.9 min, 563 (M+1, ES+).

**Example 195**

**2-[1-(3-ethoxy-4-methoxy-benzyl)-6,7-dimethoxy-3,4-dihydro-1*H*-isoquinolin-2-yl]-*N*-benzyl-acetamide:**

prepared by reaction of 2-[1-(3-hydroxy-4-methoxy-benzyl)-6,7-dimethoxy-3,4-dihydro-1*H*-isoquinolin-2-yl]-*N*-benzyl-acetamide with ethyl iodide

LC-MS: rt = 3.8 min, 505 (M+1, ES+).

**Example 196**

**2-[1-(3-propoxy-4-methoxy-benzyl)-6,7-dimethoxy-3,4-dihydro-1*H*-isoquinolin-2-yl]-*N*-benzyl-acetamide:**

prepared by reaction of 2-[1-(3-hydroxy-4-methoxy-benzyl)-6,7-dimethoxy-3,4-dihydro-1*H*-isoquinolin-2-yl]-*N*-benzyl-acetamide with propyl bromide

LC-MS: rt = 4.1 min, 519 (M+1, ES+).

**Example 197**

**2-[1-(3-allyloxy-4-methoxy-benzyl)-6,7-dimethoxy-3,4-dihydro-1*H*-isoquinolin-2-yl]-*N*-benzyl-acetamide:**

prepared by reaction of 2-[1-(3-hydroxy-4-methoxy-benzyl)-6,7-dimethoxy-3,4-dihydro-1*H*-isoquinolin-2-yl]-*N*-benzyl-acetamide with allyl bromide

LC-MS: rt = 4.0 min, 517 (M+1, ES+).

**Example 198**

**2-[1-(3-isopropoxy-4-methoxy-benzyl)-6,7-dimethoxy-3,4-dihydro-1*H*-isoquinolin-2-yl]-*N*-benzyl-acetamide:**

prepared by reaction of 2-[1-(3-hydroxy-4-methoxy-benzyl)-6,7-dimethoxy-3,4-dihydro-1*H*-isoquinolin-2-yl]-*N*-benzyl-acetamide with isopropyl bromide

LC-MS: rt = 4.0 min, 519 (M+1, ES+).

**Example 199**

**2-[1-(3-butoxy-4-methoxy-benzyl)-6,7-dimethoxy-3,4-dihydro-1*H*-isoquinolin-2-yl]-*N*-benzyl-acetamide:**

prepared by reaction of 2-[1-(3-hydroxy-4-methoxy-benzyl)-6,7-dimethoxy-3,4-dihydro-1*H*-isoquinolin-2-yl]-*N*-benzyl-acetamide with butyl bromide

LC-MS: rt = 4.3 min, 533 (M+1, ES+).

**Example 200**

**2-{1-[3-(but-2-oxy)-4-methoxy-benzyl]-6,7-dimethoxy-3,4-dihydro-1*H*-isoquinolin-2-yl}-*N*-benzyl-acetamide:**

prepared by reaction of 2-[1-(3-hydroxy-4-methoxy-benzyl)-6,7-dimethoxy-3,4-dihydro-1*H*-isoquinolin-2-yl]-*N*-benzyl-acetamide with 2-bromo-butane

LC-MS: rt = 4.2 min, 533 (M+1, ES+).

**Example 201**

**2-{1-[3-(cyclopropyl-methoxy)-4-methoxy-benzyl]-6,7-dimethoxy-3,4-dihydro-1*H*-isoquinolin-2-yl}-*N*-benzyl-acetamide:**

prepared by reaction of 2-[1-(3-hydroxy-4-methoxy-benzyl)-6,7-dimethoxy-3,4-dihydro-1*H*-isoquinolin-2-yl]-*N*-benzyl-acetamide with cyclopropyl-methyl bromide LC-MS: rt = 4.0 min, 531 (M+1, ES+).

**Example 202**

**2-{1-[3-(3-fluoro-propoxy)-4-methoxy-benzyl]-6,7-dimethoxy-3,4-dihydro-1*H*-isoquinolin-2-yl}-*N*-benzyl-acetamide:**

prepared by reaction of 2-[1-(3-hydroxy-4-methoxy-benzyl)-6,7-dimethoxy-3,4-dihydro-1*H*-isoquinolin-2-yl]-*N*-benzyl-acetamide with 1-bromo-3-fluoro-propane LC-MS: rt = 3.9 min, 537 (M+1, ES+).

**Example 203**

**2-[1-(3,4-dimethoxy-benzyl)-7-(1-methyl-prop-2-oxy)-6-methoxy-3,4-dihydro-1*H*-isoquinolin-2-yl]-*N*-benzyl-acetamide:**

At room temperature *tert*.-butyl 2,2,2-trichloroacetimidate (437 mg, 0.36 mL, 2.0 mmol) was added to a solution of 2-[1-(3,4-dimethoxy-benzyl)-7-hydroxy-6-methoxy-3,4-dihydro-1*H*-isoquinolin-2-yl]-*N*-benzyl-acetamide (95.3 mg, 0.2 mmol) in dichloromethane (5.0 mL) and cyclohexane (5.0 mL). The reaction mixture was treated with a solution of

boron trifluoride diethyl etherate (50 µL, 0.4 mmol) in 10 mL dichloromethane and stirred for 22 h. Another portion of *tert.*-butyl 2,2,2-trichloroacetimidate (244 mg, 0.20 mL, 1.1 mmol) was added. After stirring for three days a saturated solution of $NaHCO_3$ (10 mL), water (10 mL) and ethyl acetate (40 mL) were added, the phases were separated and the aqueous phase was extracted three times with ethyl acetate (30 mL). The combined organic phases were concentrated in vacuo and purified by flash-chromatography to give the titled product (80.4 mg, 75%) as pale yellow oil.
LC-MS: rt = 4.2 min, 533 (M+1, ES+).

**C.3 Reaktion of Phenols with Heteroaryl chlorides or Heteroaryl-methyl sulfones (general procedure):**

A solution of the respective heteroaryl chloride or methyl-sulfone in DMF (1.0 mL, 0.20 M) was added to a mixture of the respective phenol (0.15 mmol) and $K_2CO_3$ (75 mg). The reaction mixture was stirred at 100°C for 16 h. Water (2.0 mL) and ethyl acetate (2.0 mL) were added, the phases were separated and the aqueous phase was extracted two times with ethyl acetate. The combined organic phases were concentrated in vacuo to give the following tetrahydroisoquinoline derivatives:

**Example 204**

**2-{1-[3-(pyrimidin-2-yloxy)-4-methoxy-benzyl]-6,7-dimethoxy-3,4-dihydro-1*H*-isoquinolin-2-yl}-*N*-benzyl-acetamide:**

prepared by reaction of 2-[1-(3-hydroxy-4-methoxy-benzyl)-6,7-dimethoxy-3,4-dihydro-1*H*-isoquinolin-2-yl]-*N*-benzyl-acetamide with 2-chloro-pyrimidine
LC-MS: rt = 0.60 min, 555 (M+1, ES+).

**Example 205**

**2-{1-[4-(pyrimidin-2-yloxy)-3-methoxy-benzyl]-6,7-dimethoxy-3,4-dihydro-1*H*-isoquinolin-2-yl}-*N*-benzyl-acetamide:**

prepared by reaction of 2-[1-(4-hydroxy-3-methoxy-benzyl)-6,7-dimethoxy-3,4-dihydro-1*H*-isoquinolin-2-yl]-*N*-benzyl-acetamide with 2-chloro-pyrimidine
LC-MS: rt = 0.60 min, 555 (M+1, ES+).

**C.4 Reaktion of Phenols with Carbamoylchlarides (general procedure):**

A solution of the respective phenol (0.20 mmol) and triethylamine (0.30 mL, 2.15 mmol) in THF (1.0 mL) was treated with the respective carbamoylchloride (2.2 mmol) and stirred at reflux for 16 h. Water (2.0 mL) and ethyl acetate (2.0 mL) were added, the phases were separated and the aqueous phase was extracted two times with ethyl acetate. The combined organic phases were concentrated in vacuo to give the following tetrahydroisoquinoline derivatives:

**Example 206**

**2-{1-[4-Methoxy-3-(*N,N*-dimethylcarbamoyloxy)-benzyl]-6,7-dimethoxy-3,4-dihydro-1*H*-isoquinolin-2-yl}-*N*-benzyl-acetamide:**

prepared by reaction of 2-[1-(3-hydroxy-4-methoxy-benzyl)-6,7-dimethoxy-3,4-dihydro-1*H*-isoquinolin-2-yl]-*N*-benzyl-acetamide with *N,N*-dimethyl-carbamoyl chloride
LC-MS: rt = 0.62 min, 548 (M+1, ES+).

**Example 207**

**2-{1-[3-Methoxy-4-(*N,N*-dimethylcarbamoyloxy)-benzyl]-6,7-dimethoxy-3,4-dihydro-1*H*-isoquinolin-2-yl}-*N*-benzyl-acetamide:**

prepared by reaction of 2-[1-(4-hydroxy-3-methoxy-benzyl)-6,7-dimethoxy-3,4-dihydro-1*H*-isoquinolin-2-yl]-*N*-benzyl-acetamide with *N,N*-dimethyl-carbamoyl chloride
LC-MS: rt = 0.63 min, 548 (M+1, ES+).

**Example 208**

**2-{1-[3-Methoxy-4-(4-morpholine-carbonyloxy)-benzyl]-6,7-dimethoxy-3,4-dihydro-1*H*-isoquinolin-2-yl}-*N*-benzyl-acetamide:**

prepared by reaction of 2-[1-(4-hydroxy-3-methoxy-benzyl)-6,7-dimethoxy-3,4-dihydro-1*H*-isoquinolin-2-yl]-*N*-benzyl-acetamide with 4-morpholine-carbonyl chloride

LC-MS: rt = 0.61 min, 590 (M+1, ES+).

**D. General procedure for the preparation of the isonitrile derivatives**

Isonitriles (or isocyanides) have been prepared by reaction of the N-alkyl-formamides (formed by reaction of the corresponding amine with formic ethyl ester) with $POCl_3$.

**Abbreviations:**

| | |
|---|---|
| BSA | Bovine serum albumine |
| CHO | Chinese hamster ovary |
| DMF | Dimethylformamide |
| DMSO | Dimethylsulfoxide |
| ES | Electron spray |
| FCS | Foetal calf serum |
| FLIPR | Fluorescent imaging plate reader |
| HBSS | Hank's balanced salt solution |
| HEPES | 4-(2-Hydroxyethyl)-piperazine-1-ethanesulfonic acid |
| MeOH | Methanol |
| MS | Mass spectroscopy |
| LC | Liquid chromatography |
| PyBOP | Benzotriazole-1-yl-oxy-tris-pyrrolidino-Phosphoniumhexafluorophosphate |
| $R_f$ | Retention front |
| $R_t$ | retention time |
| RT | Room temperature |
| THF | Tetrahydrofuran |

**Claims**

**1.** Compounds of the general formula (I)

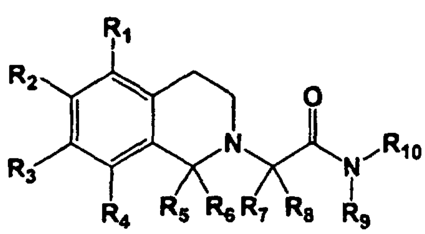

formula (I)

wherein:

$R^1$, $R^2$, $R^3$, $R^4$ independently represent cyano, nitro, halogen, hydrogen, hydroxy, lower alkyl, lower alkenyl, lower alkoxy, lower alkenyloxy, trifluoromethyl, trifluoromethoxy, cycloalkyloxy, aryloxy, aralkyloxy, heterocyclyloxy, heterocyclylalkyloxy, $R^{11}CO$-, $NR^{12}R^{13}CO$-, $R^{12}R^{13}N$-, $R^{11}OOC$-, $R^{11}SO_2NH$- or $R^{14}$-CO-NH- or $R^2$ and $R^3$ together as well as $R^1$ and $R^2$ together and $R^3$ and $R^4$ together may form with the phenyl ring a five, six or seven-membered ring containing one or two oxygen atoms;
$R^5$ represent aryl, aralkyl, lower alkenyl, trifluoromethyl, cycloalkyl, heterocyclyl or heterocyclyl-lower alkyl;
$R^6$ represent hydrogen, aryl, aralkyl, lower alkyl, lower alkenyl, trifluoromethyl, cycloalkyl, heterocyclyl or heterocyclyl-lower alkyl;
$R^7$ and $R^8$ independently represent hydrogen, aryl, aralkyl, lower alkyl, lower alkenyl, cycloalkyl, heterocyclyl or heterocyclyl-lower alkyl;
$R^9$ represents aryl, aralkyl, lower alkyl, lower alkenyl, trifluoromethyl, cycloalkyl, heterocyclyl or heterocyclyl-lower alkyl;
$R^{10}$ represents hydrogen, aryl, aralkyl, lower alkyl, lower alkenyl, trifluoromethyl, cycloalkyl, heterocyclyl or heterocyclyl-lower alkyl;
$R^{11}$ represents lower alkyl, aryl, aralkyl, heterocyclyl or heterocyclyl-lower alkyl;
$R^{12}$and $R^{13}$ independently represent hydrogen, alkyl, cycloalkyl, aryl, aralkyl, heterocyclyl or heterocyclyl-lower alkyl;
$R^{14}$ represents alkyl, aryl, cycloalkyl, heterocyclyl, $R^{12}R^{13}N$- or $R^{11}O$-

wherein the term "lower alkyl", alone or in combination, signifies a straight-chain or branched-chain alkyl group with 1 to 8 carbon atoms, the term "lower alkenyl", alone or in combination, signifies a straight-chain or a branched-chain alkenyl group with 2 to 5 carbon atoms, the term "cycloalkyl", alone or in combination, signifies a cycloalkyl ring with 3 to 8 carbon atoms; the term "aryl", alone or in combination, signifies a phenyl or a naphtyl group which optionally carries one or more substituents; and the term "heterocyclyl", alone or in combination, signifies a 5- to 10-membered monocyclic or bicyclic ring, which may be saturated, partially unsaturated or aromatic containing 1, 2 or 3 heteroatoms selected from oxygen, nitrogen and sulphur which may be the same or different and which may have up to 5 optional substituents;
and optically pure enantiomers, mixtures of enantiomers, racemates, optically pure diastereoisomers, mixtures of diastereoisomers, diastereoisomeric racemates, mixture of diastereoisomeric racemates, or meso forms and pharmaceutically acceptable salts thereof.

**2.** Compounds of the general formula (II) according to the general formula (I) of claim 1

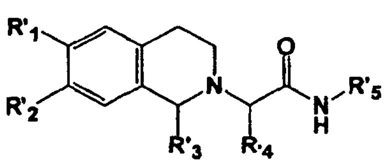

General formula II

wherein:

$R'^1$ and $R'^2$ independently represent hydrogen, hydroxy, methoxy or halogen or may form with the phenyl ring a five, six or seven membered-ring containing one or two oxygen atoms,
$R'^3$ represent aryl, aralkyl, lower alkenyl, cycloalkyl, heterocyclyl or heterocyclyl-lower alkyl;
$R'^4$ represents hydrogen, aryl, aralkyl, lower alkyl, lower alkenyl, cycloalkyl, heterocyclyl or heterocyclyl-lower alkyl;
$R'^5$ represents aryl, aralkyl, lower alkyl, lower alkenyl, cycloalkyl, heterocyclyl or heterocyclyl-lower alkyl;

and optically pure enantiomers, mixtures of enantiomers, racemates, optically pure diastereoisomers, mixtures of diastereoisomers, diastereoisomeric racemates, mixture of diastereoisomeric racemates, or meso forms and pharmaceutically acceptable salts thereof.

**3.** A compound according to any of claims 1 to 2, selected from the group consisting of:

2-[1-(3,4-Dimethoxy-benzyl)-5,8-dimethoxy-3,4-dihydro-1*H*-isoquinolin-2-yl]-*N*-(pyridin-2-yl-methyl)-acetamide;

2-[1-(3,4-dimethoxy-benzyl)-8-(cyclopropyl-methoxy)-5-methoxy-3,4-dihydro-1*H*-isoquinolin-2-yl]-*N*-(pyridin-2-yl-methyl)-acetamide;

2-[1-(3,4-dimethoxy-benzyl)-8-(2-fluoro-ethoxy)-5-methoxy-3,4-dihydro-1*H*-isoquinolin-2-yl]-*N*-(pyridin-2-yl-methyl)-acetamide;

2-[1-(3,4-dimethoxy-benzyl)-8-(2,2-difluoro-ethoxy)-5-methoxy-3,4-dihydro-1*H*-isoquinolin-2-yl]-*N*-(pyridin-2-yl-methyl)-acetamide;

2-[1-(3,4-dimethoxy-benzyl)-8-ethoxy-5-methoxy-3,4-dihydro-1*H*-isoquinolin-2-yl]-*N*-(pyridin-2-yl-methyl)-acetamide;

2-[1-(3,4-dimethoxy-benzyl)-8-propoxy-5-methoxy-3,4-dihydro-1*H*-isoquinolin-2-yl]-*N*-(pyridin-2-yl-methyl)-acetamide;

2-[1-(3,4-dimethoxy-benzyl)-8-allyloxy-5-methoxy-3,4-dihydro-1*H*-isoquinolin-2-yl]-*N*-(pyridin-2-yl-methyl)-acetamide;

2-[1-(3,4-dimethoxy-benzyl)-8-isopropoxy-5-methoxy-3,4-dihydro-1*H*-isoquinolin-2-yl]-*N*-(pyridin-2-yl-methyl)-acetamide;

2-[1-(3,4-dimethoxy-benzyl)-5-propoxy-8-methoxy-3,4-dihydro-1*H*-isoquinolin-2-yl]-*N*-(pyridin-2-yl-methyl)-acetamide;

2-[1-(3,4-Dimethoxy-benzyl)-6,7-dimethoxy-3,4-dihydro-1*H*-isoquinolin-2-yl]-*N*-benzyl-acetamide;

2-[1-(3,4-Dimethoxy-benzyl)-6,7-dimethoxy-3,4-dihydro-1*H*-isoquinolin-2-yl]-*N*-naphthalen-1-ylmethyl-acetamide;

2- {1-[4-(pyrimidin-2-yloxy)-3-methoxy-benzyl]-6,7-dimethoxy-3,4-dihydro-1*H*-isoquinolin-2-yl}-*N*-benzyl-acetamide;

2-[1-(3,4-dimethoxy-benzyl)-7-(1-methyl-prop-2-oxy)-6-methoxy-3,4-dihydro-1*H*-isoquinolin-2-yl]-*N*-benzyl-acetamide;

2-[1-(3,4-Dimethoxy-benzyl)-6-methoxy-7-isopropoxy-3,4-dihydro-1*H*-isoquinolin-2-yl]-*N*-benzyl-acetamide;

2-[1-(3,4-dimethoxy-benzyl)-7-ethoxy-6-methoxy-3,4-dihydro-1*H*-isoquinolin-2-yl]-*N*-benzyl-acetamide;

2-[1-(3,4-dimethoxy-benzyl)-7-propoxy-6-methoxy-3,4-dihydro-1*H*-isoquinolin-2-yl]-*N*-benzyl-acetamide;

2-[1-(3,4-dimethoxy-benzyl)-7-allyloxy-6-methoxy-3,4-dihydro-1*H*-isoquinolin-2-yl]-*N*-benzyl-acetamide;

*N*-benzyl-2-[1-(3,4-Dimethyl-benzyl)-6,7-dimethoxy-3,4-dihydro-1*H*-isoquinolin-2-yl]-acetamide,

*N*-benzyl-2-[1-(3,4-Diethyl-benzyl)-6,7-dimethoxy-3,4-dihydro-1*H*-isoquinolin-2-yl]-acetamide;

2-[1-(3,4-Diethyl-benzyl)-6,7-dimethoxy-3,4-dihydro-1*H*-isoquinolin-2-yl]-*N*-(pyridin-2-yl-methyl)-acetamide;

2-[1-(3,4-Diethyl-benzyl)-6,7-dimethoxy-3,4-dihydro-1*H*-isoquinolin-2-yl]-*N*-(pyridin-3-yl-methyl)-acetamide;

2-[1-(3,4-Diethyl-benzyl)-6,7-dimethoxy-3,4-dihydro-1*H*-isoquinolin-2-yl]-*N*-(pyridin-4-yl-methyl)-acetamide;

2-[1-(3,4-Dichloro-benzyl)-6,7-dimethoxy-3,4-dihydro-1*H*-isoquinolin-2-yl]-*N*-(pyridin-3-yl-methyl)-acetamide.

**4.** A process for the combinatorial preparation of compounds of the general formula I according to claim 1, wherein $R^6$, $R^7$ and $R^9$ are hydrogen, by using an Ugi-three-components-condensation reaction, comprising the one pot reaction of a compound of formula III

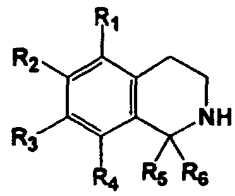

formula III

wherein $R_1$, $R_2$, $R_3$, $R_4$ and $R_5$ have the meaning given in formula I above and $R_6$ represents hydrogen, with a compound of formula IV

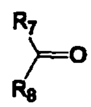

formula IV

wherein $R_7$ represents hydrogen and $R_8$ has the meaning given in formula I above, and a compound of formula V

$$R_{10}\text{-}N{\equiv}C$$

formula V

wherein $R_{10}$ has the meaning given in formula I above, if desired, isolating pharmacologically active compounds in a manner known per se, if desired, resolving a racemate obtained in a manner known per se and, if desired converting a compound or compounds obtained into a salt in a manner known per se.

**5.** A process for the preparation of compounds of formula I above, comprising reacting a compound of formula III',

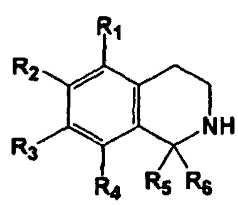

formula III'

wherein the substituents $R_1$ to $R_6$ have the meaning given in formula I above, with a compound of formula VI

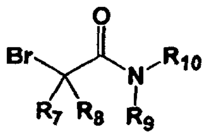

formula VI

wherein $R_7$ to $R_{10}$ have the meaning given in formula I above.

**6.** A process for the preparation of compounds of formula I above, comprising reacting a compound of formula III',

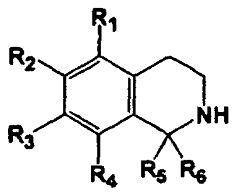

formula III'

wherein the substituents $R_1$ to $R_6$ have the meaning given in formula I above, with

a) a compound of formula IX

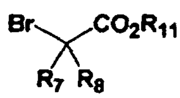

formula IX

wherein $R_7$, $R_8$ and $R_{11}$ have the meaning given in formula I above,
b) cleaving an ester obtained in a manner known per se and reacting the acid formed with
c) a compound of formula X

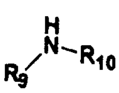

formula X

wherein the substituents $R_9$ and $R_{10}$ have the meaning given in formula I above, if desired, resolving a racemate obtained in a manner known per se and, if desired, converting a compound obtained into a salt in a manner known per se.

**7.** Pharmaceutical compositions for the treatment of disorders which are associated with the role of orexin, especially disorders such as obesity and sleep disorders, comprising containing a compound of any one of claims 1 to 3, or a pharmaceutically acceptable salt thereof, and usual carrier materials and adjuvants.

**8.** The compounds of any one of claims 1 to 3, or a pharmaceutically acceptable salt thereof, for use as medicaments for the treatment of disorders which are associated with a role of orexin, especially obesity and sleep disorders.

**9.** A process for the manufacture of pharmaceutical compositions for the treatment of disorders associated with the role of orexin, especially obesity and sleep disorders, containing one or more compounds as claimed in any one of claims 1 to 3, or a pharmaceutically acceptable salt or salts thereof, as active ingredients which process comprises mixing one or more active ingredient or ingredients with pharmaceutically acceptable excipients and adjuvants in a manner known per se.

**Patentansprüche**

**1.** Verbindungen der allgemeinen Formel (I)

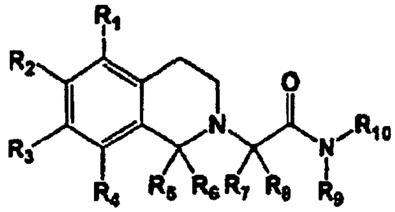

Formel (I)

worin:

$R^1$, $R^2$, $R^3$, $R^4$ unabhängig Cyano, Nitro, Halogen, Wasserstoff, Hydroxy, Niederalkyl, Niederalkenyl, Niederalkoxy, Niederalkenyloxy, Trifluormethyl, Trifluormethoxy, Cycloalkyloxy, Aryloxy, Aralkyloxy, Heterocyclyloxy, Heterocyclylalkyloxy, $R^{11}$-CO-, $NR^{12}R^{13}$CO-, $R^{12}R^{13}$N-, $R^{11}$OOC-, $R^{11}SO_2$NH- oder $R^{14}$-CO-NH- darstellen oder $R^2$ und
$R^3$ zusammen sowie $R^1$ und $R^2$ zusammen und $R^3$ und $R^4$ zusammen mit dem Phenylring einen 5-, 6- oder 7-gliedrigen Ring, der ein oder zwei Sauerstoffatome enthält, bilden können;
$R^5$ Aryl, Aralkyl, Niederalkenyl, Trifluormethyl, Cycloalkyl, Heterocyclyl oder Heterocyclylniederalkyl wiedergibt;
$R^6$ Wasserstoff, Aryl, Aralkyl, Niederalkyl, Niederalkenyl, Trifluormethyl, Cycloalkyl, Heterocyclyl oder Heterocyclylniederalkyl wiedergibt;
$R^7$ und $R^8$ unabhängig Wasserstoff, Aryl, Aralkyl, Niederalkyl, Niederalkenyl, Cycloalkyl, Heterocyclyl oder Heterocyclylniederalkyl wiedergeben;
$R^9$ Aryl, Aralkyl, Niederalkyl, Niederalkenyl, Trifluormethyl, Cycloalkyl, Heterocyclyl oder Heterocyclylniederalkyl wiedergibt;
$R^{10}$ Wasserstoff, Aryl, Aralkyl, Niederalkyl, Niederalkenyl, Trifluormethyl, Cycloalkyl, Heterocyclyl oder Heterocyclylniederalkyl wiedergibt;
$R^{11}$ Niederalkyl, Aryl, Aralkyl, Heterocyclyl oder Heterocyclylniederalkyl wiedergibt;
$R^{12}$ und $R^{13}$ unabhängig Wasserstoff, Alkyl, Cycloalkyl,
Aryl, Aralkyl, Heterocyclyl oder Heterocyclylniederalkyl wiedergeben;
$R^{14}$ Alkyl, Aryl, Cycloalkyl, Heterocyclyl, $R^{12}R^{13}$N- oder
$R^{11}$O- wiedergibt,

wobei der Begriff "Niederalkyl", einzeln oder in Kombination, eine geradkettige oder verzweigtkettige Alkylgruppe mit 1 bis 8 Kohlenstoffatomen bedeutet; der Begriff "Niederalkenyl", einzeln oder Kombination, eine geradkettige oder verzweigtkettige Alkenylgruppe mit 2 bis 5 Kohlenstoffatomen wiedergibt; der Begriff "Cycloalkyl", einzeln oder in Kombination, einen Cycloalkylring mit 3 bis 8 Kohlenstoffatomen bedeutet; der Begriff "Aryl", einzeln oder in Kombination, eine Phenyl- oder Naphthylgruppe bedeutet, die gegebenenfalls einen oder mehrere Substituenten trägt; und der Begriff "Heterocyclyl", einzeln oder in Kombination, einen 5- bis 10-gliedrigen monocyclischen oder bicyclischen Ring bedeutet, der gesättigt, teilweise ungesättigt oder aromatisch sein kann, der 1, 2 oder 3 Heteroatome, ausgewählt aus Sauerstoff, Stickstoff und Schwefel, enthält, die gleich oder verschieden sein können und der bis zu 5 wahlweise Substituenten aufweisen kann;
und gegebenenfalls reine Enantiomere, Gemische von Enantiomeren, Racemate, optisch reine Diastereoisomere, Gemische von Diastereoisomeren, Diastereomerenracemate, Gemische von Diastereoisomerenracematen oder Mesoformen und pharmazeutisch verträgliche Salze davon.

**2.** Verbindungen der allgemeinen Formel (II) gemäß der allgemeinen Formel (I) von Anspruch 1

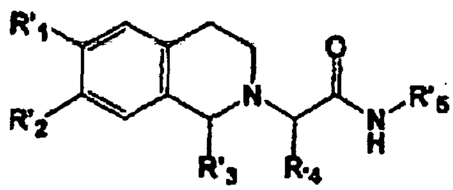

allgemeine Formel II

worin:

R'[1] und R'[2] unabhängig Wasserstoff, Hydroxy, Methoxy oder Halogen wiedergeben oder mit dem Phenylring einen 5-, 6- oder 7-gliedrigen Ring bilden können, der ein oder zwei Sauerstoffatome enthält,
R'[3] Aryl, Aralkyl, Niederalkenyl, Cycloalkyl, Heterocyclyl oder Heterocyclylniederalkyl wiedergibt;
R'[4] Wasserstoff, Aryl, Aralkyl, Niederalkyl, Niederalkenyl, Cycloalkyl, Heterocyclyl oder Heterocyclylniederalkyl wiedergibt;
R'[5] Aryl, Aralkyl, Niederalkyl, Niederalkenyl, Cycloalkyl, Heterocyclyl oder Heterocyclylniederalkyl wiedergibt;

und optisch reine Enantiomere, Gemische von Enantiomeren, Racemate, optisch reine Diastereoisomere, Gemische von Diastereoisomeren, Diastereoisomerenracemate, Gemisch von Diastereoisomerenracematen oder Mesoformen und pharmazeutisch verträgliche Salze davon.

**3.** Verbindung nach einem der Ansprüche 1 bis 2, ausgewählt aus der Gruppe, bestehend aus:

2-[1-(3,4-Dimethoxybenzyl)-5,8-dimethoxy-3,4-dihydro-1*H*-isochinolin-2-yl]-*N*-(pyridin-2-ylmethyl)acetamid;
2-[1-(3,4-Dimethoxybenzyl)-8-(cyclopropylmethoxy)-5-methoxy-3,4-dihydro-1*H*-isochinolin-2-yl]-*N*-(pyridin-2-ylmethyl)acetamid;
2-[1-(3,4-Dimethoxybenzyl)-8-(2-fluorethoxy)-5-methoxy-3,4-dihydro-1*H*-isochinolin-2-yl]-*N*-(pyridin-2-ylmethyl)acetamid;
2-[1-(3,4-Dimethoxybenzyl)-8-(2,2-difluorethoxy)-5-methoxy-3,4-dihydro-1*H*-isochinolin-2-yl]-*N*-(pyridin-2-ylmethyl)acetamid;
2-[1-(3,4-Dimethoxybenzyl)-8-ethoxy-5-methoxy-3,4-dihydro-1*H*-isochinolin-2-yl]-*N*-(pyridin-2-ylmethyl)acetamid;
2-[1-(3,4-Dimethoxybenzyl)-8-propoxy-5-methoxy-3,4-dihydro-1*H*-isochinolin-2-yl]-*N*-(pyridin-2-ylmethyl) acetamid;
2-[1-(3,4-Dimethoxybenzyl)-8-allyloxy-5-methoxy-3,4-dihydro-1*H*-isochinolin-2-yl]-*N*-(pyridin-2-ylmethyl)-acetamid;
2-[1-(3,4-Dimethoxybenzyl)-8-isopropoxy-5-methoxy-3,4-dihydro-1*H*-isochinolin-2-yl]-*N*-(pyridin-2-ylmethyl)-acetamid;
2-[1-(3,4-Dimethoxybenzyl)-5-propoxy-8-methoxy-3,4-dihydro-1*H*-isochinolin-2-yl]-*N*-(pyridin-2-ylmethyl) acetamid;
2-[1-(3,4-Dimethoxybenzyl)-6,7-dimethoxy-3,4-dihydro-1*H*-isochinolin-2-yl]-*N*-benzylacetamid;
2-[1-(3,4-Dimethoxybenzyl)-6,7-dimethoxy-3,4-dihydro-1*H*-isochinolin-2-yl]-*N*-naphthalin-1-ylmethylacetamid;
2-{1-[4-(Pyrimidin-2-yloxy)-3-methoxybenzyl]-6,7-dimethoxy-3,4-dihydro-1*H*-isochinolin-2-yl}-*N*-benzylacetamid;
2-[1-(3,4-Dimethoxybenzyl)-7-(1-methylprop-2-oxy)-6-methoxy-3,4-dihydro-1*H*-isochinolin-2-yl]-*N*-benzylacetamid;
2-[1-(3,4-Dimethoxybenzyl)-6-methoxy-7-isopropoxy-3,4-dihydro-1*H*-isochinolin-2-yl]-*N*-benzylacetamid;
2-[1-(3,4-Dimethoxybenzyl)-7-ethoxy-6-methoxy-3,4-dihydro-1*H*-isochinolin-2-yl]-*N*-benzylacetamid;
2-[1-(3,4-Dimethoxybenzyl)-7-propoxy-6-methoxy-3,4-dihydro-1*H*-isochinolin-2-yl]-*N*-benzylacetamid;
2-[1-(3,4-Dimethoxybenzyl)-7-allyloxy-6-methoxy-3,4-dihydro-1*H*-isochinolin-2-yl]-*N*-benzylacetamid;
*N*-Benzyl-2-[1-(3,4-dimethylbenzyl)-6,7-dimethoxy-3,4-dihydro-1*H*-isochinolin-2-yl]acetamid;
*N*-Benzyl-2-[1-(3,4-diethylbenzyl)-6,7-dimethoxy-3,4-dihydro-1*H*-isochinolin-2-yl]acetamid;

2-[1-(3,4-Diethylbenzyl)-6,7-dimethoxy-3,4-dihydro-1*H*-isochinolin-2-yl]-*N*-(pyridin-2-ylmethyl)acetamid;
2-[1-(3,4-Diethylbenzyl)-6,7-dimethoxy-3,4-dihydro-1*H*-isochinolin-2-yl]-*N*-(pyridin-3-ylmethyl)acetamid;
2-[1-(3,4-Diethylbenzyl)-6,7-dimethoxy-3,4-dihydro-1*H*-isochinolin-2-yl]-*N*-(pyridin-4-ylmethyl)acetamid;
2-[1-(3,4-Dichlorbenzyl)-6,7-dimethoxy-3,4-dihydro-1*H*-isochinolin-2-yl]-*N*-(pyridin-3-ylmethyl)acetamid.

**4.** Verfahren für die kombinatorische Herstellung von Verbindungen der allgemeinen Formel I nach Anspruch 1, worin $R^6$, $R^7$ und $R^9$ Wasserstoff darstellen, durch Anwenden einer Ugi-Dreikomponenten-Kondensationsreaktion, umfassend die Eintopfreaktion einer Verbindung der Formel III

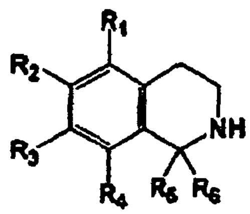

Formel III

worin $R_1$, $R_2$, $R_3$, $R_4$ und $R_5$ die in vorstehender Formel I angegebene Bedeutung aufweisen und $R_6$ Wasserstoff wiedergibt,
mit einer Verbindung der Formel IV

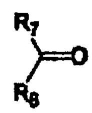

Formel IV

worin $R_7$ Wasserstoff wiedergibt und $R_8$ die in vorstehender Formel I angegebene Bedeutung aufweist,
und einer Verbindung der Formel V

$$R_{10}\text{-}N{\equiv}C$$

Formel V

worin $R_{10}$ die in vorstehender Formel I angegebene Bedeutung aufweist,
falls erwünscht, Isolieren von pharmakologischen Wirkstoffen in an sich bekannten Weise, falls erwünscht, Auftrennen eines erhaltenen Racemats in an sich bekannter Weise und, falls erwünscht, Umwandeln einer erhaltenen Verbindung oder erhaltener Verbindungen in ein Salz in an sich bekannter Weise.

**5.** Verfahren zur Herstellung von Verbindungen der vorstehenden Formel I, umfassend Umsetzen einer Verbindung der Formel III'

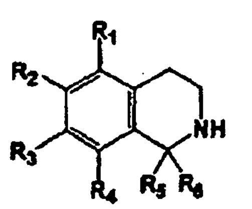

Formel III'

worin die Substituenten $R_1$ bis $R_6$ die in vorstehender Formel I angegebene Bedeutung aufweisen, mit einer Ver-

bindung der Formel VI

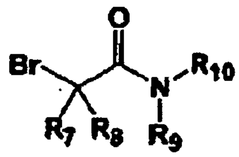

Formel VI

worin $R_7$ bis $R_{10}$ die in vorstehender Formel I angegebene Bedeutung aufweisen.

**6.** Verfahren zur Herstellung von Verbindungen der vorstehenden Formel I, umfassend Umsetzen einer Verbindung der Formel III'

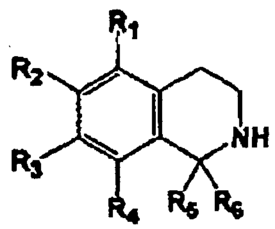

Formel III'

worin die Substituenten $R_1$ bis $R_6$ die in vorstehender Formel I angegebene Bedeutung aufweisen, mit

a) einer Verbindung der Formel IX

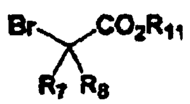

Formel IX

worin $R_7$, $R_8$ und $R_{11}$ die in vorstehender Formel I angegebene Bedeutung aufweisen,
b) Spalten eines erhaltenen Esters in an sich bekannter Weise und Umsetzen der gebildeten Säure mit
c) einer Verbindung der Formel X

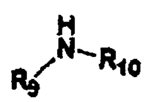

Formel X

worin die Substituenten $R_9$ und $R_{10}$ die in vorstehender Formel I angegebene Bedeutung aufweisen, falls erwünscht, Auftrennen eines erhaltenen Racemats in an sich bekannter Weise und, falls erwünscht, Umwandeln einer erhaltenen Verbindung in ein Salz in an sich bekannter Weise.

**7.** Pharmazeutische Zusammensetzungen für die Behandlung von Störungen, die mit der Rolle von Orexin verbunden sind, insbesondere Störungen, wie Fettsucht und Schlafstörungen, umfassend Enthalten einer Verbindung nach einem der Ansprüche 1 bis 3 oder eines pharmazeutisch verträglichen Salzes davon und üblicher Trägermaterialien und Hilfsmittel.

**8.** Verbindungen nach einem der Ansprüche 1 bis 3 oder ein pharmazeutisch verträgliches Salz davon zur Verwendung als Arzneimittel für die Behandlung von Störungen, die mit einer Rolle von Orexin verbunden sind, insbesondere Fettsucht und Schlafstörungen.

**9.** Verfahren zur Herstellung von pharmazeutischen Zusammensetzungen für die Behandlung von Störungen, die mit der Rolle von Orexin verbunden sind, insbesondere Fettsucht und Schlafstörungen, die eine oder mehrere Verbindungen nach einem der Ansprüche 1 bis 3 enthalten oder ein pharmazeutisch verträgliches Salz oder Salze davon als Wirkbestandteile, wobei das Verfahren Vermischen von einem oder mehreren Wirkbestandteil oder -bestandteilen mit pharmazeutisch verträglichen Exzipienten und Hilfsmitteln in an sich bekannter Weise umfasst.

## Revendications

**1.** Composés de formule générale (I)

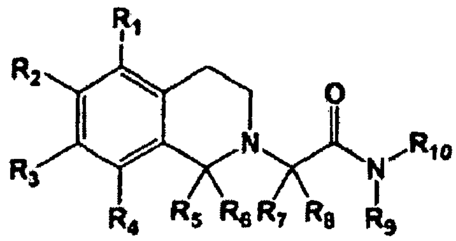

formule (I)

dans laquelle :

$R_1$, $R_2$, $R_3$ et $R_4$ représentent indépendamment un groupe cyano, nitro, halogéno, hydrogène, hydroxy, alkyle inférieur, alcényle inférieur, alkoxy inférieur, alcényloxy inférieur, trifluorométhyle, trifluorométhoxy, cycloalkyloxy, aryloxy, aralkyloxy, hétérocyclyloxy, hétérocyclylalkyloxy, $R_{11}CO$-, $NR_{12}R_{13}CO$-, $R_{12}R_{13}N$-, $R_{11}OOC$-, $R_{11}SO_2NH$- ou $R_{14}$-CO-NH-, ou $R_2$ et $R_3$, conjointement, ainsi que $R_1$ et $R_2$, conjointement, et $R_3$ et $R_4$, conjointement, peuvent former avec le noyau phényle un noyau penta-, hexa- ou heptagonal contenant un ou deux atomes d'oxygène ;
$R_5$ représente un groupe aryle, aralkyle, alcényle inférieur, trifluorométhyle, cycloalkyle, hétérocyclyle ou hétérocyclyl-alkyle inférieur ;
$R_6$ représente un atome d'hydrogène, un groupe aryle, aralkyle, alkyle inférieur, alcényle inférieur, trifluorométhyle, cycloalkyle, hétérocyclyle ou hétérocyclyl-alkyle inférieur ;
$R_7$ et $R_8$ représentent indépendamment un atome d'hydrogène, un groupe aryle, aralkyle, alkyle inférieur, alcényle inférieur, cycloalkyle, hétérocyclyle ou hétérocyclyl-alkyle inférieur ;
$R_9$ représente un groupe aryle, aralkyle, alkyle inférieur, alcényle inférieur, trifluorométhyle, cycloalkyle, hétérocyclyle ou hétérocyclyl-alkyle inférieur ;
$R_{10}$ représente un atome d'hydrogène, un groupe aryle, aralkyle, alkyle inférieur, alcényle inférieur, trifluorométhyle, cycloalkyle, hétérocyclyle ou hétérocyclyl-alkyle inférieur ;
$R_{11}$ représente un groupe alkyle inférieur, aryle, aralkyle, hétérocyclyle ou hétérocyclyl-alkyle inférieur ;
$R_{12}$ et $R_{13}$ représentent indépendamment un atome d'hydrogène, un groupe alkyle, cycloalkyle, aryle, aralkyle, hétérocyclyle ou hétérocyclyl-alkyle inférieur ;
$R_{14}$ représente un groupe alkyle, aryle, cycloalkyle, hétérocyclyle, $R_{12}R_{13}N$- ou $R_{11}O$-,

l'expression "alkyle inférieur", seule ou en association, désignant un groupe alkyle à chaîne droite ou à chaîne ramifiée ayant 1 à 8 atomes de carbone, l'expression "alcényle inférieur", seule ou en association, désignant un groupe alcényle à chaîne droite ou à chaîne ramifiée ayant 2 à 5 atomes de carbone, le terme "cycloalkyle", seul ou en association, désignant un noyau cycloalkyle ayant 3 à 8 atomes de carbone ; le terme "aryle", seul ou en association, désignant un groupe phényle ou naphtyle qui porte facultativement un ou plusieurs substituants ; et le terme "hétérocyclyle", seul ou en association, désignant un noyau monocyclique ou bicyclique penta- à décagonal, qui peut être saturé, partiellement insaturé ou aromatique, contenant 1, 2 ou 3 hétéroatomes choisis entre

des atomes d'oxygène, d'azote et de soufre, pouvant être identiques ou différents, et pouvant porter jusqu'à 5 substituants facultatifs ;
et les énantiomères optiquement purs, mélanges d'énantiomères, racémates, diastéréoisomères optiquement purs, mélanges de diastéréoisomères, racémates diastéréoisomères, mélanges de racémates diastéréoisomères ou formes méso et sels pharmaceutiquement acceptables.

**2.** Composés de formule générale (II) suivant la formule générale (I) de la revendication 1

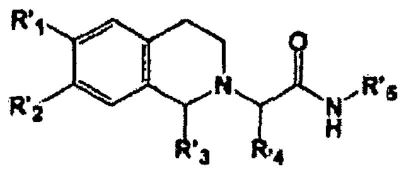

formule générale II

dans laquelle :

R'$_1$ et R'$_2$ représentent indépendamment un atome d'hydrogène, un groupe hydroxy, méthoxy ou halogéno, ou bien peuvent former avec le noyau phényle un noyau penta-, hexa- ou heptagonal contenant un ou deux atomes d'oxygène ; R'$_3$ représente un groupe aryle, aralkyle, alcényle inférieur, cycloalkyle, hétérocyclyle ou hétérocyclyl-alkyle inférieur ;
R'$_4$ représente un atome d'hydrogène, un groupe aryle, aralkyle, alkyle inférieur, alcényle inférieur, cycloalkyle, hétérocyclyle ou hétérocyclyl-alkyle inférieur ;
R'$_5$ représente un groupe aryle, aralkyle, alkyle inférieur, alcényle inférieur, cycloalkyle, hétérocyclyle ou hétérocyclyl-alkyle inférieur ;

et leurs énantiomères optiquement purs, mélanges d'énantiomères, racémates, diastéréoisomères optiquement purs, mélanges de diastéréoisomères, racémates diastéréoisomères, mélanges de racémates diastéréoisomères ou formes méso et sels pharmaceutiquement acceptables.

**3.** Composé suivant l'une quelconque des revendications 1 et 2, choisi dans le groupe consistant en :

2-[1-(3,4-diméthoxybenzyl)-5,8-diméthoxy-3,4-dihydro-1*H*-isoquinoléine-2-yl]-*N*-(pyridine-2-ylméthyl)-acétamide ;
2-[1-(3,4-diméthoxybenzyl)-8-(cyclopropylméthoxy)-5-méthoxy-3,4-dihydro-1*H*-isoquinoléine-2-yl]-*N*-(pyridine-2-ylméthyl)-acétamide ;
2-[1-(3,4-diméthoxybenzyl)-8-(2-fluoréthoxy)-5-méthoxy-3,4-dihydro-1*H*-isoquinoléine-2-yl]-*N*-(pyridine-2-ylméthyl)-acétamide ;
2-[1-(3,4-diméthoxybenzyl)-8-(2,2-difluoréthoxy)-5-méthoxy-3,4-dihydro-1*H*-isoquinoléine-2-yl]-*N*-(pyridine-2-ylméthyl)-acétamide ;
2-[1-(3,4-diméthoxybenzyl)-8-éthoxy-5-méthoxy-3,4-dihydro-1*H*-isoquinoléine-2-yl]-*N*-(pyridine-2-ylméthyl)-acétamide ;
2-[1-(3,4-diméthoxybenzyl)-8-propoxy-5-méthoxy-3,4-dihydro-1*H*-isoquinoléine-2-yl]-*N*-(pyridine-2-ylméthyl)-acétamide ;
2-[1-(3,4-diméthoxybenzyl)-8-allyloxy-5-méthoxy-3,4-dihydro-1*H*-isoquinoléine-2-yl]-*N*-(pyridine-2-ylméthyl)-acétamide ;
2-[1-(3,4-diméthoxybenzyl)-8-isopropoxy-5-méthoxy-3,4-dihydro-1*H*-isoquinoléine-2-yl]-*N*-(pyridine-2-ylméthyl)-acétamide ;
2-[1-(3,4-diméthoxybenzyl)-5-propoxy-8-méthoxy-3,4-dihydro-1*H*-isoquinoléine-2-yl]-*N*-(pyridine-2-ylméthyl)-acétamide ;
2-[1-(3,4-diméthoxybenzyl)-6,7-diméthoxy-3,4-dihydro-1*H*-isoquinoléine-2-yl]-*N*-benzylacétamide ;
2-[1-(3,4-diméthoxybenzyl)-6,7-diméthoxy-3,4-dihydro-1*H*-isoquinoléine-2-yl]-*N*-naphtalène-1-ylméthylacétamide ;
2-{1-[4-(pyrimidine-2-yloxy)-3-méthoxybenzyl]-6,7-diméthoxy-3,4-dihydro-1*H*-isoquinoléine-2-yl}-N-

benzylacétamide ;
2-[1-(3,4-diméthoxybenzyl)-7-(1-méthylprop-2-oxy)-6-méthoxy-3,4-dihydro-1*H*-isoquinoléine-2-yl]-N-benzylacétamide ;
2-[1-(3,4-diméthoxybenzyl)-6-méthoxy-7-isopropoxy-3,4-dihydro-1*H*-isoquinoléine-2-yl]-*N*-benzylacétamide ;
2-[1-(3,4-diméthoxybenzyl)-7-éthoxy-6-méthoxy-3,4-dihydro-1*H*-isoquinoléine-2-yl]-*N*-benzylacétamide ;
2-[1-(3,4-diméthoxybenzyl)-7-propoxy-6-méthoxy-3,4-dihydro-1*H*-isoquinoléine-2-yl]-*N*-benzylacétamide ;
2-[1-(3,4-diméthoxybenzyl)-7-allyloxy-6-méthoxy-3,4-dihydro-1*H*-isoquinoléine-2-yl]-*N*-benzylacétamide ;
*N*-benzyl-2-[1-(3,4-diméthylbenzyl)-6,7-diméthoxy-3,4-dihydro-1*H*-isoquinoléine-2-yl]-acétamide ;
*N*-benzyl-2-[1-(3,4-diéthylbenzyl)-6,7-diméthoxy-3,4-dihydro-1*H*-isoquinoléine-2-yl]-acétamide ;
2-[1-(3,4-diéthylbenzyl)-6,7-diméthoxy-3,4-dihydro-1*H*-isoquinoléine-2-yl]-*N*-(pyridine-2-ylméthyl)-acétamide ;
2-[1-(3,4-diéthylbenzyl)-6,7-diméthoxy-3,4-dihydro-1*H*-isoquinoléine-2-yl]-*N*-(pyridine-3-ylméthyl)-acétamide ;
2-[1-(3,4-diéthylbenzyl)-6,7-diméthoxy-3,4-dihydro-1*H*-isoquinoléine-2-yl]-*N*-(pyridine-4-ylméthyl)-acétamide ;
2-[1-(3,4-dichlorobenzyl)-6,7-diméthoxy-3,4-dihydro-1*H*-isoquinoléine-2-yl]-*N*-(pyridine-3-ylméthyl)-acétamide ;

**4.** Procédé pour la préparation combinatoire de composés de formule générale I suivant la revendication 1, dans lesquels $R_6$, $R_7$ et $R_9$ représentent des atomes d'hydrogène, en utilisant une réaction de condensation d'Ugi à trois constituants, comprenant la réaction directe d'un composé de formule III

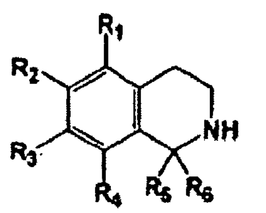

formule III

dans laquelle $R_1$, $R_2$, $R_3$, $R_4$ et $R_5$ répondent aux définitions indiquées pour la formule I ci-dessus et $R_6$ représente un atome d'hydrogène,
avec un composé de formule IV

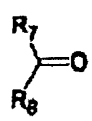

formule IV

dans laquelle $R_7$ représente un atome d'hydrogène et $R_8$ répond à la définition indiquée pour la formule I ci-dessus, et un composé de formule V

$$R_{10}\text{-}N{\equiv}C$$

formule V

dans laquelle $R_{10}$ répond à la définition indiquée pour la formule I ci-dessus,
si cela est désiré, l'isolement des composés pharmacologiquement actifs d'une manière en elle-même connue, si cela est désiré, la résolution du racémate obtenu d'une manière en elle-même connue et, si cela est désiré, la conversion d'un ou plusieurs composés obtenus en un sel d'une manière en elle-même connue.

**5.** Procédé pour la préparation de composés de formule I ci-dessus, comprenant la réaction d'un composé de formule III',

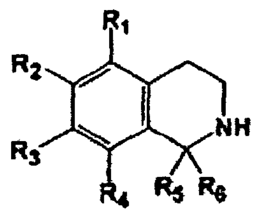

formule III'

dans laquelle les substituants $R_1$ à $R_6$ répondent aux définitions indiquées pour la formule I ci-dessus, avec un composé de formule VI

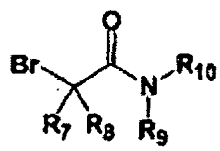

formule VI

dans laquelle $R_7$ à $R_{10}$ répondent aux définitions indiquées pour la formule I ci-dessus.

**6.** Procédé pour la préparation de composés répondant à la formule I ci-dessus, comprenant la réaction d'un composé de formule III',

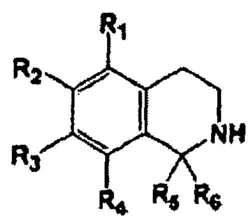

formule III'

dans laquelle les substituants $R_1$ à $R_6$ répondent aux définitions indiquées pour la formule I ci-dessus, avec

a) un composé de formule IX

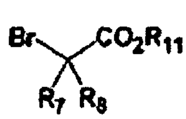

formule IX

dans laquelle $R_7$, $R_8$ et $R_{11}$ répondent aux définitions indiquées pour la formule I ci-dessus,
b) le clivage d'un ester obtenu d'une manière en elle-même connue et la réaction de l'acide formé avec
c) un composé de formule X

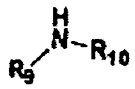

formule X

dans laquelle les substituants $R_9$ et $R_{10}$ répondent aux définitions indiquées pour la formule I ci-dessus,

si cela est désiré, la résolution d'un racémate obtenu d'une manière en elle-même connue et, si cela est désiré, la conversion d'un composé obtenu en un sel d'une manière en elle-même connue.

**7.** Composés pharmaceutiques pour le traitement d'affections qui sont associées au rôle de l'orexine, notamment dans les affections telles que l'obésité ou des troubles du sommeil, contenant un composé suivant l'une quelconque des revendications 1 à 3, ou un de ses sels pharmaceutiquement acceptables, et des matières de support et adjuvants usuels.

**8.** Composés suivant l'une quelconque des revendications 1 à 3, ou un leurs sels pharmaceutiquement acceptables, destinés à être utilisés comme médicaments pour le traitement d'affections qui sont associées à un rôle de l'orexine, notamment de l'obésité et des troubles du sommeil.

**9.** Procédé pour la production de compositions pharmaceutiques pour le traitement d'affections associées au rôle de l'orexine, notamment de l'obésité et de troubles du sommeil, contenant un ou plusieurs composés suivant l'une quelconque des revendications 1 à 3, ou un ou plusieurs de leurs sels pharmaceutiquement acceptables, comme ingrédients actifs, procédé qui comprend le mélange d'un ou plusieurs ingrédients actifs à des excipients et adjuvants pharmaceutiquement acceptables d'une manière en elle-même connue.